# EUROPEAN PATENT SPECIFICATION

(11) **EP 3 176 173 B1**
(45) Date of publication and mention of the grant of the patent: **03.06.2020**
(21) Application number: 15827529.7
(22) Date of filing: 30.07.2015
(51) Int. Cl.: C07D 519/00, A61K 31/5383, A61P 35/00, A61P 35/04, A61P 35/02

(54) **CRYSTALLINE FREE BASES OF C-MET INHIBITOR OR CRYSTALLINE ACID SALTS THEREOF, AND PREPARATION METHODS AND USES THEREOF**
KRISTALLINE FREIE BASEN DES C-MET-INHIBITORS ODER VON KRISTALLINEN SÄURESALZEN DAVON UND HERSTELLUNGSVERFAHREN UND VERWENDUNGEN DAVON
BASES CRISTALLINES LIBRES D'UN INHIBITEUR DU C-MET OU SELS ACIDES CRISTALLINS ASSOCIÉS, ET PROCÉDÉS DE PRÉPARATION ET UTILISATIONS ASSOCIÉS

(30) Priority: 01.08.2014 CN 201410378371
(43) Date of publication of application: 07.06.2017
(73) Proprietor: Jiangsu Hansoh Pharmaceutical Group Co., Ltd., Jiangsu 222047 (CN)
(72) Inventor: LI, Xiang, Lianyungang Jiangsu 222047 (CN); LV, Aifeng, Lianyungang Jiangsu 222047 (CN)
(74) Representative: Freischem & Partner Patentanwälte mbB
(86) International application number: PCT/CN2015/085514
(87) International publication number: WO 2016/015653

(56) References cited:
- WO-A1-2013/038362
- WO-A1-2013/038362
- WO-A1-2014/180182

## Description

### FIELD OF THE INVENTION

The present invention relates to the field of drugs, specifically relates to crystalline free bases of c-met inhibitor or crystalline acid salts thereof, and preparation methods and uses thereof.

### BACKGROUND OF THE INVENTION

Hepatocyte growth factor (HGF) receptor, also known as C-Met, is a tyrosine kinase receptor. Abnormal activation of C-Met is related to a poor prognosis of cancer, there is a problem of C-Met overexpression. C-Met abnormality is also found in many types of tumors, such as hepatocellular carcinoma (HCC), non-small cell lung cancer (NSCLC), bladder cancer, liver cancer, kidney cancer, stomach cancer, breast cancer, squamous cell carcinoma, brain cancer, colon cancer, etc. C-Met abnormality may be expressed as increased expression, gene amplification, gene mutation or increased expression of HGF. In these abnormal circumstances, C-Met is activated in an abnormal state, which results in carcinogenesis and poor prognosis. Abnormal activation of C-Met will lead to growth of tumor and formation of new blood vessel (angiogenesis, which can provide nutrient to the tumor), help the cancer spread to other organ (metastasis). Inhibition of C-Met signal pathway is thus an important therapeutic strategy for the treatment of cancer.

C-Met inhibitors having pharmacological activity are described by Jiangsu hansoh Company in the patent application CN201310173581.4 and the PCT application thereof (PCT/CN2014/072825), one of the compounds is 9-((8-fluoro-6-(1-methyl-1H-pyrazol-4-yl)-[1,2,4]triazolo[4,3-a]pyridine-3-yl)thio)-4-m ethyl-2H-[1,4]oxaazido[3,2-c]quinoline-3(4H)-one (referred to as "compound of formula I").

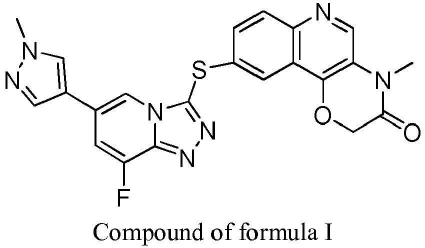

The compound of formula I is a valid C-Met / HGFR (hepatocyte growth factor receptor) kinase inhibitor, as a C-Met tyrosine kinase inhibitor, the compound of formula I can effectively block the HGF / C-Met signal transduction pathway to reach the purpose of treating abnormal cell growth (e.g. cancer) in mammals. However, in the patent application CN201310173581.4 and the PCT application thereof (PCT/CN2014/ 72825), only amorphous form of the compound of formula I was described. It is well know that the amorphous form of a drug means the drug molecules aggregate disorderly, and the drug does not contain significant lattice. The amorphous form of the drug has higher thermodynamic energy state than the crystalline form, which will result in the instability of thermodynamics. The instability of thermodynamics will lead to poor chemical stability, easy moisture absorption and solid phase transition, accordingly, the quality of the drug is extremely unstable. Therefore, it is difficult for the amorphous form to be used in drug development; furthermore, during the drug preparation, the process of drug crystallization is an effective purification method. The resulting crystalline form also has the technological operation advantage of easy further purification, easy filtration, drying and so on. Therefore, it is necessary to further research and develop new crystal forms which have good crystallinity, moderate size, good solubility, high stability in order to improve the bioavailability of the drug. The patent application CN201310173581.4 and the PCT application thereof (PCT/CN2014/072825) disclosed an amorphous free base of the compound of formula I. Said free base has a low solubility in various solvents, which is not conducive to drug dissolution in an animal or human body. Therefore, it is a very urgent task to research and develop suitable salt-form compounds in order to improve the dissolution rate and the solubility of the compound of formula I.

WO 2013/038362 A1 describes 6 - Substituted 3 - (quinolin- 6 - Ylthio) - [1,2,4] Triazolo [4, 3 -a] Pyradines As Tyrosine Kinase.

In summary, for the amorphous free base, further technical improvements are needed in drug purification, drying, storage, formulation, and dissolution and so on, in order to improve drug bioavailability.

### DESCRIPTION OF THE INVENTION

In order to solve the technical problems in the prior art, the present invention provide a crystalline free base or a crystalline acid salt of 9-((8-fluoro-6-(1-methyl-1H-pyrazol-4-yl)-[1,2,4]triazolo[4,3-a]pyridine-3-yl)thio)-4-m ethyl-2H-[1,4]oxaazido[3,2-c]quinoline-3(4H)-one (i.e., the compound of formula I), a preparation method for same and medical uses thereof. The physical and chemical properties of the compound of formula I, such as solubility, hygroscopicity and chemical stability have been greatly improved by intensive study of the different aggregation states.

In an aspect, the invention provides a crystalline free base of 9-((8-fluoro-6-(1-methyl-1H-pyrazol-4-yl)-[1,2,4]triazolo[4,3-a]pyridine-3-yl)thio)-4-m ethyl-2H-[1,4]oxaazido[3,2-c]quinoline-3(4H)-one (i.e., the compound of formula I). The polymorphs of free base include four crystal forms, referred to as crystal form I, crystal form II, crystal form III and crystal form IV respectively.

The invention provides crystal form I of free base of the compound of formula I having an X-ray powder diffraction spectrum comprising peaks at diffraction angles (2θ) of 13.0±0.2°, 17.9±0.2°, 21.2±0.2° and 31.4±0.2°.

Preferably, the X-ray powder diffraction spectrum also comprises peaks at diffraction angles (2θ) of 10.3±0.2°, 11.1±0.2°, 23.3±0.2°, 23.8±0.2° and 33.6±0.2°.

More preferably, the X-ray powder diffraction spectrum also comprises peaks at diffraction angles (2θ) of 15.7±0.2°, 17.7±0.2°, 26.8±0.2°, 28.0±0.2°, 31.7±0.2° and 32.8±0.2°.

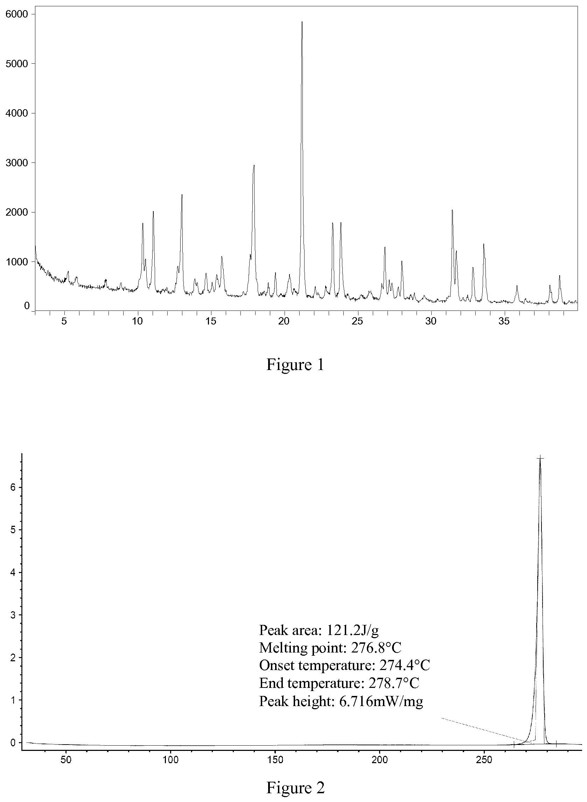

The most preferably, the X-ray powder diffraction spectrum comprises substantially the same peaks at diffraction angles (2θ) as shown in Figure 1, and the X-ray powder diffraction data are shown in table 1:

**Table 1**

| 2θ (°) | intensity % | 28 (°) | intensity % |
|---|---|---|---|
| 5.3 | 4.5 | 22.8 | 4.1 |
| 10.3 | 24.9 | 23.3 | 27.3 |
| 10.5 | 11.9 | 23.8 | 27.2 |
| 11.1 | 28.5 | 25.9 | 3.2 |
| 12.7 | 9.9 | 26.6 | 5.9 |
| 13.0 | 36.3 | 26.8 | 19.0 |
| 13.9 | 5.6 | 27.1 | 6.8 |
| 14.7 | 7.0 | 27.3 | 5.8 |
| 15.1 | 4.4 | 28.0 | 13.8 |
| 15.4 | 7.4 | 31.4 | 33.4 |
| 15.7 | 13.8 | 31.7 | 18.4 |
| 17.7 | 15.0 | 32.8 | 12.3 |
| 17.9 | 47.6 | 33.6 | 21.1 |
| 19.4 | 8.7 | 35.8 | 6.3 |
| 20.3 | 7.5 | 38.1 | 6.8 |
| 20.6 | 2.8 | 38.8 | 10.3 |
| 21.2 | 100.0 | | |

The invention provides crystal form II of free base of the compound of formula I having an X-ray powder diffraction spectrum comprising peaks at diffraction angles (2θ) of 8.6±0.2°, 11.5±0.2°, 14.1±0.2° and 19.8±0.2°.

Preferably, the X-ray powder diffraction spectrum also comprises peaks at diffraction angles (2θ) of 11.9±0.2°, 14.7±0.2°, 15.2±0.2°, 17.2±0.2° and 18.9±0.2°.

More preferably, the X-ray powder diffraction spectrum also comprises peaks at diffraction angles (2θ) of 5.8±0.2°, 7.4±0.2°, 20.9±0.2°, 30.9±0.2°, 31.4±0.2° and 37.9±0.2°.

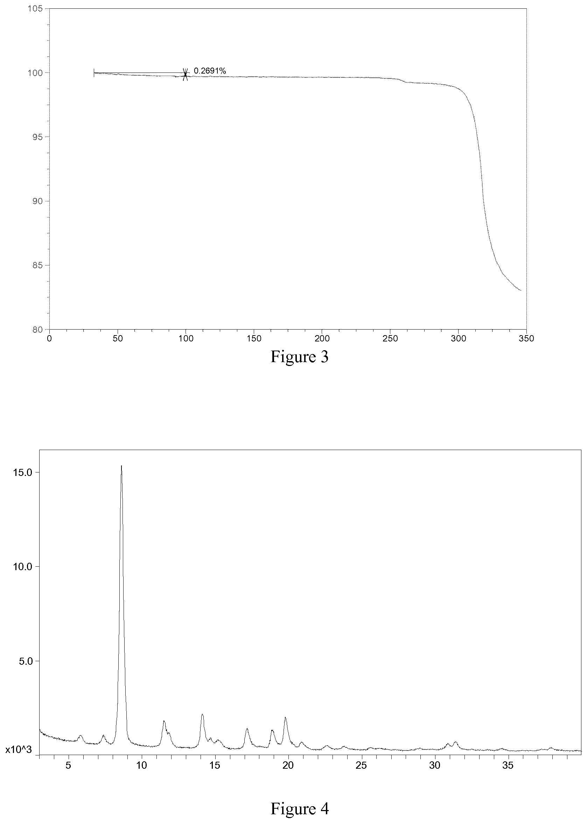

The most preferably, the X-ray powder diffraction spectrum comprises substantially the same peaks at diffraction angles (2θ) as shown in Figure 4, and the X-ray powder diffraction data are shown in table 2:

**Table 2**

| 2θ (°) | intensity % | 2θ (°) | intensity % |
|---|---|---|---|
| 5.8 | 2.1 | 17.2 | 7.8 |
| 7.4 | 2.8 | 18.9 | 5.1 |
| 8.6 | 100.0 | 19.8 | 9.6 |
| 11.5 | 13.3 | 20.9 | 2.0 |
| 11.9 | 5.6 | 30.9 | 3.5 |
| 14.1 | 10.5 | 31.4 | 4.0 |
| 14.7 | 6.2 | 37.9 | 2.2 |
| 15.2 | 4.7 | | |

The invention provides crystal form III of free base of the compound of formula I having an X-ray powder diffraction spectrum comprising peaks at diffraction angles (2θ) of 12.8±0.2°, 14.8±0.2°, 18.0±0.2° and 20.5±0.2°.

Preferably, the X-ray powder diffraction spectrum also comprises peaks at diffraction angles (2θ) of 8.9±0.2°, 9.2±0.2°, 10.6±0.2°, 15.8±0.2° and 20.7±0.2°.

More preferably, the X-ray powder diffraction spectrum also comprises peaks at diffraction angles (2θ) of 5.3±0.2°, 5.9±0.2°, 12.0±0.2°, 14.0±0.2°, 17.3±0.2° and 19.9±0.2°.

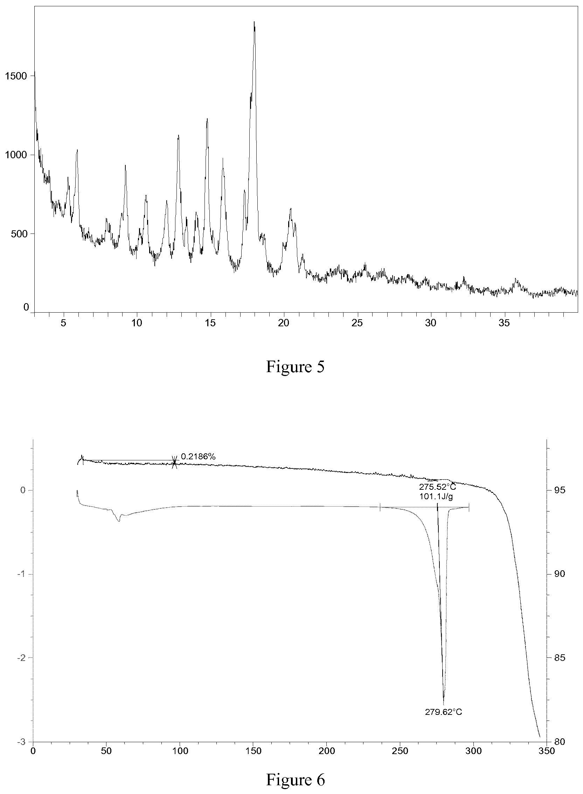

The most preferably, the X-ray powder diffraction spectrum comprises substantially the same peaks at diffraction angles (2θ) as shown in Figure 5, and the X-ray powder diffraction data are shown in table 3:

**Table 3**

| 2θ (°) | intensity % | 2θ (°) | intensity % |
|---|---|---|---|
| 5.3 | 8.2 | 14.0 | 8.6 |
| 5.9 | 11.7 | 14.8 | 31.3 |
| 7.9 | 7.6 | 15.8 | 26.2 |
| 8.9 | 18.9 | 17.3 | 10.4 |
| 9.2 | 19.1 | 18.0 | 100.0 |
| 10.6 | 14.8 | 19.9 | 11.4 |
| 12.0 | 12.8 | 20.5 | 32.8 |
| 12.8 | 30.4 | 20.7 | 24.9 |
| 13.3 | 6.6 | | |

The invention provides crystal form IV of free base of the compound of formula I having an X-ray powder diffraction spectrum comprising peaks at diffraction angles (2θ) of 8.9±0.2°, 12.6±0.2°, 17.0±0.2° and 17.9±0.2°.

Preferably, the X-ray powder diffraction spectrum also comprises peaks at diffraction angles (2θ) of 13.2±0.2°, 14.5±0.2°, 20.5±0.2°, 23.9±0.2° and 26.3±0.2°.

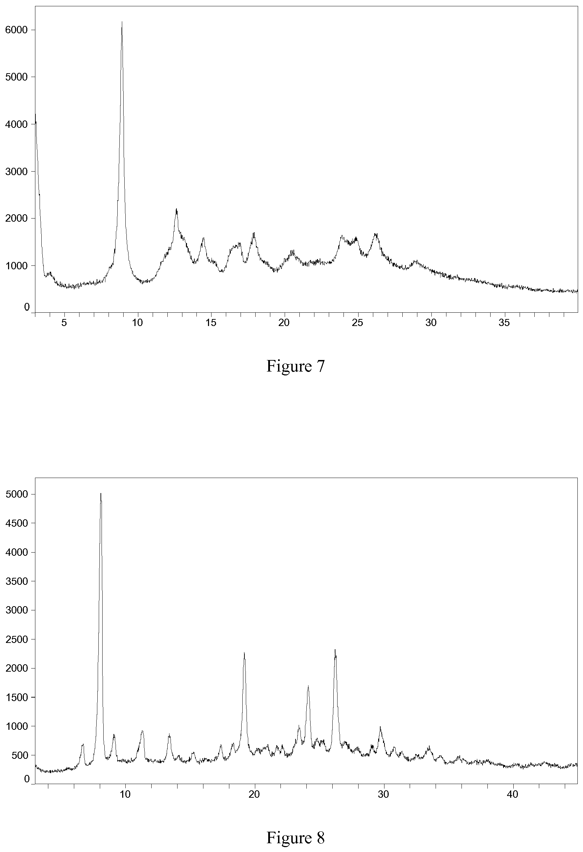

The most preferably, the X-ray powder diffraction spectrum comprises substantially the same peaks at diffraction angles (2θ) as shown in Figure 7, and the X-ray powder diffraction data are shown in table 4:

**Table 4**

| 2θ (°) | intensity % | 2θ (°) | intensity % |
|---|---|---|---|
| 8.9 | 100.0 | 17.9 | 15.1 |
| 12.6 | 34.5 | 20.5 | 11.1 |
| 13.2 | 14.6 | 23.9 | 6.7 |
| 14.5 | 13.8 | 26.3 | 13.9 |
| 17.0 | 16.1 | 29.0 | 6.5 |

In another aspect, the invention provides a crystalline acid salt of 9-((8-fluoro-6-(1-methyl-1H-pyrazol-4-yl)-[1,2,4]triazolo[4,3-a]pyridine-3-yl)thio)-4-m ethyl-2H-[1,4]oxaazido[3,2-c]quinohne-3(4H)-one.

The acid salt comprises an inorganic acid salt and an organic acid salt; the inorganic acid salt is preferably selected from the group consisting of hydrochloride, sulfate, hydrobromide, hydrofluoride, hydroiodide and phosphate, more preferably selected from the group consisting of hydrochloride, sulfate and phosphate; the organic acid salt is preferably selected from the group consisting of 2,5-dihydroxybenzeneformate, 1-hydroxy-2-naphthaleneformate, acetate, dichloroacetate, trichloroacetate, acetyl oxygenoximoate, adipate, benzene sulfonate, 4-chlorobenzene sulfonate, benzeneformate, 4-acetamidobenzeneformate, 4-aminobenzeneformate, caprate, caproate, caprilate, cinnamoate, citrate, cyclohexylsulfamate , camphorsulfonate, aspartate, camphorate, gluconate, glucuronate, glutamate, erythorbate, lactate, aspartate, malate, mandelate, pyroglutamate, tartrate, lauryl sulfate, dibenzoyltartrate, ethyl-1,2-disulfonate, esylate, formate, fumarate, galactonate, gentisate, glutarate, 2-oxoglutarate, glycollate, hippurate, isethionate, lactobionate , ascorbate, aspartate, laurate, camphorate, maleate, malonate, mesylate, 1,5-naphthalenedisulfonate, naphthalene-2-sulfonate, nicotinate, oleate, orotate, oxalate, palmitate, embonate, propionate, salicylate, 4-aminosalicylate, sebacate, stearate, butanedioate, thiocyanate, undecylenate, trifluoroacetate, succinate and p-toluenesulfonate, more preferably selected from the group consisting of mesylate, p-toluenesulfonate and 1,5-naphthalenedisulfonate.

The invention provides a polymorph of hydrochloride of the compound of formula I, comprising one crystal form, referred to as crystal form I, which has an X-ray powder diffraction spectrum comprising peaks at diffraction angles (2θ) of 8.1±0.2°, 19.2±0.2°, 24.1±0.2° and 26.2±0.2°.

Preferably, the X-ray powder diffraction spectrum also comprises peaks at diffraction angles (2θ) of 9.1±0.2°, 11.3±0.2°, 13.4±0.2°, 29.7±0.2° and 23.4±0.2°.

More preferably, the X-ray powder diffraction spectrum comprises substantially the same peaks at diffraction angles (2θ) as shown in Figure 8, and the X-ray powder diffraction data are shown in table 5:

**Table 5**

| 2θ (°) | intensity % | 2θ (°) | intensity % |
|---|---|---|---|
| 6.7 | 8.1 | 23.4 | 10.6 |
| 8.1 | 100.0 | 24.1 | 21.4 |
| 9.1 | 8.8 | 26.2 | 41.4 |
| 11.3 | 16.6 | 29.7 | 14.0 |
| 13.4 | 12.6 | 30.8 | 5.6 |
| 19.2 | 39.0 | 33.5 | 6.3 |

The invention provides a polymorph of sulfate of the compound of formula I, comprising two crystal forms, referred to as crystal form I and crystal form II respectively.

The invention provides crystal form I of sulfate of the compound of formula I having an X-ray powder diffraction spectrum comprising peaks at diffraction angles (2θ) of 18.4±0.2°, 19.7±0.2°, 23.8±0.2° and 24.5±0.2°.

Preferably, the X-ray powder diffraction spectrum also comprises peaks at diffraction angles (2θ) of 12.8±0.2°, 14.4±0.2°, 17.0±0.2°, 20.0±0.2° and 21.0±0.2°.

More preferably, the X-ray powder diffraction spectrum also comprises peaks at diffraction angles (2θ) of 8.7±0.2°, 13.2±0.2°, 19.1±0.2°, 26.3±0.2°, 26.6±0.2° and 29.0±0.2°.

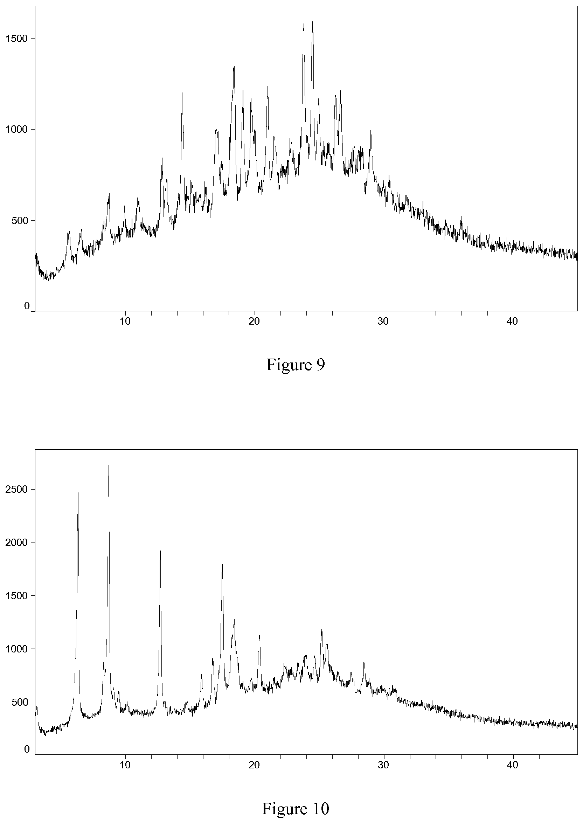

The most preferably, the X-ray powder diffraction spectrum comprises substantially the same peaks at diffraction angles (2θ) as shown in Figure 9, and the X-ray powder diffraction data are shown in table 6:

**Table 6**

| 2θ (°) | intensity % | 2θ (°) | intensity % |
|---|---|---|---|
| 5.7 | 19.7 | 19.7 | 84.5 |
| 6.6 | 14.9 | 20.0 | 49.4 |
| 8.7 | 37.2 | 21.0 | 58.7 |
| 9.9 | 13.6 | 21.6 | 32.0 |
| 11.0 | 33.6 | 22.7 | 25.3 |
| 12.8 | 45.8 | 23.8 | 89.8 |
| 13.2 | 40.1 | 24.5 | 83.2 |
| 14.4 | 78.3 | 24.9 | 28.3 |
| 17.0 | 75.6 | 26.3 | 45.2 |
| 17.4 | 27.5 | 26.6 | 41.1 |
| 18.4 | 100.0 | 29.0 | 41.6 |
| 19.1 | 38.8 | | |

The invention provides crystal form II of sulfate of the compound of formula I having an X-ray powder diffraction spectrum comprising peaks at diffraction angles (2θ) of 6.3±0.2°, 8.7±0.2°, 12.7±0.2° and 18.4±0.2°.

Preferably, the X-ray powder diffraction spectrum also comprises peaks at diffraction angles (2θ) of 8.3±0.2°, 17.5±0.2°, 18.7±0.2°, 20.4±0.2° and 25.6±0.2°.

More preferably, the X-ray powder diffraction spectrum also comprises peaks at diffraction angles (2θ) of 9.1±0.2°, 15.9±0.2°, 16.8±0.2°, 24.0±0.2°, 25.2±0.2° and 28.4±0.2°.

The most preferably, the X-ray powder diffraction spectrum comprises substantially the same peaks at diffraction angles (2θ) as shown in Figure 10, and the X-ray powder diffraction data are shown in table 7:

**Table 7**

| 2θ (°) | intensity % | 2θ (°) | intensity % |
|---|---|---|---|
| 6.3 | 84.8 | 18.4 | 49.8 |
| 8.3 | 26.9 | 18.7 | 22.9 |
| 8.7 | 100.0 | 20.4 | 21.6 |
| 9.1 | 11.6 | 24.0 | 13.4 |
| 9.5 | 6.4 | 24.6 | 5.9 |
| 12.7 | 53.8 | 25.2 | 18.0 |
| 15.9 | 11.7 | 25.6 | 26.7 |
| 16.8 | 11.0 | 28.4 | 14.8 |
| 17.5 | 46.7 | | |

The invention provides a polymorph of phosphate of the compound of formula I, comprising four crystal forms, referred to as crystal form I, crystal form II, crystal form III and crystal form IV respectively.

The invention provides crystal form I of phosphate of the compound of formula I having an X-ray powder diffraction spectrum comprising peaks at diffraction angles (2θ) of 7.9±0.2°, 12.8±0.2°, 15.9±0.2° and 18.3±0.2°.

Preferably, the X-ray powder diffraction spectrum also comprises peaks at diffraction angles (2θ) of 5.3±0.2°, 10.6±0.2°, 13.4±0.2°, 20.9±0.2° and 24.7±0.2°.

More preferably, the X-ray powder diffraction spectrum also comprises peaks at diffraction angles (2θ) of 16.5±0.2°, 18.7±0.2°, 20.6±0.2°, 21.8±0.2°, 26.2±0.2° and 27.4±0.2°.

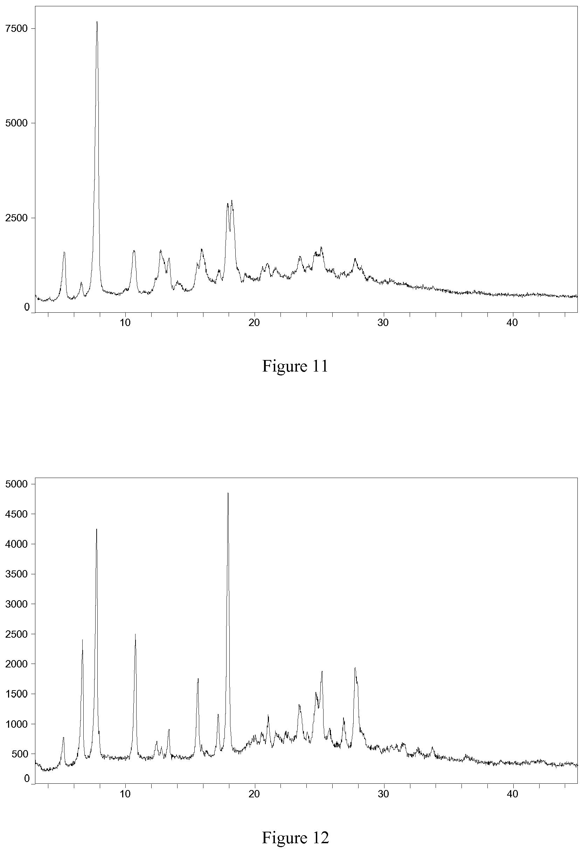

The most preferably, the X-ray powder diffraction spectrum comprises substantially the same peaks at diffraction angles (2θ) as shown in Figure 11, and the X-ray powder diffraction data are shown in table 8:

**Table 8**

| 2θ (°) | intensity % | 2θ (°) | intensity % |
|---|---|---|---|
| 5.3 | 18.2 | 19.3 | 3.4 |
| 7.9 | 100.0 | 20.6 | 4.6 |
| 10.6 | 15.6 | 20.9 | 10.5 |
| 12.8 | 26.2 | 21.8 | 6.0 |
| 13.4 | 11.5 | 23.1 | 3.5 |
| 15.9 | 27.5 | 24.7 | 16.5 |
| 16.5 | 6.3 | 25.1 | 4.0 |
| 17.3 | 4.2 | 26.2 | 4.6 |
| 18.3 | 62.0 | 27.4 | 9.5 |
| 18.7 | 9.4 | | |

The invention provides crystal form II of phosphate of the compound of formula I having an X-ray powder diffraction spectrum comprising peaks at diffraction angles (2θ) of 13.7±0.2°, 16.1±0.2°, 22.8±0.2° and 26.1±0.2°.

Preferably, the X-ray powder diffraction spectrum also comprises peaks at diffraction angles (2θ) of 11.0±0.2°, 14.6±0.2°, 20.3±0.2°, 20.8±0.2° and 25.7±0.2°.

More preferably, the X-ray powder diffraction spectrum also comprises peaks at diffraction angles (2θ) of 6.8±0.2°, 17.0±0.2°, 22.2±0.2°, 26.6±0.2°, 27.9±0.2° and 31.2±0.2°.

The most preferably, the X-ray powder diffraction spectrum comprises substantially the same peaks at diffraction angles (2θ) as shown in Figure 12, and the X-ray powder diffraction data are shown in table 9:

**Table 9**

| 2θ (°) | intensity % | 2θ (°) | intensity % |
|---|---|---|---|
| 6.8 | 26.3 | 21.5 | 11.6 |
| 8.8 | 6.1 | 22.2 | 20.1 |
| 11.0 | 71.5 | 22.8 | 100.0 |
| 12.4 | 12.9 | 24.6 | 16.3 |
| 13.7 | 87.5 | 25.7 | 47.1 |
| 14.6 | 30.8 | 26.1 | 94.2 |
| 16.1 | 74.6 | 26.6 | 30.0 |
| 17.0 | 30.3 | 27.9 | 26.4 |
| 20.3 | 32.2 | 30.7 | 14.1 |
| 20.8 | 34.1 | 31.2 | 21.7 |

The invention provides crystal form III of phosphate of the compound of formula I having an X-ray powder diffraction spectrum comprising peaks at diffraction angles (2θ) of 9.7±0.2°, 15.6±0.2°, 16.8±0.2° and 24.6±0.2°.

Preferably, the X-ray powder diffraction spectrum also comprises peaks at diffraction angles (2θ) of 4.8±0.2°, 21.2±0.2°, 25.0±0.2°, 27.8±0.2° and 28.1±0.2°.

More preferably, the X-ray powder diffraction spectrum also comprises peaks at diffraction angles (2θ) of 5.2±0.2°, 12.8±0.2°, 14.5±0.2°, 18.0±0.2°, 20.1±0.2° and 23.5±0.2°.

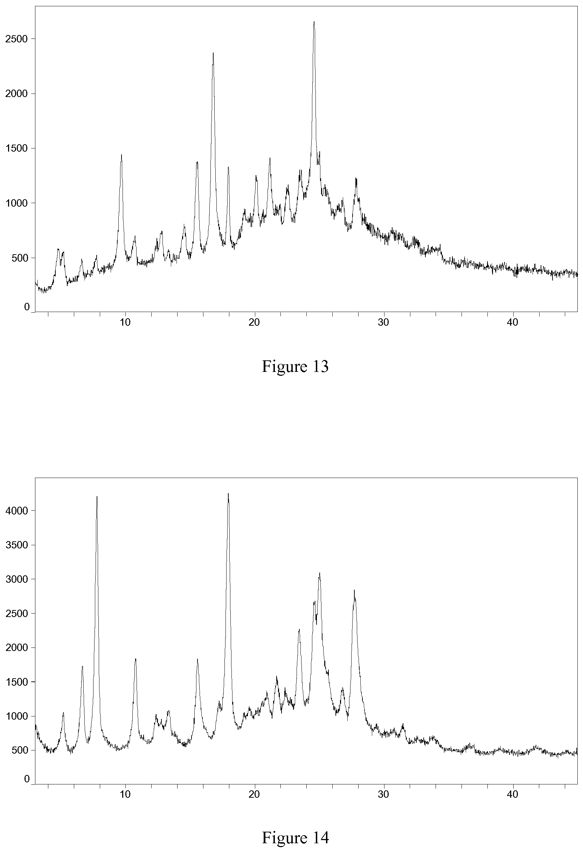

The most preferably, the X-ray powder diffraction spectrum comprises substantially the same peaks at diffraction angles (2θ) as shown in Figure 13, and the X-ray powder diffraction data are shown in table 10:

**Table 10**

| 2θ (°) | intensity % | 2θ (°) | intensity % |
|---|---|---|---|
| 4.8 | 26.0 | 19.2 | 11.7 |
| 5.2 | 20.8 | 20.1 | 16.5 |
| 7.8 | 5.2 | 21.2 | 36.9 |
| 9.7 | 49.4 | 22.6 | 12.2 |
| 10.7 | 6.1 | 23.5 | 12.5 |
| 12.4 | 10.1 | 24.6 | 86.0 |
| 12.8 | 21.4 | 25.0 | 39.2 |
| 14.5 | 14.7 | 26.8 | 11.3 |
| 15.6 | 41.0 | 27.8 | 29.3 |
| 16.8 | 100.0 | 28.1 | 24.0 |
| 18.0 | 21.0 | | |

The invention provides crystal form IV of phosphate of the compound of formula I having an X-ray powder diffraction spectrum comprising peaks at diffraction angles (2θ) of 7.8±0.2°, 17.9±0.2°, 25.0±0.2° and 27.7±0.2°.

Preferably, the X-ray powder diffraction spectrum also comprises peaks at diffraction angles (2θ) of 6.7±0.2°, 10.8±0.2°, 15.6±0.2°, 23.4±0.2° and 24.6±0.2°.

More preferably, the X-ray powder diffraction spectrum also comprises peaks at diffraction angles (2θ) of 5.2±0.2°, 12.8±0.2°, 20.9±0.2°, 21.7±0.2°, 22.3±0.2° and 26.8±0.2°.

The most preferably, the X-ray powder diffraction spectrum comprises substantially the same peaks at diffraction angles (2θ) as shown in Figure 14, and the X-ray powder diffraction data are shown in table 11:

**Table 11**

| 2θ (°) | intensity % | 2θ (°) | intensity % |
|---|---|---|---|
| 5.2 | 13.9 | 19.6 | 5.1 |
| 6.7 | 24.2 | 20.9 | 10.8 |
| 7.8 | 76.3 | 21.7 | 10.5 |
| 10.8 | 31.8 | 22.3 | 7.8 |
| 12.4 | 5.6 | 23.4 | 25.6 |
| 12.8 | 17.4 | 24.6 | 47.5 |
| 13.3 | 6.3 | 25.0 | 84.6 |
| 15.6 | 36.9 | 26.8 | 7.7 |
| 17.9 | 100.0 | 27.7 | 70.6 |

The invention provides a polymorph of mesylate of the compound of formula I, comprising five crystal forms, referred to as crystal form I, crystal form II, crystal form III, crystal form IV and crystal form V respectively.

The invention provides crystal form I of mesylate of the compound of formula I having an X-ray powder diffraction spectrum comprising peaks at diffraction angles (2θ) of 15.6±0.2°, 17.0±0.2°, 25.6±0.2° and 26.0±0.2°.

Preferably, the X-ray powder diffraction spectrum also comprises peaks at diffraction angles (2θ) of 9.8±0.2°, 21.8±0.2°, 23.5±0.2°, 23.8±0.2° and 27.5±0.2°.

More preferably, the X-ray powder diffraction spectrum also comprises peaks at diffraction angles (2θ) of 6.6±0.2°, 15.3±0.2°, 17.2±0.2°, 18.3±0.2°, 19.7±0.2° and 26.4±0.2°.

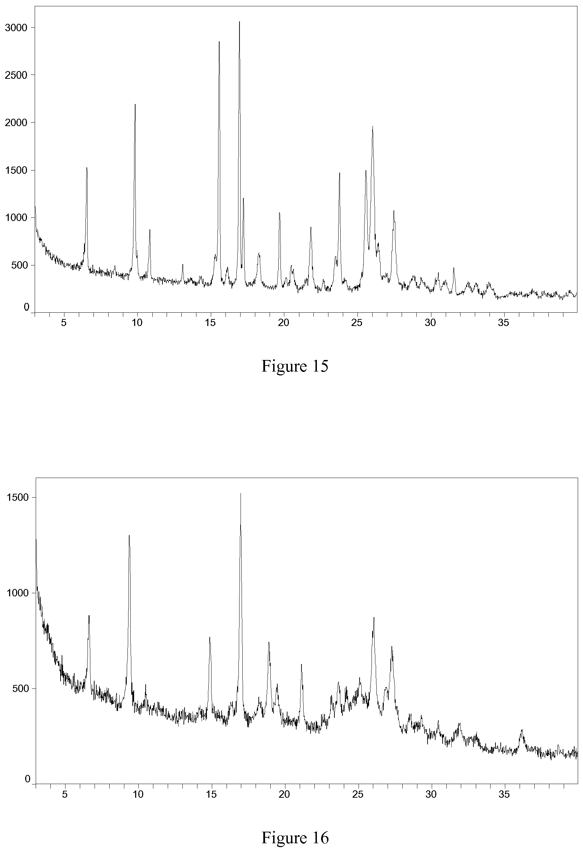

The most preferably, the X-ray powder diffraction spectrum comprises substantially the same peaks at diffraction angles (2θ) as shown in Figure 15, and the X-ray powder diffraction data are shown in table 12:

**Table 12**

| 2θ (°) | intensity % | 2θ (°) | intensity % |
|---|---|---|---|
| 6.6 | 27.5 | 23.5 | 32.6 |
| 9.8 | 46.7 | 23.8 | 53.6 |
| 10.9 | 13.5 | 25.6 | 58.2 |
| 13.1 | 5.6 | 26.0 | 100.0 |
| 15.3 | 15.2 | 26.4 | 15.8 |
| 15.6 | 71.4 | 27.5 | 34.0 |
| 16.2 | 5.8 | 28.9 | 9.5 |
| 17.0 | 66.8 | 29.3 | 10.8 |
| 17.2 | 21.7 | 30.5 | 11.7 |
| 18.3 | 17.9 | 31.0 | 10.2 |
| 19.7 | 18.9 | 31.6 | 7.3 |
| 20.1 | 7.7 | 32.5 | 11.2 |
| 20.5 | 14.5 | 33.1 | 6.5 |
| 21.5 | 6.3 | 34.0 | 10.0 |
| 21.8 | 29.8 | | |

The invention provides crystal form II of mesylate of the compound of formula I having an powder diffraction spectrum comprising peaks at diffraction angles (2θ) of 9.4±0.2°, 17.0±0.2°, 18.9±0.2° and 27.3±0.2°.

Preferably, the X-ray powder diffraction spectrum also comprises peaks at diffraction angles (2θ) of 6.6±0.2°, 14.9±0.2°, 21.1±0.2°, 26.1±0.2° and 26.9±0.2°.

More preferably, the X-ray powder diffraction spectrum comprises substantially the same peaks at diffraction angles (2θ) as shown in Figure 16, and the X-ray powder diffraction data are shown in table 13:

**Table 13**

| 2θ (°) | intensity % | 2θ (°) | intensity % |
|---|---|---|---|
| 6.6 | 33.4 | 21.1 | 22.5 |
| 9.4 | 66.5 | 23.7 | 19.8 |
| 14.9 | 42.7 | 26.1 | 46.3 |
| 17.0 | 100.0 | 26.9 | 34.7 |
| 18.9 | 61.2 | 27.3 | 61.6 |
| 19.5 | 20.1 | 36.1 | 15.3 |

The invention provides crystal form III of mesylate of the compound of formula I having an X-ray powder diffraction spectrum comprising peaks at diffraction angles (2θ) of 16.7±0.2°, 19.3±0.2°, 23.2±0.2° and 26.5±0.2°.

Preferably, the X-ray powder diffraction spectrum also comprises peaks at diffraction angles (2θ) of 8.7±0.2°, 19.5±0.2°, 21.8±0.2°, 23.6±0.2° and 24.3±0.2°.

More preferably, the X-ray powder diffraction spectrum also comprises peaks at diffraction angles (2θ) of 11.7±0.2°, 13.6±0.2°, 14.1±0.2°, 17.2±0.2°, 18.7±0.2° and 27.2±0.2°.

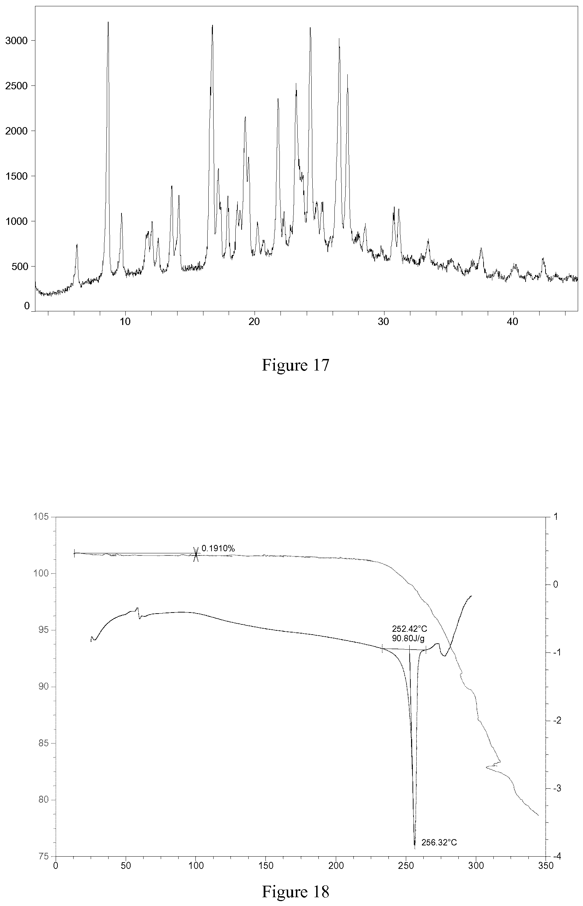

The most preferably, the X-ray powder diffraction spectrum comprises substantially the same peaks at diffraction angles (2θ) as shown in Figure 17, and the X-ray powder diffraction data are shown in table 14:

**Table 14**

| 2θ (°) | intensity % | 2θ (°) | intensity % |
|---|---|---|---|
| 6.3 | 12.5 | 21.8 | 50.8 |
| 8.7 | 71.0 | 22.3 | 10.6 |
| 9.7 | 17.0 | 23.2 | 72.7 |
| 11.7 | 20.7 | 23.6 | 56.4 |
| 12.0 | 20.6 | 24.3 | 58.6 |
| 12.5 | 5.4 | 24.8 | 12.3 |
| 13.6 | 28.1 | 25.2 | 7.1 |
| 14.1 | 23.9 | 26.5 | 80.7 |
| 16.7 | 100.0 | 27.2 | 40.6 |
| 17.2 | 40.2 | 28.5 | 6.8 |
| 17.9 | 13.0 | 30.8 | 15.4 |
| 18.7 | 21.6 | 31.1 | 15.4 |
| 19.3 | 74.2 | 33.4 | 14.1 |
| 19.5 | 53.0 | 36.8 | 7.0 |
| 20.2 | 7.6 | 37.5 | 14.4 |

The invention provides crystal form IV of mesylate of the compound of formula I having an X-ray powder diffraction spectrum comprising peaks at diffraction angles (2θ) of 16.8±0.2°, 19.1±0.2°, 19.3±0.2° and 22.1±0.2°.

Preferably, the X-ray powder diffraction spectrum also comprises peaks at diffraction angles (2θ) of 21.9±0.2°, 23.2±0.2°, 24.4±0.2°, 26.0±0.2° and 27.2±0.2°.

More preferably, the X-ray powder diffraction spectrum also comprises peaks at diffraction angles (2θ) of 8.7±0.2°, 13.4±0.2°, 13.6±0.2°, 19.6±0.2°, 21.6±0.2° and 26.6±0.2°.

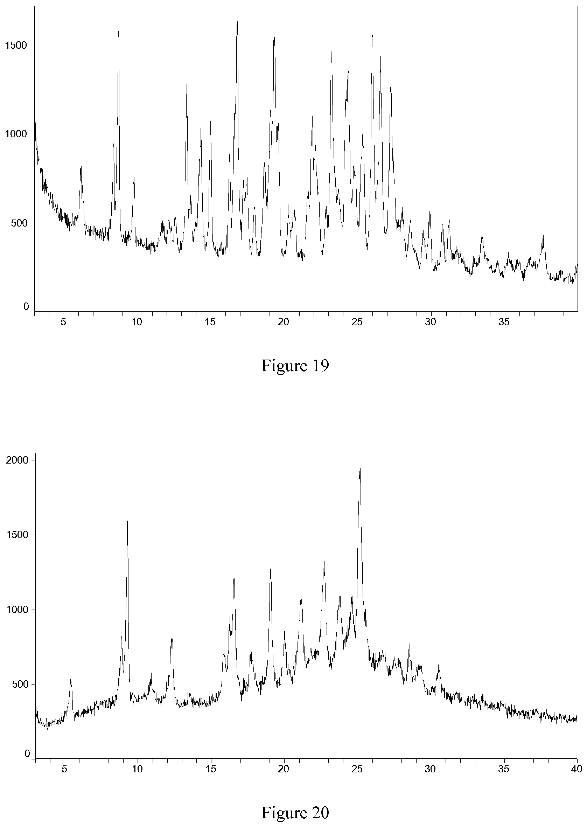

The most preferably, the X-ray powder diffraction spectrum comprises substantially the same peaks at diffraction angles (2θ) as shown in Figure 19, and the X-ray powder diffraction data are shown in table 15:

**Table 15**

| 2θ (°) | intensity % | 2θ (°) | intensity % |
|---|---|---|---|
| 6.2 | 12.7 | 20.7 | 12.8 |
| 8.4 | 20.3 | 21.6 | 26.1 |
| 8.7 | 28.7 | 21.9 | 56.8 |
| 9.8 | 7.9 | 22.1 | 59.1 |
| 12.1 | 11.2 | 23.2 | 50.7 |
| 12.6 | 6.2 | 23.7 | 17.6 |
| 13.4 | 26.3 | 24.4 | 43.1 |
| 13.6 | 22.7 | 24.8 | 21.1 |
| 14.3 | 22.1 | 25.3 | 20.2 |
| 15.0 | 19.0 | 26.0 | 43.4 |
| 16.3 | 8.7 | 26.6 | 40.7 |
| 16.8 | 60.9 | 27.2 | 40.9 |
| 17.5 | 12.6 | 28.6 | 8.4 |
| 18.0 | 6.0 | 29.5 | 7.9 |
| 18.7 | 5.3 | 29.9 | 10.2 |
| 19.1 | 78.3 | 30.8 | 5.3 |
| 19.3 | 100.0 | 31.2 | 15.5 |
| 19.6 | 37.1 | 33.4 | 13.1 |
| 20.3 | 11.0 | 37.6 | 8.5 |

The invention provides crystal form V of mesylate of the compound of formula I having an X-ray powder diffraction spectrum comprising peaks at diffraction angles (2θ) of 25.2±0.2°, 9.3±0.2°, 16.6±0.2° and 19.1±0.2°.

Preferably, the X-ray powder diffraction spectrum also comprises peaks at diffraction angles (2θ) of 22.7±0.2°, 16.3±0.2°, 21.2±0.2°, 8.9±0.2° and 12.3±0.2°.

More preferably, the X-ray powder diffraction spectrum also comprises peaks at diffraction angles (2θ) of 23.7±0.2°, 20.0±0.2°, 15.9±0.2°, 24.6±0.2°, 28.6±0.2° and 25.5±0.2°.

The most preferably, the X-ray powder diffraction spectrum comprises substantially the same peaks at diffraction angles (2θ) as shown in Figure 20, and the X-ray powder diffraction data are shown in table 16:

**Table 16**

| 2θ (°) | intensity % | 2θ (°) | intensity % |
|---|---|---|---|
| 5.4 | 19.9 | 20.0 | 25.9 |
| 8.9 | 35.8 | 21.2 | 37.3 |
| 9.3 | 96.9 | 22.7 | 54.1 |
| 10.9 | 15.0 | 23.7 | 28.9 |
| 12.3 | 34.9 | 24.6 | 23.1 |
| 15.9 | 23.4 | 25.2 | 100.0 |
| 16.3 | 42.9 | 25.5 | 20.1 |
| 16.6 | 62.2 | 28.6 | 20.9 |
| 17.8 | 19.8 | 30.5 | 13.7 |
| 19.1 | 61.6 | | |

The invention provides a polymorph of p-toluenesulfonate of the compound of formula I, comprising three crystal forms, referred to as crystal form I, crystal form II and crystal form III respectively.

The invention provides crystal form I of p-toluenesulfonate of the compound of formula I having an X-ray powder diffraction spectrum comprising peaks at diffraction angles (2θ) of 13.0±0.2°, 15.4±0.2°, 24.3±0.2° and 25.7±0.2°.

Preferably, the X-ray powder diffraction spectrum also comprises peaks at diffraction angles (2θ) of 5.3±0.2°, 12.1±0.2°, 18.4±0.2°, 22.6±0.2° and 23.2±0.2°.

More preferably, the X-ray powder diffraction spectrum also comprises peaks at diffraction angles (2θ) of 14.6±0.2°, 16.9±0.2°, 18.8±0.2°, 19.9±0.2°, 25.3±0.2° and 29.3±0.2°.

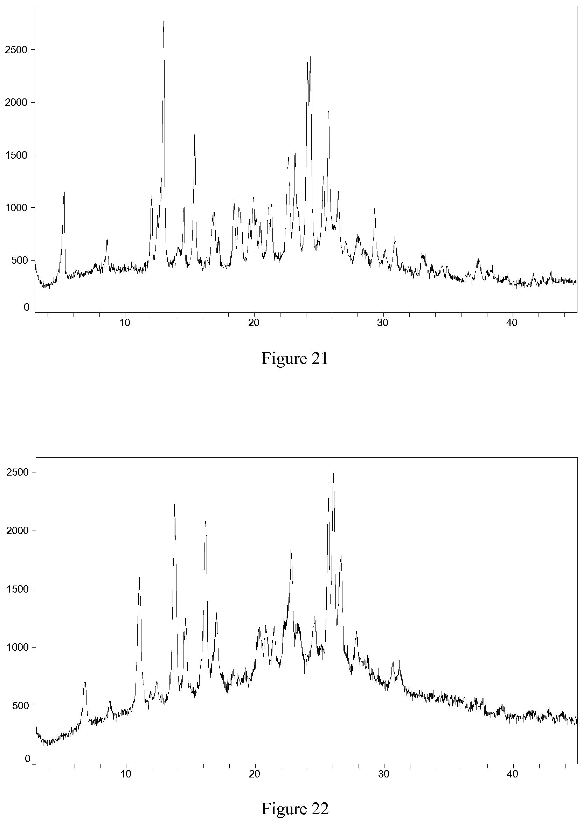

The most preferably, X-ray powder diffraction spectrum comprises substantially the same peaks at diffraction angles (2θ) as shown in Figure 21, and the X-ray powder diffraction data are shown in table 17:

**Table 17**

| 2θ (°) | intensity % | 2θ (°) | intensity % |
|---|---|---|---|
| 5.3 | 37.0 | 22.6 | 40.5 |
| 8.6 | 12.9 | 23.2 | 40.5 |
| 12.1 | 27.0 | 23.4 | 13.1 |
| 12.5 | 20.9 | 24.3 | 79.4 |
| 13.0 | 100.0 | 25.3 | 22.6 |
| 14.1 | 7.0 | 25.7 | 56.9 |
| 14.6 | 23.5 | 26.5 | 21.1 |
| 15.4 | 55.0 | 28.1 | 10.8 |
| 16.9 | 22.9 | 29.3 | 23.1 |
| 17.2 | 11.7 | 30.1 | 7.0 |
| 18.4 | 26.3 | 30.9 | 13.4 |
| 18.8 | 22.8 | 33.0 | 9.0 |
| 19.6 | 16.0 | 33.2 | 8.2 |
| 19.9 | 25.4 | 37.3 | 8.6 |
| 20.5 | 13.9 | 38.4 | 6.0 |
| 21.3 | 21.9 | | |

The invention provides crystal form II of p-toluenesulfonate of the compound of formula I having an X-ray powder diffraction spectrum comprising peaks at diffraction angles (2θ) of 13.7±0.2°, 16.1±0.2°, 25.7±0.2° and 26.1±0.2°.

Preferably, the X-ray powder diffraction spectrum also comprises peaks at diffraction angles (2θ) of 11.0±0.2°, 14.6±0.2°, 17.0±0.2°, 22.8±0.2° and 26.6±0.2°.

More preferably, the X-ray powder diffraction spectrum also comprises peaks at diffraction angles (2θ) of 6.8±0.2°, 20.3±0.2°, 20.8±0.2°, 22.2±0.2°, 24.6±0.2° and 27.9±0.2°.

The most preferably, the X-ray powder diffraction spectrum comprises substantially the same peaks at diffraction angles (2θ) as shown in Figure 22, and the X-ray powder diffraction data are shown in table 18:

**Table 18**

| 2θ (°) | intensity % | 2θ (°) | intensity % |
|---|---|---|---|
| 6.8 | 23.9 | 21.5 | 18.0 |
| 8.8 | 8.8 | 22.2 | 19.8 |
| 11.0 | 69.4 | 22.8 | 60.2 |
| 12.4 | 11.2 | 24.6 | 20.4 |
| 13.7 | 100.0 | 25.7 | 74.2 |
| 14.6 | 37.5 | 26.1 | 95.5 |
| 16.1 | 83.8 | 26.6 | 42.6 |
| 17.0 | 33.9 | 27.9 | 21.4 |
| 20.3 | 22.7 | 30.7 | 13.0 |
| 20.8 | 20.8 | 31.2 | 16.1 |

The invention provides crystal form III of p-toluenesulfonate of the compound of formula I having an X-ray powder diffraction spectrum comprising peaks at diffraction angles (2θ) of 8.2±0.2°, 14.4±0.2°, 25.9±0.2° and 26.3±0.2°.

Preferably, the X-ray powder diffraction spectrum also comprises peaks at diffraction angles (2θ) of 10.3±0.2°, 12.8±0.2°, 17.2±0.2°, 18.0±0.2° and 19.9±0.2°.

More preferably, the X-ray powder diffraction spectrum also comprises peaks at diffraction angles (2θ) of 4.8±0.2°, 13.2±0.2°, 15.1±0.2°, 19.3±0.2°, 24.2±0.2° and 24.5±0.2°.

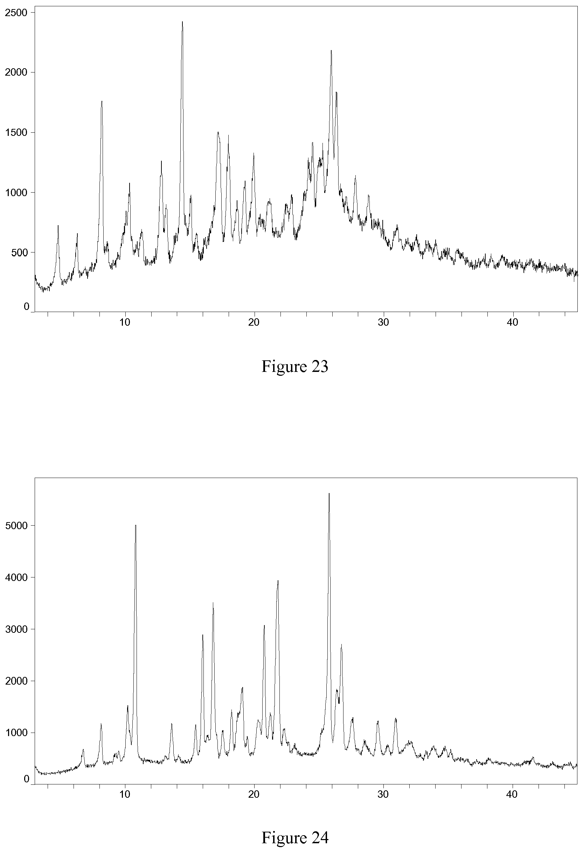

The most preferably, the X-ray powder diffraction spectrum comprises substantially the same peaks at diffraction angles (2θ) as shown in Figure 23, and the X-ray powder diffraction data are shown in table 19:

**Table 19**

| 2θ (°) | intensity % | 2θ (°) | intensity % |
|---|---|---|---|
| 4.8 | 25.3 | 19.3 | 24.8 |
| 6.3 | 19.0 | 19.9 | 34.3 |
| 8.2 | 72.1 | 21.2 | 15.3 |
| 8.6 | 10.5 | 22.4 | 13.9 |
| 9.8 | 11.3 | 22.9 | 17.9 |
| 10.0 | 18.1 | 23.9 | 13.2 |
| 10.3 | 31.2 | 24.2 | 24.9 |
| 11.3 | 12.0 | 24.5 | 28.3 |
| 12.8 | 43.1 | 25.0 | 16.4 |
| 13.2 | 23.5 | 25.3 | 21.8 |
| 14.4 | 100.0 | 25.9 | 63.2 |
| 15.1 | 23.1 | 26.3 | 48.3 |
| 17.2 | 46.5 | 27.8 | 20.3 |
| 18.0 | 45.0 | 28.8 | 14.6 |
| 18.7 | 17.4 | 31.0 | 10.1 |

The invention provides a polymorph of 1,5-naphthalenedisulfonate of the compound of formula I, comprising three crystal forms, referred to as crystal form I, crystal form II and crystal form III respectively.

The invention provides crystal form I of 1,5-naphthalenedisulfonate of the compound of formula I having an X-ray powder diffraction spectrum comprising peaks at diffraction angles (2θ) of 10.8±0.2°, 16.8±0.2°, 21.8±0.2° and 25.8±0.2°.

Preferably, the X-ray powder diffraction spectrum also comprises peaks at diffraction angles (2θ) of 10.2±0.2°, 16.0±0.2°, 19.1±0.2°, 20.8±0.2° and 26.7±0.2°.

More preferably, the X-ray powder diffraction spectrum also comprises peaks at diffraction angles (2θ) of 8.1±0.2°, 13.6±0.2°, 18.2±0.2°, 18.7±0.2°, 26.4±0.2° and 30.9±0.2°.

The most preferably, the X-ray powder diffraction spectrum comprises substantially the same peaks at diffraction angles (2θ) as shown in Figure 24, and the X-ray powder diffraction data are shown in table 20:

**Table 20**

| 2θ (°) | intensity % | 2θ (°) | intensity % |
|---|---|---|---|
| 6.8 | 7.2 | 20.8 | 46.8 |
| 8.1 | 16.4 | 21.2 | 10.2 |
| 10.2 | 22.1 | 21.8 | 63.7 |
| 10.8 | 94.5 | 25.2 | 7.8 |
| 13.6 | 15.9 | 25.8 | 100.0 |
| 15.5 | 12.7 | 26.4 | 21.4 |
| 16.0 | 51.0 | 26.7 | 40.6 |
| 16.4 | 7.9 | 27.6 | 13.3 |
| 16.8 | 60.0 | 28.5 | 5.5 |
| 17.6 | 9.7 | 29.6 | 13.5 |
| 18.2 | 15.0 | 30.9 | 14.1 |
| 18.7 | 16.7 | 31.8 | 3.6 |
| 19.1 | 25.4 | 32.1 | 5.8 |
| 19.4 | 6.9 | 33.9 | 4.6 |
| 20.3 | 10.8 | 34.8 | 4.9 |

The invention provides crystal form II of 1,5-naphthalenedisulfonate of the compound of formula I having an X-ray powder diffraction spectrum comprising peaks at diffraction angles (2θ) of 4.2±0.2°, 16.4±0.2°, 22.8±0.2° and 27.3±0.2°.

Preferably, the X-ray powder diffraction spectrum also comprises peaks at diffraction angles (2θ) of 8.5±0.2°, 17.8±0.2°, 19.1±0.2°, 22.3±0.2° and 28.1±0.2°.

More preferably, the X-ray powder diffraction spectrum also comprises peaks at diffraction angles (2θ) of 10.4±0.2°, 13.5±0.2°, 15.1±0.2°, 21.2±0.2°, 24.0±0.2° and 26.5±0.2°.

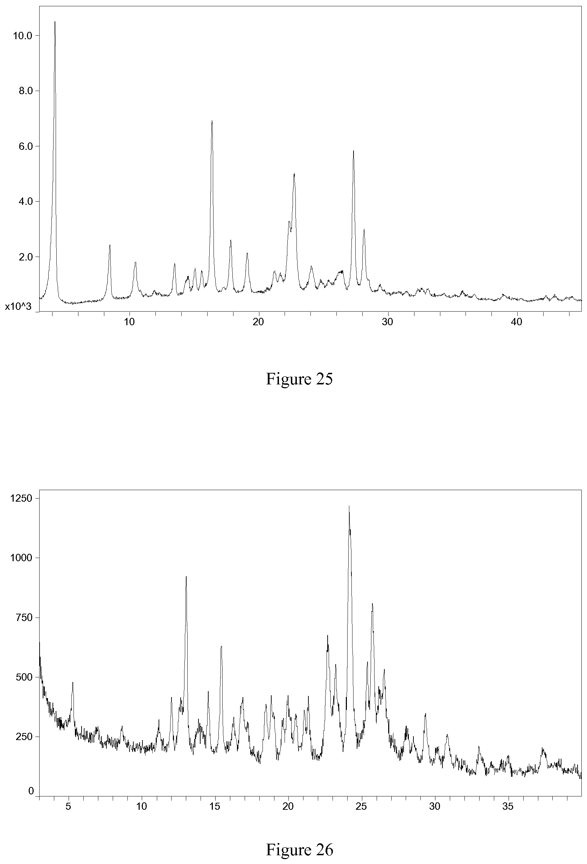

The most preferably, the X-ray powder diffraction spectrum comprises substantially the same peaks at diffraction angles (2θ) as shown in Figure 25, and the X-ray powder diffraction data are shown in table 21:

**Table 21**

| 2θ (°) | intensity % | 2θ (°) | intensity % |
|---|---|---|---|
| 4.2 | 100.0 | 22.3 | 22.2 |
| 8.5 | 19.4 | 22.8 | 40.2 |
| 10.4 | 12.7 | 24.0 | 8.1 |
| 13.5 | 11.2 | 26.5 | 6.0 |
| 15.1 | 7.9 | 27.3 | 48.6 |
| 16.4 | 60.9 | 28.1 | 20.9 |
| 17.8 | 18.5 | 28.5 | 3.6 |
| 19.1 | 14.0 | 32.6 | 2.8 |
| 21.2 | 5.4 | | |

The invention provides crystal form III of 1,5-naphthalenedisulfonate of the compound of formula I having an X-ray powder diffraction spectrum comprising peaks at diffraction angles (2θ) of 13.0±0.2°, 22.7±0.2°, 24.1±0.2° and 25.7±0.2°.

Preferably, the X-ray powder diffraction spectrum also comprises peaks at diffraction angles (2θ) of 15.4±0.2° 18.8±0.2°, 23.2±0.2°, 25.4±0.2° and 26.5±0.2°.

More preferably, the X-ray powder diffraction spectrum also comprises peaks at diffraction angles (2θ) of 12.6±0.2°, 14.5±0.2°, 16.9±0.2°, 18.5±0.2°, 20.0±0.2° and 21.4±0.2°.

The most preferably, the X-ray powder diffraction spectrum comprises substantially the same peaks at diffraction angles (2θ) as shown in Figure 26, and the X-ray powder diffraction data are shown in table 22:

**Table 22**

| 2θ (°) | intensity % | 2θ (°) | intensity % |
|---|---|---|---|
| 5.3 | 20.4 | 20.5 | 12.9 |
| 11.2 | 12.2 | 21.1 | 16.3 |
| 12.0 | 20.7 | 21.4 | 23.3 |
| 12.6 | 21.4 | 22.7 | 48.7 |
| 13.0 | 72.2 | 23.2 | 34.9 |
| 13.9 | 12.8 | 24.1 | 100.0 |
| 14.5 | 22.4 | 25.4 | 27.7 |
| 15.4 | 43.7 | 25.7 | 49.9 |
| 16.3 | 12.3 | 26.2 | 20.3 |
| 16.9 | 23.6 | 26.5 | 36.5 |
| 17.2 | 12.5 | 28.0 | 13.1 |
| 18.5 | 22.5 | 29.3 | 20.0 |
| 18.8 | 25.6 | 30.8 | 11.7 |
| 19.7 | 14.1 | 33.0 | 10.8 |
| 20.0 | 23.0 | 37.3 | 8.7 |

The term "substantially the same" related to X-ray diffraction peak position used herein means to consider the typical peak position and intensity variability. For example, those skilled in the art will understand that the measured values of the peak positions (2θ) will be changed due to the different XRPD instruments, sometimes this change may reach up to 0.2°. Moreover, those skilled in the art will understand that preparation method of XRPD sample, XRPD instrument, crystallinity of sample, sample amount, preferred orientation of the crystal and other factors will cause the change of relative peak intensity of the sample in XRPD spectrum.

In another aspect, the invention provides a method for preparing a crystalline free base of 9-((8-fluoro-6-(1-methyl-1H-pyrazol-4-yl)-[1,2,4]triazolo[4,3-a]pyridine-3-yl)thio)-4-m ethyl-2H-[1,4]oxaazido[3,2-c]quinoline-3(4H)-one, comprising the following steps of:
dissolving 9-((8-fluorine-6-(1-methyl-1H-pyrazol-4-yl)-[1,2,4]triazolo[4,3-a]pyridin-3-yl)thio)-4-methyl-2H-[1,4]oxaazino[3,2-c]quinoline-3(4H)-one in an aqueous solvent, an organic solvent or a solvent mixture under heating, then cooling the solution or mixing the solution with an anti-solvent to obtain the crystalline free base; or
evaporating a solution or suspension of 9-((8-fluorine-6-(1-methyl-1H-pyrazol-4-yl)-[1,2,4]triazolo[4,3-a]pyridin-3-yl)thio)-4-methyl-2H-[1,4]oxaazino[3,2-c]quinoline-3(4H)-one rapidly or slowly to obtain the crystalline free base; or
adding an original compound solid or other solid particle additive as a heteronuclear crystal seed to a solution of 9-((8-fluorine-6-(1-methyl-1H-pyrazol-4-yl)-[1,2,4]triazolo[4,3-a]pyridin-3-yl)thio)-4-methyl-2H-[1,4]oxaazino[3,2-c]quinoline-3(4H)-one to induce the crystalline free base; or
dispersing 9-((8-fluorine-6-(1-methyl-1H-pyrazol-4-yl)-[1,2,4]triazolo[4,3-a]pyridin-3-yl)thio)-4-methyl-2H-[1,4]oxaazino[3,2-c]quinoline-3(4H)-one in an aqueous solvent, an organic solvent or a solvent mixture or an atmosphere of these media to obtain the crystalline free base; or
heating, sublimating, grinding, freezing or fusing-cooling 9-((8-fluorine-6-(1-methyl-1H-pyrazol-4-yl)-[1,2,4]triazolo[4,3-a]pyridin-3-yl)thio)-4-methyl-2H-[1,4]oxaazino[3,2-c]quinoline-3(4H)-one to obtain the crystalline free base; or combining the above methods to obtain the crystalline free base.

The present invention provides, but is not limited to, the following methods, for example, the organic solvent (if it exists) refers to, but is not limited to, the following solvent: alcohols, chloralkanes, ketones, ethers, cyclic ethers, esters, alkanes, cycloalkanes, benzenes, amides, sulfoxides or the mixture thereof, preferably is selected from the group consisting of methanol, ethanol, n-propanol, isopropanol, n-butanol, acetonitrile, acetone, methyl ethyl ketone, tetrahydrofuran, dioxane, N,N-dimethylcarboxamide, dimethyl sulfoxide, ethyl acetate, dichloromethane, trichloroethane, carbon tetrachloride, n-heptane, n-hexane, iso-octane, pentane, cyclohexane, cyclopentane, diethyl ether, methyltertbutyl ether, isopropyl ether, benzene, toluene, xylene and the mixture thereof; supercritical fluid such as liquid carbon dioxide, ionic liquid, polymer solution and the like.

### For example:

### 1) Preparation of crystal form I of free base

A suitable solvent (including, but not limited to, isopropanol, isopropyl acetate, acetonitrile, tetrahydrofuran, 2-methoxyethanol or the mixture thereof) is added to a free base of the compound of formula I, the free base is dispersed in the solvent to form a suspension (1-200 mg/mL). The suspension is stirred until the transformation into crystal form I of free base is completed, a solid-liquid separation is carried out to obtain crystal form I of free base.

### 2) Preparation of crystal form II of free base

A suitable solvent (including, but not limited to, methanol, ethanol, acetone, dichlormethane or the mixture thereof) is added to a free base of the compound of formula I, the free base is dispersed in the solvent to form a suspension (1-200 mg/mL). The suspension is stirred until the transformation into crystal form II of free base is completed, a solid-liquid separation is carried out to obtain crystal form II of free base.

### 3) Preparation of crystal form III of free base

Acetonitrile is added to a free base of the compound of formula I, the free base is dispersed in acetonitrile to form a suspension (1-200 mg/mL). The suspension is stirred at 40-60°C until the transformation into crystal form III of free base is completed, a solid-liquid separation is carried out to obtain crystal form III of free base.

### 4) Preparation of crystal form IV of free base

A free base of the compound of formula I is heated to more than 100°C (preferably more than 120°C) and melted, and then slowly cooled to room temperature to obtain crystal form IV of free base.

In another aspect, the invention provides a method for preparing a crystalline acid salt of 9-((8-fluoro-6-(1-methyl-1H-pyrazol-4-yl)-[1,2,4]triazolo[4,3-a]pyridine-3-yl)thio)-4-m ethyl-2H-[1,4]oxaazido[3,2-c]quinoline-3(4H)-one, comprising the following steps of:
1) dissolving or dispersing a free base of 9-((8-fluoro-6-(1-methyl-1H-pyrazol-4-yl)-[1,2,4]triazolo[4,3-a]pyridine-3-yl)thio)-4-m ethyl-2H-[1,4]oxaazido[3,2-c]quinoline-3(4H)-one or dispersing in an aqueous solvent or a suitable organic solvent, then adding a liquid or solid inorganic acid or organic acid or a solution of inorganic acid or organic acid to the above system to obtain an acid salt of 9-((8-fluoro-6-(1-methyl-1H-pyrazol-4-yl)-[1,2,4]triazolo[4,3-a]pyridine-3-yl)thio)-4-m ethyl-2H-[1,4]oxaazido[3,2-c]quinoline-3(4H)-one; or
   adding a free base solid of 9-((8-fluoro-6-(1-methyl-1H-pyrazol-4-yl)-[1,2,4]triazolo[4,3-a]pyridine-3-yl)thio)-4-m ethyl-2H-[1,4]oxaazido[3,2-c]quinoline-3(4H)-one to an acid solution to obtain an acid salt of 9-((8-fluoro-6-(1-methyl-1H-pyrazol-4-yl)-[1,2,4]triazolo[4,3-a]pyridine-3-yl)thio)-4-m ethyl-2H-[1,4]oxaazido[3,2-c]quinoline-3(4H)-one;
2) collecting the solid product precipitated during the salt-forming process, or obtaining the solid product by creating supersaturation of the salt-forming system to prepare the crystalline acid salt of 9-((8-fluoro-6-(1-methyl-1H-pyrazol-4-yl)-[1,2,4]triazolo[4,3-a]pyridine-3-yl)thio)-4-m ethyl-2H-[1,4]oxaazido[3,2-c]quinoline-3(4H)-one, wherein a method for creating supersaturation comprises evaporation of solvent, or addition of an anti-solvent, or a cooling method;
and/or,
transforming one crystal form of the acid salt into another crystal form of the acid salt by a crystal transformation method, wherein the crystal transformation method comprises: heating or a crystal transformation method of a suspension in a suitable solvent;
wherein the suitable organic solvent of the salt-forming process in step 1) is selected from the group consisting of alcohols, chloralkanes, ketones, ethers, cyclic ethers, esters, alkanes, cycloalkanes, benzenes, amides, sulfoxides and the mixture thereof, preferably methanol, ethanol, n-propanol, isopropanol,acetonitrile, acetone, 1,4-dioxane, tetrahydrofuran, N,N-dimethylcarboxamide, ethyl acetate, isopropyl acetate, 2-methoxyethyl ether and the mixture thereof;
wherein the "acid salt" used herein refers to a suitable, pharmaceutically acceptable salt which formed by the compound of the present invention and an acid substance, wherein the acid salt comprises an inorganic acid salt or organic acid salt; wherein the inorganic acid salt was preferably selected from the group consisting of hydrochloride, sulfate, hydrobromide, hydrofluoride, hydroiodide and phosphate, more preferably hydrochloride, sulfate and phosphate; wherein the organic acid salt was preferably selected from the group consisting of 2,5-dihydroxybenzeneformate, 1-hydroxy-2-naphthaleneformate, acetate, dichloroacetate, trichloroacetate, acetyl oxygenoximoate, adipate, benzene sulfonate, 4-chlorobenzene sulfonate, benzeneformate, 4-acetamidobenzeneformate, 4-aminobenzeneformate, caprate, caproate, caprilate, cinnamoate, citrate, cyclohexylsulfamate , camphorsulfonate, aspartate, camphorate, gluconate, glucuronate, glutamate, erythorbate, lactate, aspartate, malate, mandelate, pyroglutamate, tartrate, lauryl sulfate, dibenzoyltartrate, ethyl-1,2-disulfonate, esylate, formate, fumarate, galactonate, gentisate, glutarate, 2-oneglutarate, glycollate, hippurate, isethionate, lactobionate , ascorbate, aspartate, laurate, camphorate, maleate, malonate, mesylate, 1,5-naphthalenedisulfonate, naphthalene-2-sulfonate, nicotinate, oleate, orotate, oxalate, palmitate, embonate, propionate, salicylate, 4-aminosalicylate, sebacate, stearate, butanedioate, thiocyanate, undecylenate, trifluoroacetate, succinate, and p-toluenesulfonate, more preferably mesylate, p-toluenesulfonate and 1,5-naphthalenedisulfonate.

### For example:

### 1) Preparation of crystal form I of hydrochloride

A suitable solvent (including, methanol, acetonitrile, acetone, ethyl acetate or the mixture thereof) is added to a free base of the compound of formula I, then an equal or excess molar equivalent of hydrochloric acid is added. The mixture is stirred, after the salt-forming reaction is completed, a solid-liquid separation is carried out to obtain crystal form I of hydrochloride.

### 2) Preparation of crystal form I of sulfate

A suitable solvent (including, acetonitrile, acetone or the mixture thereof) is added to a free base of the compound of formula I, then an equal or excess molar equivalent of sulfuric acid is added. The mixture is stirred, after the salt-forming reaction is completed, a solid-liquid separation is carried out to obtain crystal form I of sulfate.

### 3) Preparation of crystal form II of sulfate

An ethanol-water solution with volume ratio: 5%-95% is added to a free base of the compound of formula I, then an equal or excess molar equivalent of sulfuric acid is added. The mixture is stirred, after the salt-forming reaction is completed, a solid-liquid separation is carried out to obtain crystal form II of sulfate.

### 4) Preparation of crystal form I of phosphate

Methanol or an ethanol-water solution with volume ratio: 5%-95% is added to a free base of the compound of formula I, then an equal or excess molar equivalent of phosphoric acid is added. The mixture is stirred, after the salt-forming reaction is completed, a solid-liquid separation is carried out to obtain crystal form I of phosphate.

### 5) Preparation of crystal form II of phosphate

A suitable solvent (including, acetonitrile, ethyl acetate, tetrahydrofuran or the mixture thereof) is added to a free base of the compound of formula I, then an equal or excess molar equivalent of phosphoric acid is added. The mixture is stirred, after the salt-forming reaction is completed, a solid-liquid separation is carried out to obtain crystal form II of phosphate.

### 6) Preparation of crystal form III of phosphate

A suitable solvent (including, acetone) is added to a free base of the compound of formula I, then an equal or excess molar equivalent of phosphoric acid is added. The mixture is stirred, after the salt-forming reaction is completed, a solid-liquid separation is carried out to obtain crystal form III of phosphate.

### 7) Preparation of crystal form IV of phosphate

A suitable solvent (including, methanol) is added to a free base of the compound of formula I, then an equal or excess molar equivalent of phosphoric acid is added. The mixture is stirred, after the salt-forming reaction is completed, a solid-liquid separation is carried out to obtain crystal form IV of phosphate.

### 8) Preparation of crystal form I of mesylate

A suitable solvent (including, acetone, tetrahydrofuran, isopropyl acetate, ethyl acetate, 2-methoxyethyl ether, 1,4-dioxane or the mixture thereof) is added to a free base of the compound of formula I, then an equal or excess molar equivalent of methanesulfonic acid is added. The mixture is stirred, after the salt-forming reaction is completed, a solid-liquid separation is carried out to obtain crystal form I of mesylate.

### 9) Preparation of crystal form II of mesylate

A methanol-water solution with volume ratio: 5%-95% is added to a free base of the compound of formula I, then the mixture is stirred. An equal or excess molar equivalent of methanesulfonic acid is added until the solution is clear. After mesylate is precipitated, a solid-liquid separation is carried out to obtain crystal form II of mesylate.

### 10) Preparation of crystal form III of mesylate

Methanol is added to a free base of the compound of formula I, then an equal or excess molar equivalent of methanesulfonic acid is added slowly. After the compound is dissolved, a seed crystal of crystal form III of mesylate is added immediately, the mixture is stirred, after the salt-forming reaction is completed, a solid-liquid separation is carried out to obtain crystal form III of mesylate.

Or in the absence of a crystal seed, crystal form IV of mesylate is obtained firstly, then crystal form IV of mesylate is dried in vacuum at 100-120°C, overnight, crystal form IV of mesylate is transformed to crystal form III of mesylate.

Or crystal form V of mesylate is dispersed in a single or mixed anti-solvent, such as n-heptane / ethyl acetate solution, then the mixture is stirred at room temperature or under heating, crystal form V of mesylate is transformed to crystal form III of mesylate.

### 11) Preparation of crystal form IV of mesylate

Methanol is added to a free base of the compound of formula I, then an equal or excess molar equivalent of methanesulfonic acid is added. The mixture is stirred, after the salt-forming reaction is completed, a solid-liquid separation is carried out to obtain crystal form IV of mesylate.

### 12) Preparation of crystal form V of mesylate

Dimethyl sulfoxide (DMSO) is added to a free base of the compound of formula I, then an equal or excess molar equivalent of methanesulfonic acid is added. The mixture is stirred, after the salt-forming reaction is completed, a suitable amount of ethyl acetate is added and the stirring is continued, then a solid-liquid separation is carried out to obtain crystal form V of mesylate.

### 13) Preparation of crystal form I of p-toluenesulfonate

A suitable solvent (including, methanol, acetonitrile, acetone or the mixture thereof) is added to a free base of the compound of formula I, then an equal or excess molar equivalent of p-toluenesulfonic acid is added. The mixture is stirred, after the salt-forming reaction is completed, a solid-liquid separation is carried out to obtain crystal form I of p-toluenesulfonate.

### 14) Preparation of crystal form II of p-toluenesulfonate

A suitable solvent (including, ethyl acetate) is added to a free base of the compound of formula I, then an equal or excess molar equivalent of p-toluenesulfonic acid is added. The mixture is stirred, after the salt-forming reaction is completed, a solid-liquid separation is carried out to obtain crystal form II of p-toluenesulfonate.

### 15) Preparation of crystal form III of p-toluenesulfonate

An ethanol-water solution with volume ratio: 5%-95% is added to a free base of the compound of formula I, then an equal or excess molar equivalent of p-toluenesulfonic acid is added. The mixture is stirred, after the salt-forming reaction is completed, a solid-liquid separation is carried out to obtain crystal form III of p-toluenesulfonate.

### 16) Preparation of crystal form I of 1,5-naphthalenedisulfonate

A suitable solvent (including, methanol, acetonitrile, acetone or the mixture thereof) is added to a free base of the compound of formula I, then an equal or excess molar equivalent of 1,5-naphthalenedisulfonic acid is added. The mixture is stirred, after the salt-forming reaction is completed, a solid-liquid separation is carried out to obtain crystal form I of 1,5-naphthalenedisulfonate.

### 17) Preparation of crystal form II of 1,5-naphthalenedisulfonate

A suitable solvent (including, ethyl acetate) is added to a free base of the compound of formula I, then an equal or excess molar equivalent of 1,5-naphthalenedisulfonic acid is added. The mixture is stirred, after the salt-forming reaction is completed, a solid-liquid separation is carried out to obtain crystal form II of 1,5-naphthalenedisulfonate.

### 18) Preparation of crystal form III of 1,5-naphthalenedisulfonate

A suitable solvent (including, methanol) is added to a free base of the compound of formula I, then an equal or excess molar equivalent of 1,5-naphthalenedisulfonic acid is added. The mixture is stirred, after the salt-forming reaction is completed, a solid-liquid separation is carried out to obtain crystal form III of 1,5-naphthalenedisulfonate.

In another aspect, the invention provides a pharmaceutical composition, comprising a therapeutically effective amount of the crystalline free base or the crystalline acid salt of the compound of formula I, and a pharmaceutically acceptable carrier or excipient.

Above crystalline free base or polymorph thereof, the above crystalline acid salt or polymorph thereof of the compound of formula I or the above pharmaceutical composition can be used in the preparation of a medicament for the treatment of a protein kinase-related disease, wherein the protein kinase is selected from the group consisting of C-Met and VEGFR receptor tyrosine kinase.

There can also be a use in a method for modulating a catalytic activity of a protein kinase, comprising a step of contacting the protein kinase with the above crystalline free base or polymorph thereof, the above crystalline acid salt or polymorph thereof of compound of formula I, or the above pharmaceutical composition, wherein the protein kinase is selected from the group consisting of C-Met and VEGFR receptor tyrosine kinase.

In another aspect, the invention provides the above crystalline free base or polymorph thereof, the above crystalline acid salt or polymorph thereof of compound of formula I, or the above pharmaceutical composition for use in the treatment of cancer and metastasis, including cancer (solid tumor), hematopoietic tumor of lymphatic system, hematopoietic tumor of bone marrow system, mesenchymal tumor, central and peripheral nervous system tumor or other tumor. The cancer may be selected from the group consisting of bladder cancer, breast cancer, colon cancer, kidney cancer, liver cancer, stomach cancer, lung cancer (non-small cell lung cancer) and skin cancer; the hematopoietic tumor of lymphatic system is selected from the group consisting of leukemia, acute lymphocytic leukemia and chronic lymphocytic leukemia; the hematopoietic tumor of bone marrow system is selected from the group consisting of acute or chronic myelogenous leukemia, myelodysplastic syndrome and promyelocytic leukemia; the mesenchymal tumor is selected from the group consisting of fibrosarcoma, rhabdomyosarcoma, soft tissue sarcoma and osteosarcoma; the central and peripheral nervous system tumor is selected from the group consisting of astrocytoma, neuroblastoma, glioma and nerve ending tumor; and the other tumor is selected from the group consisting of malignant melanoma, seminoma, teratocarcinoma, thyroid follicular cancer and Kaposi's sarcoma.

Preferably, the invention provides use of the above crystalline free base or polymorph thereof, the above crystalline acid salt or polymorph thereof of compound of formula I, or the above pharmaceutical composition in the preparation of a medicament for the treatment of liver cancer, lung cancer, breast cancer, squamous cell carcinoma of the skin and stomach cancer.

### DESCRIPTION OF THE DRAWINGS

**FIGURE** 1 is the X-ray powder diffraction spectrum of crystal form I of free base of the compound of formula I; the abscissa is angle 2θ (°), and the ordinate is intensity.
**FIGURE** 2 is the DSC spectrum of crystal form I of free base of the compound of formula I; the abscissa is temperature (°C), and the ordinate is heat flow (W/g), the exothermic peak is downward; wherein the peak area of the shown peak: 121.2J/g, melting point: 276.8°C, onset temperature: 274.4°C, end temperature: 278.7°C, peak height: 6.716mW/mg.
**FIGURE** 3 is the TGA spectrum of crystal form I of free base of the compound of formula I; the abscissa is temperature (°C), and the ordinate is weightlessness ratio (%).
**FIGURE** 4 is the X-ray powder diffraction spectrum of crystal form II of free base of the compound of formula I; the abscissa is angle 2θ (°), and the ordinate is intensity.
**FIGURE 5** is the X-ray powder diffraction spectrum of crystal form III of free base of the compound of formula I; the abscissa is angle 2θ (°), and the ordinate is intensity.
**FIGURE 6** is the DSC/TGA stacking spectrum of crystal form III of free base of the compound of formula I; the abscissa is temperature (°C), the left ordinate is heat flow (W/g), the exothermic peak is upward; and the right ordinate is weightlessness ratio (%).
**FIGURE 7** is the X-ray powder diffraction spectrum of crystal form IV of free base of the compound of formula I; the abscissa is angle 2θ (°), and the ordinate is intensity.
**FIGURE 8** is the X-ray powder diffraction spectrum of crystal form I of hydrochloride of the compound of formula I; the abscissa is angle 2θ (°), and the ordinate is intensity.
**FIGURE 9** is the X-ray powder diffraction spectrum of crystal form I of sulfate of the compound of formula I; the abscissa is angle 2θ (°), and the ordinate is intensity.
**FIGURE 10** is the X-ray powder diffraction spectrum of crystal form II of sulfate of the compound of formula I; the abscissa is angle 2θ (°), and the ordinate is intensity.
**FIGURE 11** is the X-ray powder diffraction spectrum of crystal form I of phosphate of the compound of formula I; the abscissa is angle 2θ (°), and the ordinate is intensity.
**FIGURE 12** is the X-ray powder diffraction spectrum of crystal form II of phosphate of the compound of formula I; the abscissa is angle 2θ (°), and the ordinate is intensity.
**FIGURE 13** is the X-ray powder diffraction spectrum of crystal form III of phosphate of the compound of formula I; the abscissa is angle 2θ (°), and the ordinate is intensity.
**FIGURE 14** is the X-ray powder diffraction spectrum of crystal form IV of phosphate of the compound of formula I; the abscissa is angle 2θ (°), and the ordinate is intensity.
**FIGURE 15** is the X-ray powder diffraction spectrum of crystal form I of mesylate of the compound of formula I; the abscissa is angle 2θ (°), and the ordinate is intensity.
**FIGURE 16** is the X-ray powder diffraction spectrum of crystal form II of mesylate of the compound of formula I; the abscissa is angle 2θ (°), and the ordinate is intensity.
**FIGURE 17** is the X-ray powder diffraction spectrum of crystal form III of mesylate of the compound of formula I; the abscissa is angle 2θ (°), and the ordinate is intensity.
**FIGURE 18** is the DSC/TGA stacking spectrum of crystal form III of mesylate of the compound of formula I; the abscissa is temperature (°C), the right ordinate is heat flow (W/g), the exothermic peak is upward; and the left ordinate is weightlessness ratio (%).
**FIGURE 19** is the X-ray powder diffraction spectrum of crystal form IV of mesylate of the compound of formula I; the abscissa is angle 2θ (°), and the ordinate is intensity.
**FIGURE 20** is the X-ray powder diffraction spectrum of crystal form V of mesylate of the compound of formula I; the abscissa is angle 2θ (°), and the ordinate is intensity.
**FIGURE 21** is the X-ray powder diffraction spectrum of crystal form I of p-toluenesulfonate of the compound of formula I; the abscissa is angle 2θ (°), and the ordinate is intensity.
**FIGURE 22** is the X-ray powder diffraction spectrum of crystal form II of p-toluenesulfonate of the compound of formula I; the abscissa is angle 2θ (°), and the ordinate is intensity.
**FIGURE 23** is the X-ray powder diffraction spectrum of crystal form III of p-toluenesulfonate of the compound of formula I; the abscissa is angle 2θ (°), and the ordinate is intensity.
**FIGURE 24** is the X-ray powder diffraction spectrum of crystal form I of 1,5-naphthalenedisulfonate of the compound of formula I; the abscissa is angle 2θ (°), and the ordinate is intensity.
**FIGURE 25** is the X-ray powder diffraction spectrum of crystal form II of 1,5-naphthalenedisulfonate of the compound of formula I; the abscissa is angle 2θ (°), and the ordinate is intensity.
**FIGURE 26** is the X-ray powder diffraction spectrum of crystal form III of 1,5-naphthalenedisulfonate of the compound of formula I; the abscissa is angle 2θ (°), and the ordinate is intensity.
**FIGURE 27** is the DSC spectrum of crystal form III of 1,5-naphthalenedisulfonate of the compound of formula I; the abscissa is temperature (°C), and the ordinate is heat flow (W/g), the exothermic peak is upward.
**FIGURE 28** is the plasma drug concentration-time curve of crystal form III of mesylate and crystal form I of free base of the compound of formula I.

### PREFERRED EMBODIMENTS

The following specific examples are used to further describe the particular aspects of the solutions of the invention.

### Method and Material

The crystal forms of free base of the compound of formula I and salt crystal forms thereof were characterized by their X-ray powder diffraction spectra. Therefore, the X-ray powder diffraction spectrum of the mentioned salt was collected by a Bruker D8 Discover X-ray powder diffractometer with GADDS (General Area Detector Diffraction System) CS using Cu Kα radiation (1.54 Å) in reflective mode. Tube voltage and current amount were set to 40kV and 40mA respectively. In the 2θ range of 3.0 ° to 40 ° or 45 °, the sample was scanned for 60 seconds. For peak position represented by 2θ, a corundum standard was used to calibrate the diffractometer. All analysis was usually implemented at 20°C-30°C room temperature. The data was collected and integrated by GADDS using WNT software version 4.1.14T. The diffraction spectrum was analyzed by DiffracPlus software with version 9.0.0.2 Eva which was published in 2003. The sample of XRPD was prepared as follows: The sample was placed on a monocrystalline silicon wafer, then the sample powder was pressed by a glass sheet or an equivalent to ensure that the surface of the sample was flat and had a suitable height. And then the sample holder was placed in the Bruker XRPD instrument, and the X-ray powder diffraction spectrum was colleted using the above instrument parameters. The measured difference related to the analysis result of the X-ray powder diffraction was produced by various factors including: (a) the error of sample preparation (e.g., sample height), (b) the instrument error, (c) the calibration error, (d) operator error (including those errors that occur in the determination of peak positions), and (e) properties of the substance (e.g. preferred orientation error). Calibration error and sample height error often lead to the shifts of all the peaks in the same direction. In general, the calibration factor will make the measured peak positions in consistent with the expected peak positions and in the range of 2θ expected values ± 0.2°. Angle 2θ values (°) and intensity values (% relative to the highest peak value) of each polymorph obtained in the Examples of the present invention are shown in Tables 1 to 22.

### Preparation of amorphous free base

The amorphous free base of the compound of formual I was prepared according to Example 22 of Chinese Patent Application CN201310173581.4 and the PCT application thereof (PCT / CN2014 / 072825). Specific procedure was as follows: To a 30 mL microwave tube, 8-fluoro-6-(1-methyl-1H-pyrazol-4-yl)-[1,2,4] triazolo[4,3-a]pyridin-3-thiol (93 mg, 0.375 mmol), 9-bromo-4-methyl-2H-[1,4]oxazino[3,2-c]quinolin-3(4H)-one (100 mg, 0.341 mmol), tris(dibenzylideneacetone)dipalladium (20 mg, 0.034 mmol), 4,5-bis(diphenylphosphino)-9,9-dimethylxanthene (40 mg, 0.068 mmol), sodium tert-butoxide (40 mg, 0.409 mmol) and anhydrous N,N-dimethylformamide (5 mL) were added successively. The reaction mixture was purged with N₂ and heated by microwaves to 120 °C for 4 hours. After the reaction was stopped, N,N-dimethylformamide was removed by rotary evaporation. The residue was purified by reverse phase column chromatography to obtain 36 mg white amorphous 9-((8-fluoro-6-(1-methyl-1H-pyrazol-4-yl)-[1,2,4]triazolo[4,3-a]pyridin-3-yl)thio)-4-methyl-2H-[1,4]oxazino[3,2-c]quinolin-3(4H)-one 22.

### Solubility of amorphous free base in different simulated biological media

Procedure: About 10 mg of amorphous free base was weighed and placed in a 2 mL glass vial, 1 mL of simulated biological media (simulated artificial gastric juice, simulated artificial intestinal juice - fasting, and simulated artificial intestinal juice - satiety), and a magnetic stirrer was added, then the vial was sealed. The mixture was magnetically stirred at 37°C and about 0.4 mL of the sample was taken at different time points. The mixture was filtered with a centrifuge tube (pore size of filter membrane: 0.45 µm), the filtrate was taken and the content of the compound of formula I therein was analyzed by HPLC. Measurement results were shown in the table below:

| Crystal Form | Simulated biological media | Solubility (1 hour) mg/mL | Solubility (4 hours) mg/mL | Solubility (22 hours) mg/mL |
|---|---|---|---|---|
| Amorphous free base | Simulated artificial gastric juices | 0.011 | 0.009 | 0.009 |
| | Simulated artificial intestinal juice-fasting | 0.002 | 0.002 | 0.002 |
| | Simulated artificial intestinal juice-satiety | 0.003 | 0.002 | 0.002 |

### Example 1 Preparation of crystal form I of free base

A certain amount of free base of the compound of formula I (amorphous) was weighed and placed in a container. A solvent was added and the free base of the compound of formula I was dispersed in the solvent to form a suspension (1-200 mg/mL). The suspension was stirred at room temperature (20-25°C) until the amorphous compound of formula I was transformed to crystal form I of free base. A solid-liquid separation was carried out to obtain a solid, i.e., crystal form I of free base, its X-ray powder diffraction spectrum was shown in Figure 1.

The melting point of crystal form I of free base was measured by a differential scanning calorimetry (DSC, model: Neszsch DSC 204 F1). Measurement conditions: heating from room temperature to 300°C at a heating rate of 10°C per minute in a nitrogen atmosphere, and the nitrogen flow of 20 mL per minute. The DSC spectrum of crystal form I of free base was shown in Figure 2. The melting point of crystal form I of free base (onset temperature) was 274.4°C. The thermal weightlessness of crystal form I of free base was measured by a thermal gravimetric analyzer (TGA, model: TA Q500). Measurement conditions: heating from room temperature to 350°C at a heating rate of 10°C per minute in a nitrogen atmosphere, and the nitrogen flow of 50 mL per minute. The TGA spectrum of crystal form I of free base was shown in Figure 3. Due to almost no weight loss below 100°C, it could be determined that crystal form I of free base was an anhydrous compound.

| Initial state of the compound of formula I | Amorphous | Amorphous | Amorphous | Amorphous | Amorphous | Amorphous | | Amorphous | |
|---|---|---|---|---|---|---|---|---|---|
| Amount of the compound of formula I | 20 mg | 20 mg | 20 mg | 20 mg | 20 mg | 20 mg | | 20 mg | |
| Solvent name | Isopropanol | Isopropyl acetate | Acetonitrile | Tetrahydrofuran | 2-methoxyethanol | Isopropanol | Isopropyl acetate | Isopropanol | Acetonitrile |
| Solvent amount | 1 mL | 1 mL | 1 mL | 1 mL | 1 mL | 0.5 mL | 0.5 mL | 0.5 mL | 0.5 mL |
| Concentration | 20 mg/mL | 20 mg/mL | 20 mg/mL | 20 mg/mL | 20 mg/mL | Total concentration of mixed solvent | | Total concentration of mixed solvent | |
| Experimental temperature | Room temperature | Room temperature | Room temperature | Room temperature | Room temperature | Room temperature | | Room temperature | |
| Experimental results | Crystal Form I | Crystal Form I | Crystal Form I | Crystal Form I | Crystal Form I | Crystal Form I | | Crystal Form I | |

### Solubility of crystal form I of free base in different simulated biological media

Procedure: About 10 mg of crystal form I of free base was weighed and placed in a 2 mL glass vial, 1 mL of simulated biological media (simulated artificial gastric juice, simulated artificial intestinal juice-fasting, and simulated artificial intestinal juice-satiety), and a magnetic stirrer was added, then the vial was sealed. The mixture was magnetically stirred at 37°C and about 0.4 mL of the sample was taken at different time points. The mixture was filtered with a centrifuge tube (pore size of filter membrane: 0.45 µm), the filtrate was taken and the content of the compound of formula I therein was analyzed by HPLC. Measurement results were shown in the table below:

| Crystal Form | Simulated biological media | Solubility (1 hour) mg/mL | Solubility (4 hours) mg/mL | Solubility (22 hours) mg/mL |
|---|---|---|---|---|
| Crystal form I of free base | Simulated artificial gastric juices | 0.018 | 0.019 | 0.019 |
| | Simulated artificial intestinal juice-fasting | 0.001 | 0.001 | 0.002 |
| | Simulated artificial intestinal juice-satiety | 0.004 | 0.004 | 0.004 |

### Example 2 Preparation of crystal form II of free base

A certain amount of free base of the compound of formula I (amorphous) was weighed and placed in a container. A solvent was added and the free base of the compound of formula I was dispersed in the solvent to form a suspension (1-200 mg/mL). A suitable solvent was added. The suspension was stirred at room temperature (20-25°C) until the amorphous compound of formula I was transformed to crystal form II of free base. A solid-liquid separation was carried out to obtain a solid, i.e., crystal form II of free base, its X-ray powder diffraction spectrum was shown in Figure 4.

| Initial state of the compound of formula I | Amorphous | Amorphous | Amorphous | Amorphous | Amorphous | Amorphous | | Amorphous | |
|---|---|---|---|---|---|---|---|---|---|
| Amount of the compound of formula I | 20 mg | 20 mg | 20 mg | 20 mg | 20 mg | 20 mg | | 20 mg | |
| Solvent name | Isopropanol | Isopropyl acetate | Acetonitrile | Tetrahydrofuran | 2-methoxyethanol | Isopropanol | Isopropyl acetate | Isopropanol | Acetonitrile |
| Solvent amount | 1 mL | 1 mL | 1 mL | 1 mL | 1 mL | 0.5 mL | 0.5 mL | 0.5 mL | 0.5 mL |
| Concentration | 20 mg/mL | 20 mg/mL | 20 mg/mL | 20 mg/mL | 20 mg/mL | Total concentration of mixed solvent | | Total concentration of mixed solvent | |
| Experimental temperature | Room temperature | Room temperature | Room temperature | Room temperature | Room temperature | Room temperature | | Room temperature | |
| Experimental results | Crystal Form II | Crystal Form II | Crystal Form II | Crystal Form II | Crystal Form II | Crystal Form II | | Crystal Form II | |

### Example 3 Preparation of crystal form III of free base

About 20 mg of crystal form II of free base of the compound of formula I was weighed and placed in a 2 mL HPLC vial, 1.0 mL of acetonitrile was added, the mixture was magnetically stirred about 1 day at 50°C and sufficiently crystallized to obtain a solid compound, i.e., crystal form III of free base, its X-ray powder diffraction spectrum was shown in Figure 5.

The melting point of crystal form III of free base was measured by a differential scanning calorimetry (DSC, model: TA Q2000). Measurement conditions: heating from room temperature to 300°C at a heating rate of 10°C per minute in a nitrogen atmosphere, and the nitrogen flow of 20 mL per minute. The DSC spectrum of crystal form III of free base was shown in Figure 5. The melting point of crystal form III of free base (onset temperature) was 275.5°C. The thermal weightlessness of crystal form III of free base was measured by a thermal gravimetric analyzer (TGA, model: TA Q500). Measurement conditions: heating from room temperature to 350°C at a heating rate of 10°C per minute in a nitrogen atmosphere, and the nitrogen flow of 50 mL per minute. The TGA spectrum of crystal form III of free base was shown in Figure 6. Due to almost no weight loss below 100°C, it could be determined that crystal form III of free base was an anhydrous compound.

### Solubility of crystal form III of free base in different simulated biological media

Procedure: About 10 mg of crystal form III of free base was weighed and placed in a 2 mL glass vial, 1 mL of simulated biological media (simulated artificial gastric juice, simulated artificial intestinal juice-fasting, and simulated artificial intestinal juice-satiety), and a magnetic stirrer was added, then the vial was sealed. The mixture was magnetically stirred at 37°C and about 0.4 mL of the sample was taken at different time points. The mixture was filtered with a centrifuge tube (pore size of filter membrane: 0.45 µm), the filtrate was taken and the content of the compound of formula I therein was analyzed by HPLC. Measurement results were shown in the table below:

| Crystal Form | Simulated biological media | Solubility (1 hour) mg/mL | Solubility (4 hours) mg/mL | Solubility (22 hours) mg/mL |
|---|---|---|---|---|
| Crystal III of free base | form Simulated artificial gastric juices | 0.040 | 0.043 | 0.043 |
| | Simulated artificial intestinal juice-fasting | 0.004 | 0.004 | 0.004 |
| | Simulated artificial intestinal juice-satiety | 0.011 | 0.011 | 0.010 |

### Example 4 Preparation of crystal form IV of free base

About 10 mg of crystal form II of free base of the compound of formula I was weighed and placed in a TGA (Thermal gravimetric analyzer, model: TA Q500), and then heated from room temperature 25°C to 120°C at a rate of 10°C/min to remove the solvent, then cooled slowly to room temperature (10°C / min) to obtain a solid. Its X-ray powder diffraction spectrum was shown in Figure 7.

### Example 5 Preparation of crystal form I of hydrochloride

About 20 mg of free base of the compound of formula I was weighed and placed in a 2 mL HPLC vial, 1.0 mL of methanol was added, and then 4.8 µL of 37% hydrochloric acid solution was added. The mixture was magnetically stirred about 2 days at room temperature and sufficiently crystallized to obtain crystal form I of hydrochloride, its X-ray powder diffraction spectrum was shown in Figure 8.

| Initial state of the compound of formula I | Free base | Free base | Free base | Free base |
|---|---|---|---|---|
| Amount of the compound of formula I | 20 mg | 20 mg | 20 mg | 20 mg |
| Solvent name | Methanol | Isopropyl acetate | Acetonitrile | Acetone |
| Solvent amount | 1 mL | 1 mL | 1 mL | 1 mL |
| Concentration | 20 mg/mL | 20 mg/mL | 20 mg/mL | 20 mg/mL |
| 37% HCl | 4.8 µL | 4.8 µL | 4.8 µL | 4.8 µL |
| Experimental temperature | Room temperature | Room temperature | Room temperature | Room temperature |
| Experimental results | Crystal form I of hydrochloride | Crystal form I of hydrochloride | Crystal form I of hydrochloride | Crystal form I of hydrochloride |

### Example 6 Preparation of crystal form I of sulfate

About 20 mg of free base of the compound of formula I was weighed and placed in a 2 mL HPLC vial, 1.0 mL of acetonitrile or acetone was added, and then 4.9 µL of 98% concentrated sulfuric acid solution was added. The mixture was magnetically stirred about 2 days at room temperature and sufficiently crystallized to obtain crystal form I of sulfate, its X-ray powder diffraction spectrum was shown in Figure 9.

| Initial state of the compound of formula I | Amorphous | Amorphous |
|---|---|---|
| Amount of the compound of formula I | 20 mg | 20 mg |
| Solvent name | Acetonitrile | Acetone |
| Solvent amount | 1 mL | 1 mL |
| Concentration | 20 mg/mL | 20 mg/mL |
| Concentrated sulfuric acid | 4.9 µL | 4.9 µL |
| Experimental temperature | Room temperature | Room temperature |
| Experimental results | Crystal form I of sulfate | Crystal form I of sulfate |

### Example 7 Preparation of crystal form II of sulfate

About 20 mg of free base of the compound of formula I was weighed and placed in a 2 mL HPLC vial, 1.0 mL of 50% ethanol was added, and then 4.9 µL of 98% concentrated sulfuric acid solution was added. The mixture was magnetically stirred about 2 days at room temperature and sufficiently crystallized to obtain crystal form II of sulfate, its X-ray powder diffraction spectrum was shown in Figure 10.

### Example 8 Preparation of crystal form I of phosphate

About 20 mg of free base of the compound of formula I was weighed and placed in a 2 mL HPLC vial, 1.0 mL of methanol or 50% ethanol was added, and then 7.5 µL of 85% phosphoric acid solution was added. The mixture was magnetically stirred about 2 days at room temperature and sufficiently crystallized to obtain crystal form I of phosphate, its X-ray powder diffraction spectrum was shown in Figure 11.

| Initial state of the compound of formula I | Amorphous | Amorphous |
|---|---|---|
| Amount of the compound of formula I | 20 mg | 20 mg |
| Solvent name | Methanol | 50% Ethanol /water |
| Solvent amount | 1 mL | 1 mL |
| Concentration | 20 mg/mL | 20 mg/mL |
| 85% phosphoric acid solution | 7.5 µL | 7.5 µL |
| Experimental temperature | Room temperature | Room temperature |
| Experimental results | Crystal form I of phosphate | Crystal form I of phosphate |

### Solubility of crystal form I of phosphate in different simulated biological media

Procedure: About 10 mg of crystal form I of phosphate was weighed and placed in a 2 mL glass vial, 1 mL of simulated biological media (simulated artificial gastric juice, simulated artificial intestinal juice-fasting, and simulated artificial intestinal juice-satiety), and a magnetic stirrer was added, then the vial was sealed. The mixture was magnetically stirred at 37°C and about 0.4 mL of the sample was taken at different time points. The mixture was filtered with a centrifuge tube (pore size of filter membrane: 0.45 µm), the filtrate was taken and the content of the compound of formula I therein was analyzed by HPLC. Measurement results were shown in the table below:

| Crystal Form | Simulated biological media | Solubility (1 hour) mg/mL | Solubility (4 hours) mg/mL | Solubility (22 hours) mg/mL |
|---|---|---|---|---|
| Crystal form I of phosphate | Simulated artificial gastric juices | 0.128 | 0.135 | 0.135 |
| | Simulated artificial intestinal juice-fasting | 0.008 | 0.004 | 0.003 |
| | Simulated artificial intestinal juice-satiety | 0.032 | 0.033 | 0.027 |

The solubility of crystal form I of phosphate, was much greater than the solubility of the free base, the solubility of the compound of formula I was thus improved which was in favor of increasing the bioavailability.

### Example 9 Preparation of crystal form II of phosphate

About 20 mg of free base of the compound of formula I was weighed and placed in a 2 mL HPLC vial, 1.0 mL of acetonitrile, ethyl acetate or tetrahydrofuran was added, and then 7.5 µL of 85% phosphoric acid solution was added. The mixture was magnetically stirred about 2 days at room temperature and sufficiently crystallized to obtain crystal form II of phosphate, its X-ray powder diffraction spectrum was shown in Figure 12.

| Initial state of the compound of formula I | Amorphous | Amorphous | Amorphous |
|---|---|---|---|
| Amount of the compound of formula I | 20 mg | 20 mg | 20 mg |
| Solvent name | Acetonitrile | Ethyl acetate | Tetrahydrofuran |
| Solvent amount | 1 mL | 1 mL | 1 mL |
| Concentration | 20 mg/mL | 20 mg/mL | 20 mg/mL |
| 85% phosphoric acid solution | 7.5 µL | 7.5 µL | 7.5 µL |
| Experimental temperature | Room temperature | Room temperature | Room temperature |
| Experimental results | Crystal form II of phosphate | Crystal form II of phosphate | Crystal form II of phosphate |

### Example 10 Preparation of crystal form III of phosphate

About 20 mg of free base of the compound of formula I was weighed and placed in a 2 mL HPLC vial, 1.0 mL of acetone was added, and then 7.5 µL of 85% phosphoric acid solution was added. The mixture was magnetically stirred about 2 days at room temperature and sufficiently crystallized to obtain crystal form III of phosphate, its X-ray powder diffraction spectrum was shown in Figure 13.

### Example 11 Preparation of crystal form IV of phosphate

About 20 mg of free base of the compound of formula I was weighed and placed in a 2 mL HPLC vial, 5.0 mL of methanol was added, and then 37.5 µL of 85% phosphoric acid solution was added. The mixture was magnetically stirred about 2 days at room temperature and sufficiently crystallized to obtain crystal form IV of phosphate, its X-ray powder diffraction spectrum was shown in Figure 14.

### Example 12 Preparation of crystal form I of mesylate

A certain amount of free base of the compound of formula I was weighed and placed in a container. A suitable solvent was added, then an equal or excess molar equivalent of methanesulfonic acid was added. The mixture was stirred, after the salt-forming reaction was completed, a solid-liquid separation was carried out to obtain crystal form I of mesylate (hydrate), its X-ray powder diffraction spectrum was shown in Figure 15. For example:
About 20 mg of free base of the compound of formula I was weighed and placed in a 2 mL HPLC vial, 1.0 mL of acetone was added, and then 6.2 µL of 98% methanesulfonic acid solution was added. The mixture was magnetically stirred about 2 days at room temperature and sufficiently crystallized to obtain crystal form I of mesylate.

| Initial state of the compound of formula I | Free base | Free base | Free base | Free base | Free base | Free base |
|---|---|---|---|---|---|---|
| Amount of compound I | 20 mg | 20 mg | 20 mg | 20 mg | 20 mg | 20 mg |
| Solvent name | Acetone | Ethyl acetate | Tetrahydro furan | Isopropyl acetate | 2-Methoxy ethyl ether | 1,4-Dioxane |
| Solvent amount | 1 mL | 1 mL | 1 mL | 1 mL | 1 mL | 1 mL |
| Concentration | 20 mg/mL | 20 mg/mL | 20 mg/mL | 20 mg/mL | 20 mg/mL | 20 mg/mL |
| 98% methanesulfonic acid | 6.2 µL | 6.2 µL | 6.2 µL | 6.2 µL | 6.2 µL | 6.2 µL |
| Experimental temperature | Room temperature | Room temperature | Room temperature | Room temperature | Room temperature | Room temperature |
| Experimental results | Crystal form I of mesylate | Crystal form I of mesylate | Crystal form I of mesylate | Crystal form I of mesylate | Crystal form I of mesylate | Crystal form I of mesylate |

### Example 13 Preparation of crystal form II of mesylate

A certain amount of free base of the compound of formula I was weighed and placed in a container. A mixed solvent of methanol and water (methanol/water volume ratio: 5%-95%) was added. The mixture was stirred, then an equal or excess molar equivalent of methanesulfonic acid was added until the solution became clear. After mesylate was precipitated, a solid-liquid separation is carried out to obtain crystal form II of mesylate (hydrate), its X-ray powder diffraction spectrum was shown in Figure 16.

### Example 14 Preparation of crystal form III of mesylate

### Method 1:

About 20 mg of free base of the compound of formula I was weighed and placed in a 2 mL HPLC vial, 1.0 mL methanol was added, and then 6.2 µL of 98% methanesulfonic acid was added. The mixture was magnetically stirred about 2 days at room temperature and sufficiently crystallized to obtain mesylate, and then dried for 1 day in a vacuum oven at 100°C to obtain crystal form III of mesylate, its X-ray powder diffraction spectrum was shown in Figure 17.

### Method 2:

Crystal form IV of mesylate was dried in vacuum overnight. Crystal form IV of mesylate was transformed to crystal form III of mesylate, its X-ray powder diffraction spectrum was consistent with Figure 17.

### Method 3:

About 300 mg of crystal form V of mesylate was weighed and placed in a 20 mL glass vial, then 15 mL of 25% ethyl acetate/ n-heptane (v/v) solution was added. The mixture was stirred for 24 hours at 40°C, was and then filtered to obtain crystal form III of mesylate, its X-ray powder diffraction spectrum was consistent with Figure 17.

The melting point of crystal form III of mesylate was measured by a differential scanning calorimetry (DSC, model: TA Q2000). Measurement conditions: heating from room temperature to 300°C at a heating rate of 10°C per minute in a nitrogen atmosphere, and the nitrogen flow of 20 mL per minute. The DSC spectrum of crystal form III of mesylate was shown in Figure 18. The melting point of crystal form III of mesylate (onset temperature) was 252.4°C. The thermal weightlessness of crystal form III of mesylate was measured by a thermal gravimetric analyzer (TGA, model: TA Q500). Measurement conditions: heating from room temperature to 350°C at a heating rate of 10°C per minute in a nitrogen atmosphere, and the nitrogen flow of 50 mL per minute. The TGA spectrum of crystal form III of mesylate was shown in Figure 18. Due to almost no weight loss below 100°C, it could be determined that crystal form III of mesylate was an anhydrous compound.

### Solubility of crystal form III of mesylate in different simulated biological media

Procedure: About 10 mg of crystal form III of mesylate was weighed and placed in a 2 mL glass vial, 1 mL of simulated biological media (simulated artificial gastric juice, simulated artificial intestinal juice-fasting, and simulated artificial intestinal juice-satiety), and a magnetic stirrer was added, then the vial was sealed. The mixture was magnetically stirred at 37°C and about 0.4 mL of the sample was taken at different time points. The mixture was filtered with a centrifuge tube (pore size of filter membrane: 0.45 µm), the filtrate was taken and the content of the compound of formula I therein was analyzed by HPLC. Measurement results were shown in the table below:

| Crystal Form | Simulated biological media | Solubility (1 hour) mg/mL | Solubility (4 hours) mg/mL | Solubility (22 hours) mg/mL |
|---|---|---|---|---|
| Crystal form III of mesylate | Simulated artificial gastric juices | 0.211 | 0.200 | 0.0125 |
| | Simulated artificial intestinal juice-fasting | 0.005 | 0.005 | 0.005 |
| | Simulated artificial intestinal juice-satiety | 0.020 | 0.014 | 0.010 |

The solubility of crystal form III of mesylate was much greater than the solubility of the free base, the solubility of the compound of formula I was thus improved which was in favor of increasing the bioavailability.

The accelerated stability test of crystal form III of mesylate was used for researching the physical and chemical stability of crystal form III of mesylate. Specific procedure: After the sample was placed in an accelerated stability for a sufficient time under the conditions shown in the table below, the sample was taken out and dissolved in the mobile phase, and then its purity was determined by HPLC. Before the start of the accelerated stability test, the purity of the initial sample was determined by HPLC. The ratio of the purity of the sample after the accelerated stability test to the purity of the initial sample was used as a stability criterion, if the ratio was less than 95% purity, the sample was considered as unstable. Specific experimental conditions and results were shown in the table below:

| Crystal Form | Accelerated test condition | Period | Initial purity | Purity after the accelerated stability test | Purity ratio | Crystal transformation after the accelerated stability test |
|---|---|---|---|---|---|---|
| Crystal form III of mesylate | 80 °C | 1 day | 99.9% | 99.7% | 99.8% | No transformation |
| | 40 °C / 75% relative humidity | 1 week | 98.2% | 98.8% | 100.6% | No transformation |

From the results in the above table, it could be seen that crystal form III of mesylate has good physical and chemical stability under the above conditions.

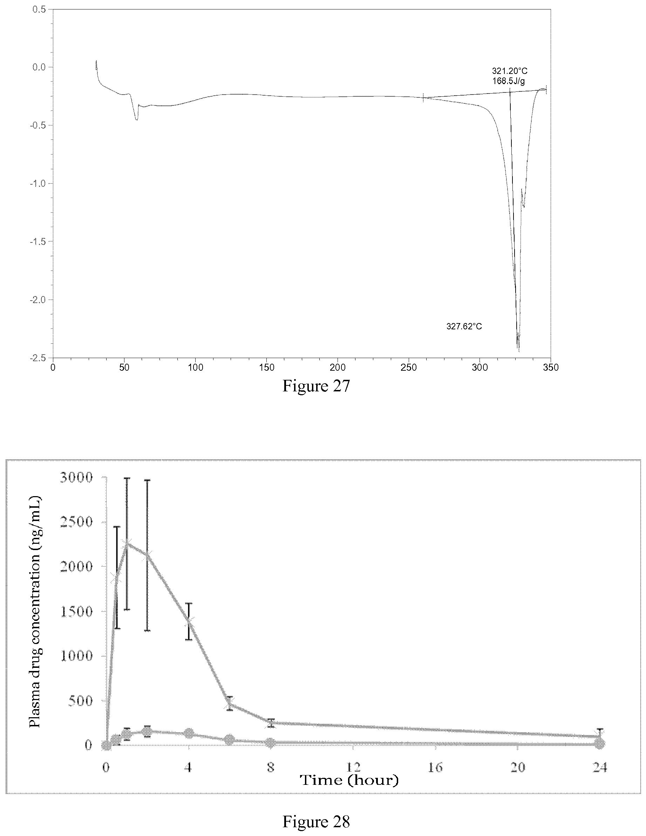

It could be seen from Figure 28 that the bioavailability of crystal form III of mesylate was increased by 5 times in comparison to form I of the free base. Experimental conditions: the experimental animal was a dog, a dose of 5mg / kg, single administration by gavage. Therefore, the salt-form compound has a significant improvement in comparison to the free base.

### Example 15 Preparation of crystal form IV of mesylate

About 20 mg of free base of the compound of formula I was weighed and placed in a 2 mL HPLC vial, 1.0 mL of methanol was added, and then 6.2 µL of 98% methanesulfonic acid solution was added. The mixture was magnetically stirred about 2 days at room temperature and sufficiently crystallized to obtain crystal form IV of mesylate, its X-ray powder diffraction spectrum was shown in Figure 19.

### Example 16 Preparation of crystal form V of mesylate

About 100 mg of free base of the compound of formula I was weighed and placed in a 10 mL glass vial, 2.0 mL of DMSO was added, and then 31.2 µL of 98% methanesulfonic acid solution was added. The mixture was stirred at room temperature until the solution was clear. The mixture was filtered, then 3 mL of ethyl acetate was add to the filtrate. After stirring for 24 hours, the mixture was filtered to obtain crystal form V of mesylate (DMSO solvate). Its X-ray powder diffraction spectrum was shown in Figure 20.

The formation of crystal form V of mesylate (DMSO solvate) played a role on purifying and refining of the compound of formula I.

| Crystal Form | Purity |
|---|---|
| Free base of formula I | 95.0% |
| DMSO solvate of mesylate | 99.7% |

In addition, the formation of crystal form V of mesylate brings great convenience in the unit operation, because the compound of formula I can be dissolved in DMSO, it is easy to achieve on-line filtration, and the solution is transported to the GMP workshop through a pipeline, then the compound of formula I is reacted with methanesulfonic acid, the resulting product is precipitated from the solution, then crystal form V of mesylate is obtained by filtration, thereby purifying the compound of formula I. As an important product of this advanced purification method, crystal form V has practical application value.

### Example 17 Preparation of crystal form I of p-toluenesulfonate

About 20 mg of free base of the compound of formula I was weighed and placed in a 2 mL HPLC vial, 1.0 mL of methanol was added, and then 8.6 µL of 98% p-toluenesulfonic acid solution was added. The mixture was magnetically stirred about 2 days at room temperature and sufficiently crystallized to obtain crystal form I of p-toluenesulfonate, its X-ray powder diffraction spectrum was shown in Table 17.

| Initial state of the compound of formula I | Free base | Free base | Free base |
|---|---|---|---|
| Amount of the compound of formula I | 20 mg | 20 mg | 20 mg |
| Solvent name | Methanol | Acetonitrile | Acetone |
| Solvent amount | 1 mL | 1 mL | 1 mL |
| Concentration | 20 mg/mL | 20 mg/mL | 20 mg/mL |
| 98% p-toluenesulfonic acid | 8.6 µL | 8.6 µL | 8.6 µL |
| Experimental temperature | Room temperature | Room temperature | Room temperature |
| Experimental results | Crystal form I of p-toluenesulfonate | Crystal form I of p-toluenesulfonate | Crystal form I of p-toluenesulfonate |

### Example 18 Preparation of crystal form II of p-toluenesulfonate

About 20 mg of free base of the compound of formula I was weighed and placed in a 2 mL HPLC vial, 1.0 mL of ethyl acetate was added, and then 8.6 µL of 98% p-toluenesulfonic acid solution was added. The mixture was magnetically stirred about 2 days at room temperature and sufficiently crystallized to obtain crystal form II of p-toluenesulfonate, its X-ray powder diffraction spectrum was shown in Table 18.

### Example 19 Te preparation of crystal form III of p-toluenesulfonate

About 20 mg of free base of the compound of formula I was weighed and placed in a 2 mL HPLC vial, 1.0 mL of 50% methanol was added, and then 8.6 µL of 98% p-toluenesulfonic acid solution was added. The mixture was magnetically stirred about 2 days at room temperature and sufficiently crystallized to obtain crystal form III of p-toluenesulfonate, its X-ray powder diffraction spectrum was shown in Table 19.

### Example 20 Preparation of crystal form I of 1,5-naphthalenedisulfonate

A certain amount of free base of the compound of formula I was weighed and placed in a container. Methanol, acetonitrile or acetone was added, and then an equal or excess molar equivalent of 1,5-naphthalenedisulfonic acid was added. The mixture was stirred, after the salt-forming reaction was completed, a solid-liquid separation was carried out to obtain crystal form I of 1,5-naphthalenedisulfonate.

About 20 mg of free base of the compound of formula I was weighed and placed in a 2 mL HPLC vial, 1.0 mL of acetone was added, and then 23.8 mg of 1,5-naphthalenedisulfonic acid was added. The mixture was magnetically stirred about 2 days at room temperature and sufficiently crystallized to obtain crystal form I of 1,5-naphthalenedisulfonate, its X-ray powder diffraction spectrum was shown in Table 20.

| Initial state of the compound of formula I | Free base | Free base | Free base |
|---|---|---|---|
| Amount of the compound of formula I | 20 mg | 20 mg | 20 mg |
| Solvent name | Methanol | Acetonitrile | Acetone |
| Solvent amount | 1 mL | 1 mL | 1 mL |
| Concentration | 20 mg/mL | 20 mg/mL | 20 mg/mL |
| 2-Naphthalenesulfonic acid | 23.8 mg | 23.8 mg | 23.8 mg |
| Experimental temperature | Room temperature | Room temperature | Room temperature |
| Experimental results | Crystal form I of 1,5-naphthalenedisulfonate | Crystal form I of 1,5-naphthalenedisulfonate | Crystal form I of 1,5-naphthalenedisulfonate |

### Example 21 Preparation of crystal form II of 1,5-naphthalenedisulfonate

About 20 mg of free base of the compound of formula I was weighed and placed in a 2 mL HPLC vial, 1.0 mL of ethyl acetate was added, and then 23.8 mg 1,5-naphthalenedisulfonic acid was added. The mixture was magnetically stirred about 2 days at room temperature and sufficiently crystallized to obtain crystal form II of 1,5-naphthalenedisulfonate, its X-ray powder diffraction spectrum was shown in Figure 20.

### Example 22 Preparation of crystal form III of 1,5-naphthalenedisulfonate

About 100 mg of free base of the compound of formula I was weighed and placed in a 20 mL HPLC vial, 5.0 mL of methanol was added, and then 119 mg 1,5-naphthalenedisulfonic acid was added. The mixture was magnetically stirred about 2 days at room temperature and sufficiently crystallized to obtain crystal form III of 1,5-naphthalenedisulfonate, its X-ray powder diffraction spectrum was shown in Figure 27.

The melting point of crystal form III of 1,5-naphthalenedisulfonate was measured by a differential scanning calorimetry (DSC, model: TA Q2000). Measurement conditions: heating from room temperature to 300°C at a heating rate of 10°C per minute in a nitrogen atmosphere, and the nitrogen flow of 20 mL per minute. The DSC spectrum of crystal form III of 1,5-naphthalenedisulfonate was shown in Figure 26. The melting point of crystal form III of 1,5-naphthalenedisulfonate (onset temperature) was 321.2°C.

### Solubility of crystal form III of 1,5-naphthalenedisulfonate in different simulated biological media

Procedure: About 10 mg of crystal form III of 1,5-naphthalenedisulfonate was weighed and placed in a 2 mL glass vial, 1 mL of simulated biological media (simulated artificial gastric juice, simulated artificial intestinal juice-fasting, and simulated artificial intestinal juice-satiety), and a magnetic stirrer was added, then the vial was sealed. The mixture was magnetically stirred at 37°C and about 0.4 mL of the sample was taken at different time points. The mixture was filtered with a centrifuge tube (pore size of filter membrane: 0.45 µm), the filtrate was taken and the content of the compound of formula I therein was analyzed by HPLC. Measurement results were shown in the table below:

| Crystal form | Simulated biological media | Solubility (1 hour) mg/mL | Solubility (4 hours) mg/mL | Solubility (22 hours) mg/mL |
|---|---|---|---|---|
| Crystal form III of 1,5-naphthalenedisulfonate | Simulated artificial gastric juices | 0.182 | 0.027 | 0.030 |
| | Simulated artificial intestinal juice-fasting | 0.012 | 0.010 | 0.005 |
| | Simulated artificial intestinal juice-satiety | 0.048 | 0.033 | 0.013 |

The solubility of crystal form III of 1,5-naphthalenedisulfonate was much greater than the solubility of the free base, the solubility of the compound of formula I was thus improved which was in favor of increasing the bioavailability.

## Claims

1. A crystalline free base of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one, wherein the crystalline free base is selected from the group consisting of crystal form I, crystal form II, crystal form III and crystal form IV, wherein an X-ray powder diffraction spectrum of crystal form I of the crystalline free base comprises peaks at diffraction angles (2θ) of 13.0±0.2°, 17.9±0.2°, 21.2±0.2° and 31.4±0.2°, preferably, also comprises peaks at diffraction angles (2θ) of 10.3±0.2°, 11.1±0.2°, 23.3±0.2°, 23.8±0.2° and 33.6±0.2°, more preferably, further also comprises peaks at diffraction angles (2θ) of 15.7±0.2°, 17.7±0.2°, 26.8±0.2°, 28.0±0.2°, 31.7±0.2° and 32.8±0.2°, the most preferably, the X-ray powder diffraction spectrum comprises substantially the same peaks at diffraction angles (2θ) as shown in Figure 1; wherein an X-ray powder diffraction spectrum of crystal form II of the crystalline free base comprises peaks at diffraction angles (2θ) of 8.6±0.2°, 11.5±0.2°, 14.1±0.2° and 19.8±0.2°, preferably, also comprises peaks at diffraction angles (2θ) of 11.9±0.2°, 14.7±0.2°, 15.2±0.2°, 17.2±0.2° and 18.9±0.2°, more preferably, further also comprises peaks at diffraction angles (2θ) of 5.8±0.2°, 7.4±0.2°, 20.9±0.2°, 30.9±0.2°, 31.4±0.2° and 37.9±0.2°, the most preferably, the X-ray powder diffraction spectrum comprises substantially the same peaks at diffraction angles (2θ) as shown in Figure 4; wherein an X-ray powder diffraction spectrum of crystal form III of the crystalline free base comprises peaks at diffraction angles (2θ) of 12.8±0.2°, 14.8±0.2°, 18.0±0.2° and 20.5±0.2°, preferably, also comprises peaks at diffraction angles (2θ) of 8.9±0.2°, 9.2±0.2°, 10.6±0.2°, 15.8±0.2° and 20.7±0.2°, more preferably, further also comprises peaks at diffraction angles (2θ) of 5.3±0.2°, 5.9±0.2°, 12.0±0.2°, 14.0±0.2°, 17.3±0.2° and 19.9±0.2°, the most preferably, the X-ray powder diffraction spectrum comprises substantially the same peaks at diffraction angles (2θ) as shown in Figure 5; wherein an X-ray powder diffraction spectrum of crystal form IV of the crystalline free base comprises peaks at diffraction angles (2θ) of 8.9±0.2°, 12.6±0.2°, 17.0±0.2° and 17.9±0.2°, preferably, also comprises peaks at diffraction angles (2θ) of 13.2±0.2°, 14.5±0.2°, 20.5±0.2°, 23.9±0.2° and 26.3±0.2°, the most preferably, the X-ray powder diffraction spectrum comprises substantially the same peaks at diffraction angles (2θ) as shown in Figure 7.

2. A crystalline acid salt of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one, wherein the acid salt comprises an inorganic acid salt or an organic acid salt; wherein the inorganic acid salt is preferably selected from the group consisting of hydrochloride, sulfate, hydrobromate, hydrofluoride, hydroiodide and phosphate, more preferably selected from the group consisting of hydrochloride, sulfate and phosphate; wherein the organic acid salt is preferably selected from the group consisting of 2,5-dihydroxybenzeneformate, 1-hydroxy-2-naphthaleneformate, acetate, dichloroacetate, trichloroacetate, acetohydroxamate, adipate, benzene sulfonate, 4-chlorobenzene sulfonate, benzeneformate, 4-acetamidobenzeneformate, 4-aminobenzeneformate, caprate, caproate, caprilate, cinnamoate, citrate, cyclohexylsulfamate, camphorsulfonate, aspartate, camphorate, gluconate, glucuronate, glutamate, erythorbate, lactate, aspartate, malate, mandelate, pyroglutamate, tartrate, lauryl sulfate, dibenzoyltartrate, ethyl-1,2-disulfonate, esylate, formate, fumarate, galactonate, gentisate, glutarate, 2-oxoglutarate, glycollate, hippurate, isethionate, lactobionate, ascorbate, aspartate, laurate, camphorate, maleate, malonate, mesylate, 1,5-naphthalenedisulfonate, naphthalene-2-sulfonate, nicotinate, oleate, orotate, oxalate, palmitate, embonate, propionate, salicylate, 4-aminosalicylate, sebacate, stearate, butanedioate, thiocyanate, undecylenate, trifluoroacetate, succinate and p-toluenesulfonate, more preferably selected from the group consisting of mesylate, p-toluenesulfonate and 1,5-naphthalenedisulfonate.

3. The crystalline acid salt of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one according to claim 2, **characterized in that** the acid salt is crystal form I of hydrochloride having an X-ray powder diffraction spectrum comprising peaks at diffraction angles (2θ) of 8.1±0.2°, 19.2±0.2°, 24.1±0.2° and 26.2±0.2°, preferably, also comprising peaks at diffraction angles (2θ) of 9.1±0.2°, 11.3±0.2°, 13.4±0.2°, 29.7±0.2° and 23.4±0.2°, the most preferably, the X-ray powder diffraction spectrum comprising substantially the same peaks at diffraction angles (2θ) as shown in Figure 8.

4. The crystalline acid salt of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one according to claim 2, wherein the acid salt is sulfate, preferably selected from the group consisting of crystal form I and crystal form II of sulfate, wherein an X-ray powder diffraction spectrum of crystal form I of sulfate comprises peaks at diffraction angles (2θ) of 18.4±0.2°, 19.7±0.2°, 23.8±0.2° and 24.5±0.2°, preferably, also comprises peaks at diffraction angles (2θ) of 12.8±0.2°, 14.4±0.2°, 17.0±0.2°, 20.0±0.2° and 21.0±0.2°, more preferably, further also comprises peaks at diffraction angles (2θ) of 8.7±0.2°, 13.2±0.2°, 19.1±0.2°, 26.3±0.2°, 26.6±0.2° and 29.0±0.2°, the most preferably, the X-ray powder diffraction spectrum comprises substantially the same peaks at diffraction angles (2θ) as shown in Figure 9; wherein an X-ray powder diffraction spectrum of crystal form II of sulfate comprises peaks at diffraction angles (2θ) of 6.3±0.2°, 8.7±0.2°, 12.7±0.2° and 18.4±0.2°, preferably, also comprises peaks at diffraction angles (2θ) of 8.3±0.2°, 17.5±0.2°, 18.7±0.2°, 20.4±0.2° and 25.6±0.2°, more preferably, further also comprises peaks at diffraction angles (2θ) of 9.1±0.2°, 15.9±0.2°, 16.8±0.2°, 24.0±0.2°, 25.2±0.2° and 28.4±0.2°, the most preferably, the X-ray powder diffraction spectrum comprises substantially the same peaks at diffraction angles (2θ) as shown in Figure 10.

5. The crystalline acid salt of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one according to claim 2, wherein the acid salt is phosphate, preferably selected from the group consisting of crystal form I, crystal form II, crystal form III and crystal form IV of phosphate, wherein an X-ray powder diffraction spectrum of crystal form I of phosphate comprises peaks at diffraction angles (2θ) of 7.9±0.2°, 12.8±0.2°, 15.9±0.2° and 18.3±0.2°, preferably, also comprises peaks at diffraction angles (2θ) of 5.3±0.2°, 10.6±0.2°, 13.4±0.2°, 20.9±0.2° and 24.7±0.2°, more preferably, further also comprises peaks at diffraction angles (2θ) of 16.5±0.2°, 18.7±0.2°, 20.6±0.2°, 21.8±0.2°, 26.2±0.2° and 27.4±0.2°, the most preferably, the X-ray powder diffraction spectrum comprises substantially the same peaks at diffraction angles (2θ) as shown in Figure 11; wherein an X-ray powder diffraction spectrum of crystal form II of phosphate comprises peaks at diffraction angles (2θ) of 13.7±0.2°, 16.1±0.2°, 22.8±0.2° and 26.1±0.2°, preferably, also comprises peaks at diffraction angles (2θ) of 11.0±0.2°, 14.6±0.2°, 20.3±0.2°, 20.8±0.2° and 25.7±0.2°, more preferably, further also comprises peaks at diffraction angles (2θ) of 6.8±0.2°, 17.0±0.2°, 22.2±0.2°, 26.6±0.2°, 27.9±0.2° and 31.2±0.2°, the most preferably, the X-ray powder diffraction spectrum comprises substantially the same peaks at diffraction angles (2θ) as shown in Figure 12; wherein an X-ray powder diffraction spectrum of crystal form III of phosphate comprises peaks at diffraction angles (2θ) of 9.7±0.2°, 15.6±0.2°, 16.8±0.2° and 24.6±0.2°, preferably, also comprises peaks at diffraction angles (2θ) of 4.8±0.2°, 21.2±0.2°, 25.0±0.2°, 27.8±0.2° and 28.1±0.2°, more preferably, further also comprises peaks at diffraction angles (2θ) of 5.2±0.2°, 12.8±0.2°, 14.5±0.2°, 18.0±0.2°, 20.1±0.2° and 23.5±0.2°, the most preferably, the X-ray powder diffraction spectrum comprises substantially the same peaks at diffraction angles (2θ) as shown in Figure 13; wherein an X-ray powder diffraction spectrum of crystal form IV of phosphate comprises peaks at diffraction angles (2θ) of 7.8±0.2°, 17.9±0.2°, 25.0±0.2° and 27.7±0.2°, preferably, also comprises peaks at diffraction angles (2θ) of 6.7±0.2°, 10.8±0.2°, 15.6±0.2°, 23.4±0.2° and 24.6±0.2°, more preferably, further also comprises peaks at diffraction angles (2θ) of 5.2±0.2°, 12.8±0.2°, 20.9±0.2°, 21.7±0.2°, 22.3±0.2° and 26.8±0.2°, the most preferably, the X-ray powder diffraction spectrum comprises substantially the same peaks at diffraction angles (2θ) as shown in Figure 14.

6. The crystalline acid salt of9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one according to claim 2, wherein the acid salt is mesylate, preferably selected from the group consisting of crystal form I, crystal form II, crystal form III, crystal form IV and crystal form V of mesylate, wherein an X-ray powder diffraction spectrum of crystal form I of mesylate comprises peaks at diffraction angles (2θ) of 15.6±0.2°, 17.0±0.2°, 25.6±0.2° and 26.0±0.2°, preferably, also comprises peaks at diffraction angles (2θ) of 9.8±0.2°, 21.8±0.2°, 23.5±0.2°, 23.8±0.2° and 27.5±0.2°, more preferably, further also comprises peaks at diffraction angles (2θ) of 6.6±0.2°, 15.3±0.2°, 17.2±0.2°, 18.3±0.2°, 19.7±0.2° and 26.4±0.2°, the most preferably, the X-ray powder diffraction spectrum comprises substantially the same peaks at diffraction angles (2θ) as shown in Figure 15; wherein an X-ray powder diffraction spectrum of crystal form II of mesylate comprises peaks at diffraction angles (2θ) of 9.4±0.2°, 17.0±0.2°, 18.9±0.2° and 27.3±0.2°, preferably, also comprises peaks at diffraction angles (2θ) of 6.6±0.2°, 14.9±0.2°, 21.1±0.2°, 26.1±0.2° and 26.9±0.2°, more preferably, the X-ray powder diffraction spectrum comprises substantially the same peaks at diffraction angles (2θ) as shown in Figure 16; wherein an X-ray powder diffraction spectrum of crystal form III of mesylate comprises peaks at diffraction angles (2θ) of 16.7±0.2°, 19.3±0.2°, 23.2±0.2° and 26.5±0.2°, preferably, also comprises peaks at diffraction angles (2θ) of 8.7±0.2°, 19.5±0.2°, 21.8±0.2°, 23.6±0.2° and 24.3±0.2°, more preferably, further also comprises peaks at diffraction angles (2θ) of 11.7±0.2°, 13.6±0.2°, 14.1±0.2°, 17.2±0.2°, 18.7±0.2° and 27.2±0.2°, the most preferably, the X-ray powder diffraction spectrum comprises substantially the same peaks at diffraction angles (2θ) as shown in Figure 17; wherein an X-ray powder diffraction spectrum of crystal form IV of mesylate comprises peaks at diffraction angles (2θ) of 16.8±0.2°, 19.1±0.2°, 19.3±0.2° and 22.1±0.2°, preferably, also comprises peaks at diffraction angles (2θ) of 21.9±0.2°, 23.2±0.2°, 24.4±0.2°, 26.0±0.2° and 27.2±0.2°, more preferably, further also comprises peaks at diffraction angles (2θ) of 8.7±0.2°, 13.4±0.2°, 13.6±0.2°, 19.6±0.2°, 21.6±0.2° and 26.6±0.2°, the most preferably, the X-ray powder diffraction spectrum comprises substantially the same peaks at diffraction angles (2θ) as shown in Figure 19; wherein an X-ray powder diffraction spectrum of crystal form V of mesylate comprises peaks at diffraction angles (2θ) of 25.2±0.2°, 9.3±0.2°, 16.6±0.2° and 19.1±0.2°, preferably, also comprises peaks at diffraction angles (2θ) of 22.7±0.2°, 16.3±0.2°, 21.2±0.2°, 8.9±0.2° and 12.3±0.2°, more preferably, further also comprises peaks at diffraction angles (2θ) of 23.7±0.2°, 20.0±0.2°, 15.9±0.2°, 24.6±0.2°, 28.6±0.2° and 25.5±0.2°, the most preferably, the X-ray powder diffraction spectrum comprises substantially the same peaks at diffraction angles (2θ) as shown in Figure 20.

7. The crystalline acid salt of9-((8-fluoro-6-(1-methyl-1H-pyrazol-4-yl)-[l,2,4]triazolo[4,3-a]pyridine-3-yl)thio)-4-methyl-2H-[l,4]oxaazido[3,2-c]quinoline-3(4H)-one according to claim 2, wherein the acid salt is p-toluenesulfonate, preferably selected from the group consisting of crystal form I, crystal form II and crystal form III of p-toluenesulfonate, wherein an X-ray powder diffraction spectrum of crystal form I of p-toluenesulfonate comprises peaks at diffraction angles (2θ) of 13.0±0.2°, 15.4±0.2°, 24.3±0.2° and 25.7±0.2°, preferably, also comprises peaks at diffraction angles (2θ) of 5.3±0.2°, 12.1±0.2°, 18.4±0.2°, 22.6±0.2° and 23.2±0.2°, more preferably, further also comprises peaks at diffraction angles (2θ) of 14.6±0.2°, 16.9±0.2°, 18.8±0.2°, 19.9±0.2°, 25.3±0.2° and 29.3±0.2°, the most preferably, the X-ray powder diffraction spectrum comprises substantially the same peaks at diffraction angles (2θ) as shown in Figure 21; wherein an X-ray powder diffraction spectrum of crystal form II of p-toluenesulfonate comprises peaks at diffraction angles (2θ) of 13.7±0.2°, 16.1±0.2°, 25.7±0.2° and 26.1±0.2°, preferably, also comprises peaks at diffraction angles (2θ) of 11.0±0.2°, 14.6±0.2°, 17.0±0.2°, 22.8±0.2° and 26.6±0.2°, more preferably, further also comprises peaks at diffraction angles (2θ) of 6.8±0.2°, 20.3±0.2°, 20.8±0.2°, 22.2±0.2°, 24.6±0.2° and 27.9±0.2°, the most preferably, the X-ray powder diffraction spectrum comprises substantially the same peaks at diffraction angles (2θ) as shown in Figure 22; wherein an X-ray powder diffraction spectrum of crystal form III of p-toluenesulfonate comprises peaks at diffraction angles (2θ) of 8.2±0.2°, 14.4±0.2°, 25.9±0.2° and 26.3±0.2°, preferably, also comprises peaks at diffraction angles (2θ) of 10.3±0.2°, 12.8±0.2°, 17.2±0.2°, 18.0±0.2° and 19.9±0.2°, more preferably, further also comprises peaks at diffraction angles (2θ) of 4.8±0.2°, 13.2±0.2°, 15.1±0.2°, 19.3±0.2°, 24.2±0.2° and 24.5±0.2°, the most preferably, the X-ray powder diffraction spectrum comprises substantially the same peaks at diffraction angles (2θ) as shown in Figure 23.

8. The crystalline acid salt of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one according to claim 2, wherein the acid salt is 1,5-naphthalenedisulfonate, preferably selected from the group consisting of crystal form I, crystal form II and crystal form III of 1,5-naphthalenedisulfonate, wherein an X-ray powder diffraction spectrum of crystal form I of 1,5-naphthalenedisulfonate comprises peaks at diffraction angles (2θ) of 10.8±0.2°, 16.8±0.2°, 21.8±0.2° and 25.8±0.2°, preferably, also comprises peaks at diffraction angles (2θ) of 10.2±0.2°, 16.0±0.2°, 19.1±0.2°, 20.8±0.2° and 26.7±0.2°, more preferably, further also comprises peaks at diffraction angles (2θ) of 8.1±0.2°, 13.6±0.2°, 18.2±0.2°, 18.7±0.2°, 26.4±0.2° and 30.9±0.2°, the most preferably, the X-ray powder diffraction spectrum comprises substantially the same peaks at diffraction angles (2θ) as shown in Figure 24; wherein an X-ray powder diffraction spectrum of crystal form II of 1,5-naphthalenedisulfonate comprises peaks at diffraction angles (2θ) of 4.2±0.2°, 16.4±0.2°, 22.8±0.2° and 27.3±0.2°, preferably, also comprises peaks at diffraction angles (2θ) of 8.5±0.2°, 17.8±0.2°, 19.1±0.2°, 22.3±0.2° and 28.1±0.2°, more preferably, further also comprises peaks at diffraction angles (2θ) of 10.4±0.2°, 13.5±0.2°, 15.1±0.2°, 21.2±0.2°, 24.0±0.2° and 26.5±0.2°, the most preferably, the X-ray powder diffraction spectrum comprises substantially the same peaks at diffraction angles (2θ) as shown in Figure 25; wherein an X-ray powder diffraction spectrum of crystal form III of 1,5-naphthalenedisulfonate comprises peaks at diffraction angles (2θ) of 13.0±0.2°, 22.7±0.2°, 24.1±0.2° and 25.7±0.2°, preferably, also comprises peaks at diffraction angles (2θ) of 15.4±0.2°, 18.8±0.2°, 23.2±0.2°, 25.4±0.2° and 26.5±0.2°, more preferably, further also comprises peaks at diffraction angles (2θ) of 12.6±0.2°, 14.5±0.2°, 16.9±0.2°, 18.5±0.2°, 20.0±0.2° and 21.4±0.2°, the most preferably, the X-ray powder diffraction spectrum comprises substantially the same peaks at diffraction angles (2θ) as shown in Figure 26.

9. A method for preparing a crystalline free base of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one, comprising the following steps of:
dissolving 9-((8-fluorine-6-(1-methyl-1H-pyrazol-4-yl)-[1,2,4]triazolo[4,3-a]pyridin-3-yl)thio)-4-methyl-2H-[1,4]oxaazino[3,2-c]quinoline-3(4H)-one in an aqueous solvent, an organic solvent or a solvent mixture under heating, then cooling the solution or mixing the solution with an anti-solvent to obtain the crystalline free base; or
evaporating a solution or suspension of 9-((8-fluorine-6-(1-methyl-1H-pyrazol-4-yl)-[1,2,4]triazolo[4,3-a]pyridin-3-yl)thio)-4-methyl-2H-[1,4]oxaazino[3,2-c]quinoline-3(4H)-one rapidly or slowly to obtain the crystalline free base; or
adding an original compound solid or other solid particle additive as a heteronuclear crystal seed to a solution of 9-((8-fluorine-6-(1-methyl-1H-pyrazol-4-yl)-[1,2,4]triazolo[4,3-a]pyridin-3-yl)thio)-4-methyl-2H-[1,4]oxaazino[3,2-c]quinoline-3(4H)-one to induce the crystalline free base; or
dispersing 9-((8-fluorine-6-(1-methyl-1H-pyrazol-4-yl)-[1,2,4]triazolo[4,3-a]pyridin-3-yl)thio)-4-methyl-2H-[1,4]oxaazino[3,2-c]quinoline-3(4H)-one in an aqueous solvent, an organic solvent, a solvent mixture or an atmosphere of these media to obtain the crystalline free base; or
heating, sublimating, grinding, freezing or fusing-cooling 9-((8-fluorine-6-(1-methyl-1H-pyrazol-4-yl)-[1,2,4]triazolo[4,3-a]pyridin-3-yl)thio)-4-methyl-2H-[1,4]oxaazino[3,2-c]quinoline-3(4H)-one to obtain the crystalline free base; or
or combining the above methods to obtain the crystalline free base;
wherein the organic solvent is preferably selected from the group consisting of alcohols, chloralkanes, ketones, ethers, cyclic ethers, esters, alkanes, cycloalkanes, benzenes, amides, sulfoxides and the mixture thereof, more preferably methanol, ethanol, n-propanol, isopropanol, n-butanol, acetonitrile, acetone, methyl ethyl ketone, tetrahydrofuran, dioxane, N,N-dimethylcarboxamide, dimethyl sulfoxide, ethyl acetate, dichloromethane, trichloroethane, carbon tetrachloride, n-heptane, n-hexane, iso-octane, pentane, cyclohexane, cyclopentane, diethyl ether, methyltertbutyl ether, isopropyl ether, benzene, toluene, xylene and the mixture thereof.

10. A method for preparing a crystalline acid salt of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one, comprising the following steps of:
1) dissolving or dispersing a free base of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one in an aqueous solvent or a suitable organic solvent, then adding a liquid or solid inorganic acid or organic acid or a solution of inorganic acid or organic acid to the above system to obtain an acid salt of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one; or
adding a free base solid of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one to an acid solution to obtain an acid salt of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one;
2) collecting the solid product precipitated during the salt-forming process, or obtaining the solid product by creating supersaturation of the salt-forming system to prepare the crystalline acid salt of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one, wherein a method for creating supersaturation comprises evaporation of solvent, or addition of an anti-solvent, or a cooling method;
and / or,
transforming one crystal form of the acid salt into another crystal form of the acid salt by a crystal transformation method, wherein the crystal transformation method comprises:
heating or a crystal transformation method of a suspension in a suitable solvent;
wherein the suitable organic solvent of the salt-forming process in step 1) is selected from the group consisting of alcohols, chloralkanes, ketones, ethers, cyclic ethers, esters, alkanes, cycloalkanes, benzenes, amides, sulfoxides and the mixture thereof, preferably methanol, ethanol, n-propanol, isopropanol, acetonitrile, acetone, 1,4-dioxane, tetrahydrofuran, N,N-dimethylcarboxamide, ethyl acetate, isopropyl acetate, 2-methoxyethyl ether and the mixture thereof;
wherein the "acid salt" comprises an inorganic acid salt or an organic acid salt; wherein the inorganic acid salt is preferably selected from the group consisting of hydrochloride, sulfate, hydrobromide, hydrofluoride, hydroiodide and phosphate, more preferably hydrochloride, sulfate and phosphate; wherein the organic acid salt is preferably selected from the group consisting of 2,5-dihydroxybenzeneformate, 1-hydroxy-2-naphthaleneformate, acetate, dichloroacetate, trichloroacetate, acetohydroxamate, adipate, benzene sulfonate, 4-chlorobenzene sulfonate, benzeneformate, 4-acetamidobenzeneformate, 4-aminobenzeneformate, caprate, caproate, caprilate, cinnamoate, citrate, cyclohexylsulfamate, camphorsulfonate, aspartate, camphorate, gluconate, glucuronate, glutamate, erythorbate, lactate, aspartate, malate, mandelate, pyroglutamate, tartrate, lauryl sulfate, dibenzoyltartrate, ethyl-1,2-disulfonate, esylate, formate, fumarate, galactonate, gentisate, glutarate, 2-oneglutarate, glycollate, hippurate, isethionate, lactobionate , ascorbate, aspartate, laurate, camphorate, maleate, malonate, mesylate, 1,5-naphthalenedisulfonate, naphthalene-2-sulfonate, nicotinate, oleate, orotate, oxalate, palmitate, embonate, propionate, salicylate, 4-aminosalicylate, sebacate, stearate, butanedioate, thiocyanate, undecylenate, trifluoroacetate, succinate and p-toluenesulfonate, more preferably mesylate, p-toluenesulfonate and 1,5-naphthalenedisulfonate.

11. The preparation method according to claim 10, **characterized in that**
a) a suitable solvent is added to the free base of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one, then an equal or excess molar equivalent of methanesulfonic acid is added, and then the mixture is stirred, after the salt-forming reaction is completed, a solid-liquid separation is carried out to obtain crystal form I of mesylate, wherein the suitable solvent is selected from the group consisting of acetone, tetrahydrofuran, isopropyl acetate, ethyl acetate, 2-methoxyethyl ether, 1,4-dioxane and the mixture thereof, or
b) a methanol-water solution with volume ratio: 5%-95% is added to the free base of the 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one, the mixture is stirred, an equal or excess molar equivalent of methanesulfonic acid is added until the solution is clear, after mesylate is precipitated, a solid-liquid separation is carried out to obtain crystal form II of mesylate or
c) methanol is added to the free base of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one, then an equal or excess molar equivalent of methanesulfonic acid is added slowly, after the compound is dissolved, a crystal seed of crystal form III of mesylate is added immediately, the mixture is stirred, after the salt-forming reaction is completed, a solid-liquid separation is carried out to obtain crystal form III of mesylate; or
in the absence of a crystal seed, crystal form IV of mesylate is obtained firstly, then crystal form IV of mesylate is dried in vacuum at 100-120°C, overnight, crystal form IV of mesylate is transformed to crystal form III of mesylate; or
crystal form V of mesylate is dispersed in a single or mixed anti-solvent, then the mixture is stirred at room temperature or under heating, crystal form V of mesylate is transformed to crystal form III of mesylate, or
or d) methanol is added to the free base of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one, an equal or excess molar equivalent of methanesulfonic acid is added slowly, the mixture is stirred, after the salt-forming reaction is completed, a solid-liquid separation is carried out to obtain crystal form IV of mesylate, or
e) dimethyl sulfoxide is added to the free base of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one, then an equal or excess molar equivalent of methanesulfonic acid is added slowly, the mixture is stirred, after the salt-forming reaction is completed, a suitable amount of ethyl acetate is added and the stirring is continued, then a solid-liquid separation is carried out to obtain crystal form V of mesylate.

12. A pharmaceutical composition, **characterized in that** it comprises a therapeutically effective amount of the crystalline free base of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one according to claim 1 or the crystalline acid salt or polymorph thereof according to any one of claims 2-8, and a pharmaceutically acceptable carrier or excipient.

13. The crystalline free base of 9-((8-fluoro-6-(1-methyl-1H-pyrazol-4-yl)-[1,2,4]triazolo[4,3-a]pyridine-3-yl)thio)-4-methyl-2H-[1,4]oxaazido[3,2-c]quinoline-3(4H)-one or polymorph thereof according to claim 1, the crystalline acid salt or polymorph thereof according to any one of claims 2-8, or the pharmaceutical composition according to claim 12 for use in the treatment of cancer and metastasis, including cancer, hematopoietic tumor of lymphatic system, hematopoietic tumor of bone marrow system, mesenchymal tumor, central and peripheral nervous system tumor or other tumor;
preferably, wherein the cancer is selected from the group consisting of bladder cancer, breast cancer, colon cancer, kidney cancer, liver cancer, lung cancer, stomach cancer and skin cancer; wherein the hematopoietic tumor of lymphatic system is selected from the group consisting of leukemia, acute lymphocytic leukemia and chronic lymphocytic leukemia; wherein the hematopoietic tumor of bone marrow system is selected from the group consisting of acute or chronic myelogenous leukemia, myelodysplastic syndrome and promyelocytic leukemia; wherein the mesenchymal tumor is selected from the group consisting of fibrosarcoma, rhabdomyosarcoma, soft tissue sarcoma and osteosarcoma; wherein the central and peripheral nervous system tumor is selected from the group consisting of astrocytoma, neuroblastoma, glioma and nerve ending tumor; wherein the other tumor is selected from the group consisting of malignant melanoma, seminoma, teratocarcinoma, thyroid follicular cancer and Kaposi's sarcoma; preferably liver cancer, lung cancer, breast cancer, squamous cell carcinoma of the skin and stomach cancer.

## Patentansprüche

1. Kristalline freie Base von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on, wobei die kristalline freie Base ausgewählt ist aus der Gruppe bestehend aus der Kristallform I, der Kristallform II, der Kristallform III und der Kristallform IV, wobei ein Pulverröntgenbeugungsspektrum der Kristallform I der kristallinen freien Base Peaks bei Beugungswinkeln (2θ) von 13,0±0,2°, 17,9±0,2°, 21,2±0,2° und 31,4±0,2° umfasst, vorzugsweise auch Peaks bei Beugungswinkeln (2θ) von 10,3±0,2°, 11,1±0,2°, 23,3±0,2°, 23,8±0,2° und 33,6±0,2° umfasst, noch mehr bevorzugt weiterhin auch Peaks bei Beugungswinkeln (2θ) von 15,7±0,2°, 17,7±0,2°, 26,8±0,2°, 28,0±0,2°, 31,7±0,2° und 32,8±0,2° umfasst, wobei das Pulverröntgenbeugungsspektrum am meisten bevorzugt im Wesentlichen dieselben Peaks bei Beugungswinkeln (2θ) aufweist, wie sie in Figur 1 aufgeführt sind; wobei ein Pulverröntgenbeugungsspektrum der Kristallform II der kristallinen freien Base Peaks bei Beugungswinkeln (2θ) von 8,6±0,2°, 11,5±0,2°, 14,1±0,2° und 19,8±0,2° umfasst, vorzugsweise auch Peaks bei Beugungswinkeln (2θ) von 11,9±0,2°, 14,7±0,2°, 15,2±0,2°, 17,2±0,2° und 18,9±0,2° umfasst, noch mehr bevorzugt weiterhin auch Peaks bei Beugungswinkeln (2θ) von 5,8±0,2°, 7,4±0,2°, 20,9±0,2°, 30,9±0,2°, 31,4±0,2° und 37,9±0,2° umfasst, wobei das Pulverröntgenbeugungsspektrum am meisten bevorzugt im Wesentlichen dieselben Peaks bei Beugungswinkeln (2θ) aufweist, wie sie in Figur 4 aufgeführt sind; wobei ein Pulverröntgenbeugungsspektrum der Kristallform III der kristallinen freien Base Peaks bei Beugungswinkeln (2θ) von 12,8±0,2°, 14,8±0,2°, 18,0±0,2° und 20,5±0,2° umfasst, vorzugsweise auch Peaks bei Beugungswinkeln (2θ) von 8,9±0,2°, 9,2±0,2°, 10,6±0,2°, 15,8±0,2° und 20,7±0,2° umfasst, noch mehr bevorzugt weiterhin auch Peaks bei Beugungswinkeln (2θ) von 5,3±0,2°, 5,9±0,2°, 12,0±0,2°, 14,0±0,2°, 17,3±0,2° und 19,9±0,2° umfasst, wobei das Pulverröntgenbeugungsspektrum am meisten bevorzugt im Wesentlichen dieselben Peaks bei Beugungswinkeln (2θ) aufweist, wie sie in Figur 5 aufgeführt sind; wobei ein Pulverröntgenbeugungsspektrum der Kristallform IV der kristallinen freien Base Peaks bei Beugungswinkeln (2θ) von 8,9±0,2°, 12,6±0,2°, 17,0±0,2° und 17,9±0,2° umfasst, vorzugsweise auch Peaks bei Beugungswinkeln (2θ) von 13,2±0,2°, 14,5±0,2°, 20,5±0,2°, 23,9±0,2° und 26,3±0,2° umfasst, wobei das Pulverröntgenbeugungsspektrum am meisten bevorzugt im Wesentlichen dieselben Peaks bei Beugungswinkeln (2θ) aufweist, wie sie in Figur 7 aufgeführt sind.

2. Kristallines saures Salz von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on, wobei das saure Salz ein anorganisches saures Salz oder ein organisches saures Salz umfasst; wobei das anorganische saure Salz vorzugsweise ausgewählt ist aus der Gruppe bestehend aus Hydrochlorid, Sulfat, Hydrobromat, Hydrofluorid, Hydroiodid und Phosphat, noch mehr bevorzugt ausgewählt ist aus der Gruppe bestehend aus Hydrochlorid, Sulfat und Phosphat; wobei das organische saure Salz vorzugsweise ausgewählt ist aus der Gruppe bestehend aus 2,5-Dihydroxybenzolformiat, 1-Hydroxy-2-naphthalinformiat, Acetat, Dichloracetat, Trichloracetat, Acetohydroxamat, Adipat, Benzolsulfonat, 4-Chlorbenzolsulfonat, Benzolformiat, 4-Acetamidobenzolformiat, 4-Aminobenzolformiat, Caprat, Caproat, Caprylat, Cinnamoat, Citrat, Cyclohexylsulfamat, Camphersulfonat, Aspartat, Camphorat, Gluconat, Glucuronat, Glutamat, Erythrobat, Lactat, Aspartat, Malat, Mandelat, Pyroglutamat, Tartrat, Laurylsulfat, Dibenzoyltartrat, Ethyl-1,2-disulfonat, Esylat, Formiat, Fumarat, Galactonat, Gentisat, Glutarat, 2-Oxoglutarat, Glycollat, Hippurat, Isethionat, Lactobionat, Ascorbat, Aspartat, Laurat, Camphorat, Maleat, Malonat, Mesylat, 1,5-Naphthalindisulfonat, Naphthalin-2-sulfonat, Nicotinat, Oleat, Orotat, Oxalat, Palmitat, Embonat, Propionat, Salicylat, 4-Aminosalicylat, Sebacat, Stearat, Butanedioat, Thiocyanat, Undecylenat, Trifluoracetat, Succinat und p-Toluolsulfonat, wobei es noch mehr bevorzugt ausgewählt ist aus der Gruppe bestehend aus Mesylat, p-Toluolsulfonat und 1,5-Naphthalindisulfonat.

3. Kristallines saures Salz von 9-((8-Fluor-6-(1-methyl-1H-pyrazol-4-yl)-[1.2.4]triazolo[4,3-a]pyridin-3-yl)thio)-4-methyl-2H-[1,4]oxaazido[3,2-c]chinolin-3(4H)-on nach Anspruch 2, **dadurch gekennzeichnet, dass** es sich beim sauren Salz um die Kristallform I des Hydrochlorids mit einem Pulverröntgenbeugungsspektrum handelt, das Peaks bei Beugungswinkeln (2θ) von 8,1±0,2°, 19,2±0,2°, 24,1±0,2° und 26,2±0,2° aufweist, das vorzugsweise auch Peaks bei Beugungswinkeln (2θ) von 9,1±0,2°, 11,3±0,2°, 13,4±0,2°, 29,7±0,2° und 23,4±0,2° aufweist, wobei das Pulverröntgenbeugungsspektrum am meisten bevorzugt im Wesentlichen dieselben Peaks bei Beugungswinkeln (2θ) aufweist, wie sie in Figur 8 aufgeführt sind.

4. Kristallines saures Salz von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on nach Anspruch 2, wobei es sich beim sauren Salz um das Sulfat handelt, das vorzugsweise ausgewählt ist aus der Gruppe bestehend aus der Kristallform I und der Kristallform II des Sulfats, wobei ein Pulverröntgenbeugungsspektrum der Kristallform I des Sulfats Peaks bei Beugungswinkeln (2θ) von 18,4±0,2°, 19,7±0,2°, 23,8±0,2° und 24,5±0,2° umfasst, vorzugsweise auch Peaks bei Beugungswinkeln (2θ) von 12,8±0,2°, 14,4±0,2°, 17,0±0,2°, 20,0±0,2° und 21,0±0,2° umfasst, noch mehr bevorzugt weiterhin auch Peaks bei Beugungswinkeln (2θ) von 8,7±0,2°, 13,2±0,2°, 19,1±0,2°, 26,3±0,2°, 26,6±0,2° und 29,0±0,2° umfasst, wobei das Pulverröntgenbeugungsspektrum am meisten bevorzugt im Wesentlichen dieselben Peaks bei Beugungswinkeln (2θ) aufweist, wie sie in Figur 9 aufgeführt sind; wobei ein Pulverröntgenbeugungsspektrum der Kristallform II des Sulfats Peaks bei Beugungswinkeln (2θ) von 6,3±0,2°, 8,7±0,2°, 12,7±0,2° und 18,4±0,2° umfasst, vorzugsweise auch Peaks bei Beugungswinkeln (2θ) von 8,3±0,2°, 17,5±0,2°, 18,7±0,2°, 20,4±0,2° und 25,6±0,2° umfasst, noch mehr bevorzugt weiterhin auch Peaks bei Beugungswinkeln (2θ) von 9,1±0,2°, 15,9±0,2°, 16,8±0,2°, 24,0±0,2°, 25,2±0,2° und 28,4±0,2° umfasst, wobei das Pulverröntgenbeugungsspektrum am meisten bevorzugt im Wesentlichen dieselben Peaks bei Beugungswinkeln (2θ) aufweist, wie sie in Figur 10 aufgeführt sind.

5. Kristallines saures Salz von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on nach Anspruch 2, wobei es sich beim sauren Salz um das Phosphat handelt, das vorzugsweise ausgewählt ist aus der Gruppe bestehend aus der Kristallform I, der Kristallform II, der Kristallform II und der Kristallform IV des Phosphats, wobei ein Pulverröntgenbeugungsspektrum der Kristallform I des Phosphats Peaks bei Beugungswinkeln (2θ) von 7,9±0,2°, 12,8±0,2°, 15,9±0,2° und 18,3±0,2° umfasst, vorzugsweise auch Peaks bei Beugungswinkeln (2θ) von 5,3±0,2°, 10,6±0,2°, 13,4±0,2°, 20,9±0,2° und 24,7±0,2° umfasst, noch mehr bevorzugt weiterhin auch Peaks bei Beugungswinkeln (2θ) von 16,5±0,2°, 18,7±0,2°, 20,6±0,2°, 21,8±0,2°, 26,2±0,2° und 27,4±0,2° umfasst, wobei das Pulverröntgenbeugungsspektrum am meisten bevorzugt im Wesentlichen dieselben Peaks bei Beugungswinkeln (2θ) aufweist, wie sie in Figur 11 aufgeführt sind; wobei ein Pulverröntgenbeugungsspektrum der Kristallform II des Phosphats Peaks bei Beugungswinkeln (2θ) von 13,7±0,2°, 16,1±0,2°, 22,8±0,2° und 26,1±0,2° umfasst, vorzugsweise auch Peaks bei Beugungswinkeln (2θ) von 11,0±0,2°, 14,6±0,2°, 20,3±0,2°, 20,8±0,2° und 25,7±0,2° umfasst, noch mehr bevorzugt weiterhin auch Peaks bei Beugungswinkeln (2θ) von 6,8±0,2°, 17,0±0,2°, 22,2±0,2°, 26,6±0,2°, 27,9±0,2° und 31,2±0,2° umfasst, wobei das Pulverröntgenbeugungsspektrum am meisten bevorzugt im Wesentlichen dieselben Peaks bei Beugungswinkeln (2θ) aufweist, wie sie in Figur 12 aufgeführt sind; wobei ein Pulverröntgenbeugungsspektrum der Kristallform III des Phosphats Peaks bei Beugungswinkeln (2θ) von 9,7±0,2°, 15,6±0,2°, 16,8±0,2° und 24,6±0,2° umfasst, vorzugsweise auch Peaks bei Beugungswinkeln (2θ) von 4,8±0,2°, 21,2±0,2°, 25,0±0,2°, 27,8±0,2° und 28,1±0,2° umfasst, noch mehr bevorzugt weiterhin auch Peaks bei Beugungswinkeln (2θ) von 5,2±0,2°, 12,8±0,2°, 14,5±0,2°, 18,0±0,2°, 20,1±0,2° und 23,5±0,2° umfasst, wobei das Pulverröntgenbeugungsspektrum am meisten bevorzugt im Wesentlichen dieselben Peaks bei Beugungswinkeln (2θ) aufweist, wie sie in Figur 13 aufgeführt sind; wobei ein Pulverröntgenbeugungsspektrum der Kristallform IV des Phosphats Peaks bei Beugungswinkeln (2θ) von 7,8±0,2°, 17,9±0,2°, 25,0±0,2° und 27,7±0,2° umfasst, vorzugsweise auch Peaks bei Beugungswinkeln (2θ) von 6,7±0,2°, 10,8±0,2°, 15,6±0,2°, 23,4±0,2° und 24,6±0,2° umfasst, noch mehr bevorzugt weiterhin auch Peaks bei Beugungswinkeln (2θ) von 5,2±0,2°, 12,8±0,2°, 20,9±0,2°, 21,7±0,2°, 22,3±0,2° und 26,8±0,2° umfasst, wobei das Pulverröntgenbeugungsspektrum am meisten bevorzugt im Wesentlichen dieselben Peaks bei Beugungswinkeln (2θ) aufweist, wie sie in Figur 14 aufgeführt sind.

6. Kristallines saures Salz von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on nach Anspruch 2, wobei es sich beim sauren Salz um das Mesylat handelt, das vorzugsweise ausgewählt ist aus der Gruppe bestehend aus der Kristallform I, der Kristallform II, der Kristallform III, der Kristallform IV und der Kristallform V des Mesylats, wobei ein Pulverröntgenbeugungsspektrum der Kristallform I des Mesylats Peaks bei Beugungswinkeln (2θ) von 15,6±0,2°, 17,0±0,2°, 25,6±0,2° und 26,0±0,2° umfasst, vorzugsweise auch Peaks bei Beugungswinkeln (2θ) von 9,8±0,2°, 21,8±0,2°, 23,5±0,2°, 23,8±0,2° und 27,5±0,2° umfasst, noch mehr bevorzugt weiterhin auch Peaks bei Beugungswinkeln (2θ) von 6,6±0,2°, 15,3±0,2°, 17,2±0,2°, 18,3±0,2°, 19,7±0,2° und 26,4±0,2° umfasst, wobei das Pulverröntgenbeugungsspektrum am meisten bevorzugt im Wesentlichen dieselben Peaks bei Beugungswinkeln (2θ) aufweist, wie sie in Figur 15 aufgeführt sind; wobei ein Pulverröntgenbeugungsspektrum der Kristallform II des Mesylats Peaks bei Beugungswinkeln (2θ) von 9,4±0,2°, 17,0±0,2°, 18,9±0,2° und 27,3±0,2° umfasst, vorzugsweise auch Peaks bei Beugungswinkeln (2θ) von 6,6±0,2°, 14,9±0,2°, 21,1±0,2°, 26,1±0,2° und 26,9±0,2° umfasst, wobei das Pulverröntgenbeugungsspektrum noch mehr bevorzugt im Wesentlichen dieselben Peaks bei Beugungswinkeln (2θ) aufweist, wie sie in Figur 16 aufgeführt sind; wobei ein Pulverröntgenbeugungsspektrum der Kristallform III des Mesylats Peaks bei Beugungswinkeln (2θ) von 16,7±0,2°, 19,3±0,2°, 23,2±0,2° und 26,5±0,2° umfasst, vorzugsweise auch Peaks bei Beugungswinkeln (2θ) von 8,7±0,2°, 19,5±0,2°, 21,8±0,2°, 23,6±0,2° und 24,3±0,2° umfasst, noch mehr bevorzugt weiterhin auch Peaks bei Beugungswinkeln (2θ) von 11,7±0,2°, 13,6±0,2°, 14,1±0,2°, 17,2±0,2°, 18,7±0,2° und 27,2±0,2° umfasst, wobei das Pulverröntgenbeugungsspektrum am meisten bevorzugt im Wesentlichen dieselben Peaks bei Beugungswinkeln (2θ) aufweist, wie sie in Figur 17 aufgeführt sind; wobei ein Pulverröntgenbeugungsspektrum der Kristallform IV des Mesylats Peaks bei Beugungswinkeln (2θ) von 16,8±0,2°, 19,1±0,2°, 19,3±0,2° und 22,1±0,2° umfasst, vorzugsweise auch Peaks bei Beugungswinkeln (2θ) von 21,9±0,2°, 23,2±0,2°, 24,4±0,2°, 26,0±0,2° und 27,2±0,2° umfasst, noch mehr bevorzugt weiterhin auch Peaks bei Beugungswinkeln (2θ) von 8,7±0,2°, 13,4±0,2°, 13,6±0,2°, 19,6±0,2°, 21,6±0,2° und 26,6±0,2° umfasst, wobei das Pulverröntgenbeugungsspektrum am meisten bevorzugt im Wesentlichen dieselben Peaks bei Beugungswinkeln (2θ) aufweist, wie sie in Figur 19 aufgeführt sind; wobei ein Pulverröntgenbeugungsspektrum der Kristallform V des Mesylats Peaks bei Beugungswinkeln (2θ) von 25,2±0,2°, 9,3±0,2°, 16,6±0,2° und 19,1±0,2° umfasst, vorzugsweise auch Peaks bei Beugungswinkeln (2θ) von 22,7±0,2°, 16,3±0,2°, 21,2±0,2°, 8,9±0,2° und 12,3±0,2° umfasst, noch mehr bevorzugt weiterhin auch Peaks bei Beugungswinkeln (2θ) von 23,7±0,2°, 20,0±0,2°, 15,9±0,2°, 24,6±0,2°, 28,6±0,2° und 25,5±0,2° umfasst, wobei das Pulverröntgenbeugungsspektrum am meisten bevorzugt im Wesentlichen dieselben Peaks bei Beugungswinkeln (2θ) aufweist, wie sie in Figur 20 aufgeführt sind.

7. Kristallines saures Salz von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on nach Anspruch 2, wobei es sich beim sauren Salz um das p-Toluolsulfonat handelt, das vorzugsweise ausgewählt ist aus der Gruppe bestehend aus der Kristallform I, der Kristallform II und der Kristallform III des p-Toluolsulfonats, wobei ein Pulverröntgenbeugungsspektrum der Kristallform I des p-Toluolsulfonats Peaks bei Beugungswinkeln (2θ) von 13,0±0,2°, 15,4±0,2°, 24,3±0,2° und 25,7±0,2° umfasst, vorzugsweise auch Peaks bei Beugungswinkeln (2θ) von 5,3±0,2°, 12,1±0,2°, 18,4±0,2°, 22,6±0,2° und 23,2±0,2° umfasst, noch mehr bevorzugt weiterhin auch Peaks bei Beugungswinkeln (2θ) von 14,6±0,2°, 16,9±0,2°, 18,8±0,2°, 19,9±0,2°, 25,3±0,2° und 29,3±0,2° umfasst, wobei das Pulverröntgenbeugungsspektrum am meisten bevorzugt im Wesentlichen dieselben Peaks bei Beugungswinkeln (2θ) aufweist, wie sie in Figur 21 aufgeführt sind; wobei ein Pulverröntgenbeugungsspektrum der Kristallform II des p-Toluolsulfonats Peaks bei Beugungswinkeln (2θ) von 13,7±0,2°, 16,1±0,2°, 25,7±0,2° und 26,1±0,2° umfasst, vorzugsweise auch Peaks bei Beugungswinkeln (2θ) von 11,0±0,2°, 14,6±0,2°, 17,0±0,2°, 22,8±0,2° und 26,6±0,2° umfasst, noch mehr bevorzugt weiterhin auch Peaks bei Beugungswinkeln (2θ) von 6,8±0,2°, 20,3±0,2°, 20,8±0,2°, 22,2±0,2°, 24,6±0,2° und 27,9±0,2° umfasst, wobei das Pulverröntgenbeugungsspektrum am meisten bevorzugt im Wesentlichen dieselben Peaks bei Beugungswinkeln (2θ) aufweist, wie sie in Figur 22 aufgeführt sind; wobei ein Pulverröntgenbeugungsspektrum der Kristallform III des p-Toluolsulfonats Peaks bei Beugungswinkeln (2θ) von 8,2±0,2°, 14,4±0,2°, 25,9±0,2° und 26,3±0,2° umfasst, vorzugsweise auch Peaks bei Beugungswinkeln (2θ) von 10,3±0,2°, 12,8±0,2°, 17,2±0,2°, 18,0±0,2° und 19,9±0,2° umfasst, noch mehr bevorzugt weiterhin auch Peaks bei Beugungswinkeln (2θ) von 4,8±0,2°, 13,2±0,2°, 15,1±0,2°, 19,3±0,2°, 24,2±0,2° und 24,5±0,2° umfasst, wobei das Pulverröntgenbeugungsspektrum am meisten bevorzugt im Wesentlichen dieselben Peaks bei Beugungswinkeln (2θ) aufweist, wie sie in Figur 23 aufgeführt sind.

8. Kristallines saures Salz von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on nach Anspruch 2, wobei es sich beim sauren Salz um das 1,5-Naphthalindisulfonat handelt, das vorzugsweise ausgewählt ist aus der Gruppe bestehend aus der Kristallform I, der Kristallform II und der Kristallform III des 1,5-Naphthalindisulfonats, wobei ein Pulverröntgenbeugungsspektrum der Kristallform I des 1,5-Naphthalindisulfonats Peaks bei Beugungswinkeln (2θ) von 10,8±0,2°, 16,8±0,2°, 21,8±0,2° und 25,8±0,2° umfasst, vorzugsweise auch Peaks bei Beugungswinkeln (2θ) von 10,2±0,2°, 16,0±0,2°, 19,1±0,2°, 20,8±0,2° und 26,7±0,2° umfasst, noch mehr bevorzugt weiterhin auch Peaks bei Beugungswinkeln (2θ) von 8,1±0,2°, 13,6±0,2°, 18,2±0,2°, 18,7±0,2°, 26,4±0,2° und 30,9±0,2° umfasst, wobei das Pulverröntgenbeugungsspektrum am meisten bevorzugt im Wesentlichen dieselben Peaks bei Beugungswinkeln (2θ) aufweist, wie sie in Figur 24 aufgeführt sind; wobei ein Pulverröntgenbeugungsspektrum der Kristallform II des 1,5-Naphthalindisulfonats Peaks bei Beugungswinkeln (2θ) von 4,2±0,2°, 16,4±0,2°, 22,8±0,2° und 27,3±0,2° umfasst, vorzugsweise auch Peaks bei Beugungswinkeln (2θ) von 8,5±0,2°, 17,8±0,2°, 19,1±0,2°, 22,3±0,2° und 28,1±0,2° umfasst, noch mehr bevorzugt weiterhin auch Peaks bei Beugungswinkeln (2θ) von 10,4±0,2°, 13,5±0,2°, 15,1±0,2°, 21,2±0,2°, 24,0±0,2° und 26,5±0,2° umfasst, wobei das Pulverröntgenbeugungsspektrum am meisten bevorzugt im Wesentlichen dieselben Peaks bei Beugungswinkeln (2θ) aufweist, wie sie in Figur 25 aufgeführt sind; wobei ein Pulverröntgenbeugungsspektrum der Kristallform III des 1,5-Naphthalindisulfonats Peaks bei Beugungswinkeln (2θ) von 13,0±0,2°, 22,7±0,2°, 24,1±0,2° und 25,7±0,2° umfasst, vorzugsweise auch Peaks bei Beugungswinkeln (2θ) von 15,4±0,2°, 18,8±0,2°, 23,2±0,2°, 25,4±0,2° und 26,5±0,2° umfasst, noch mehr bevorzugt weiterhin auch Peaks bei Beugungswinkeln (2θ) von 12,6±0,2°, 14,5±0,2°, 16,9±0,2°, 18,5±0,2°, 20,0±0,2° und 21,4±0,2° umfasst, wobei das Pulverröntgenbeugungsspektrum am meisten bevorzugt im Wesentlichen dieselben Peaks bei Beugungswinkeln (2θ) aufweist, wie sie in Figur 26 aufgeführt sind.

9. Verfahren zur Herstellung einer kristallinen freien Base von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on, umfassend die folgenden Schritte des:
Lösens von 9-((8-Fluor-6-(1-methyl-1H-pyrazol-4-yl)-[1,2,4]triazolo[4,3-a]pyridin-3-yl)thio)-4-methyl-2H-[1,4]oxaazino[3,2-c]chinolin-3(4H)-on in einem wässrigen Lösungsmittel, einem organischen Lösungsmittel oder einem Lösungsmittelgemisch unter Erwärmen, dann des Abkühlens der Lösung oder des Mischens der Lösung mit einem Antilösungsmittel, wodurch die kristalline freie Base erhalten wird; oder des
schnellen oder langsamen Einengens einer Lösung oder Suspension von 9-((8-Fluor-6-(1-methyl-1H-pyrazol-4-yl)-[1.2.4]triazolo[4,3-a]pyridin-3-yl)thio)-4-methyl-2H-[1,4]oxaazino[3,2-c]chinolin-3(4H)-on, wodurch die kristalline freie Base erhalten wird; oder des
Zugebens einer festen Originalverbindung oder eines anderen festen Partikeladditivs als heteronukleärer Kristallkeim zu einer Lösung von 9-((8-Fluor-6-(1-methyl-1H-pyrazol-4-yl)-[1.2.4]triazolo[4,3-a]pyridin-3-yl)thio)-4-methyl-2H-[1,4]oxaazino[3,2-c]chinolin-3(4H)-on, wodurch die kristalline freie Base induziert wird; oder des
Dispergierens von 9-((8-Fluor-6-(1-methyl-1H-pyrazol-4-yl)-[1,2,4]triazolo[4,3-a]pyridin-3-yl)thio)-4-methyl-2H-[1,4]oxaazino[3,2-c]chinolin-3(4H)-on in einem wässrigen Lösungsmittel, einem organischen Lösungsmittel, einem Lösungsmittelgemisch oder einer Atmosphäre dieser Medien, wodurch die kristalline freie Base erhalten wird; oder des
Erwärmens, Sublimierens, Mahlens, Gefrierens oder Schmelzens/Abkühlens von 9-((8-Fluor-6-(1-methyl-1H-pyrazol-4-yl)-[1.2.4]triazolo[4,3-a]pyridin-3-yl)thio)-4-methyl-2H-[1,4]oxaazino[3,2-c]chinolin-3(4H)-on, wodurch die kristalline freie Base erhalten wird; oder des
Kombinierens der obigen Verfahren, wodurch die kristalline freie Base erhalten wird;
wobei das organische Lösungsmittel vorzugsweise ausgewählt ist aus der Gruppe bestehend aus Alkoholen, Chloralkanen, Ketonen, Ethern, cyclischen Ethern, Estern, Alkanen, Cycloalkanen, Benzolen, Amiden, Sulfoxiden und einer Mischung davon, noch mehr bevorzugt Methanol, Ethanol, n-Propanol, Isopropanol, n-Butanol, Acetonitril, Aceton, Methylethylketon, Tetrahydrofuran, Dioxan, N,N-Dimethylcarboxamid, Dimethylsulfoxid, Ethylacetat, Dichlormethan, Trichlorethan, Kohlenstofftetrachlorid, n-Heptan, n-Hexan, iso-Octan, Pentan, Cyclohexan, Cyclopentan, Diethylether, Methyltertbutylether, Isopropylether, Benzol, Toluol, Xylol und einer Mischung davon.

10. Verfahren zur Herstellung eines kristallinen sauren Salzes von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on, umfassend die folgenden Schritte des:
1) Lösens oder Dispergierens einer freien Base von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on in einem wässrigen Lösungsmittel oder einem geeigneten organischen Lösungsmittel, dann des Zugebens einer flüssigen oder festen anorganischen Säure oder organischen Säure oder einer Lösung einer anorganischen Säure oder organischen Säure zum obigen System, wodurch ein saures Salz von 9-((8-Fluor-6-(1-methyl-1H-pyrazol-4-yl)-[1.2.4]triazolo[4,3-a]pyridin-3-yl)thio)-4-methyl-2H-[1,4]oxaazino[3,2-c]chinolin-3(4H)-on erhalten wird; oder des
Zugebens einer festen freien Base von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on zu einer sauren Lösung, wodurch ein saures Salz von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on erhalten wird;
2) Isolierens des beim salzbildenden Verfahren ausgefällten festen Produkts oder Erhaltens des festen Produkts durch das Erzeugen einer Übersättigung des salzbildenden Systems, wodurch das kristalline saure Salz von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on hergestellt wird, wobei ein Verfahren zur Erzeugung einer Übersättigung ein Abdampfen von Lösungsmittel oder Zugeben eines Antilösungsmittels oder ein Kühlverfahren umfasst;
und /oder
Umwandelns einer Kristallform des sauren Salzes in eine andere Kristallform des sauren Salzes durch ein Kristalltransformationsverfahren, wobei das Kristalltransformationsverfahren: ein Erwärmen umfasst, oder ein Kristalltransformationsverfahren einer Suspension in einem geeigneten Lösungsmittel;
wobei das geeignete organische Lösungsmittel des salzbildenden Verfahrens in Schritt 1) ausgewählt ist aus der Gruppe bestehend aus Alkoholen, Chloralkanen, Ketonen, Ethern, cyclischen Ethern, Estern, Alkanen, Cycloalkanen, Benzolen, Amiden, Sulfoxiden und einer Mischung davon, vorzugsweise Methanol, Ethanol, n-Propanol, Isopropanol, Acetonitril, Aceton, 1,4-Dioxan, Tetrahydrofuran, N,N-Dimethylcarboxamid, Ethylacetat, Isopropylacetat, 2-Methoxyethylether und einer Mischung davon;
wobei das "saure Salz" ein anorganisches saures Salz oder ein organisches saures Salz umfasst; wobei das anorganische saure Salz vorzugsweise ausgewählt ist aus der Gruppe bestehend aus Hydrochlorid, Sulfat, Hydrobromid, Hydrofluorid, Hydroiodid und Phosphat, noch mehr bevorzugt Hydrochlorid, Sulfat und Phosphat; wobei das organische saure Salz vorzugsweise ausgewählt ist aus der Gruppe bestehend aus 2,5-Dihydroxybenzolformiat, 1-Hydroxy-2-naphthalinformiat, Acetat, Dichloracetat, Trichloracetat, Acetohydroxamat, Adipat, Benzolsulfonat, 4-Chlorbenzolsulfonat, Benzolformiat, 4-Acetamidobenzolformiat, 4-Aminobenzolformiat, Caprat, Caproat, Caprylat, Cinnamoat, Citrat, Cyclohexylsulfamat, Camphersulfonat, Aspartat, Camphorat, Gluconat, Glucuronat, Glutamat, Erythrobat, Lactat, Aspartat, Malat, Mandelat, Pyroglutamat, Tartrat, Laurylsulfat, Dibenzoyltartrat, Ethyl-1,2-disulfonat, Esylat, Formiat, Fumarat, Galactonat, Gentisat, Glutarat, 2-Onglutarat, Glycollat, Hippurat, Isethionat, Lactobionat, Ascorbat, Aspartat, Laurat, Camphorat, Maleat, Malonat, Mesylat, 1,5-Naphthalindisulfonat, Naphthalin-2-sulfonat, Nicotinat, Oleat, Orotat, Oxalat, Palmitat, Embonat, Propionat, Salicylat, 4-Aminosalicylat, Sebacat, Stearat, Butandioat, Thiocyanat, Undecylenat, Trifluoracetat, Succinat und p-Toluolsulfonat, noch mehr bevorzugt Mesylat, p-Toluolsulfonat und 1,5-Naphthalindisulfonat.

11. Herstellungsverfahren nach Anspruch 10, **dadurch gekennzeichnet, dass**
a) ein geeignetes Lösungsmittel zur freien Base von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on gegeben wird, dann ein gleiches oder überschüssiges Moläquivalent von Methansulfonsäure zugegeben wird und die Mischung dann gerührt wird, nach Abschluss der salzbildenden Reaktion eine Fest-Flüssig-Trennung durchgeführt wird, wodurch die Kristallform I des Mesylats erhalten wird, wobei das geeignete Lösungsmittel ausgewählt ist aus der Gruppe bestehend aus Aceton, Tetrahydrofuran, Isopropylacetat, Ethylacetat, 2-Methoxyethylether, 1,4-Dioxan und einer Mischung davon, oder
b) eine Methanol-Wasser-Lösung mit einem Volumenverhältnis von: 5 % - 95 % zur freien Base von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on gegeben wird, die Mischung gerührt wird, ein gleiches oder überschüssiges Moläquivalent von Methansulfonsäure zugegeben wird, bis die Lösung klar ist, nach dem Ausfallen des Mesylats eine Fest-Flüssig-Trennung durchgeführt wird, wodurch die Kristallform II des Mesylats erhalten wird, oder
c) Methanol zur freien Base von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on gegeben wird, dann ein gleiches oder überschüssiges Moläquivalent von Methansulfonsäure langsam zugegeben wird, nach dem Lösen der Verbindung ein Kristallkeim der Kristallform III des Mesylats sofort zugegeben wird, die Mischung gerührt wird, nach Abschluss der salzbildenden Reaktion eine Fest-Flüssig-Trennung durchgeführt wird, wodurch die Kristallform III des Mesylats erhalten wird; oder
in Abwesenheit eines Kristallkeims zuerst die Kristallform IV des Mesylats erhalten wird, die Kristallform IV des Mesylats dann unter Vakuum über Nacht bei 100-120 °C getrocknet wird, die Kristallform IV des Mesylats zur Kristallform III des Mesylats transformiert wird; oder
die Kristallform V des Mesylats in einem einzelnen oder gemischten Antilösungsmittel dispergiert wird, die Mischung dann bei Raumtemperatur oder unter Erwärmen gerührt wird, die Kristallform V des Mesylats zur Kristallform III des Mesylats transformiert wird, oder
d) Methanol zur freien Base von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on gegeben wird, ein gleiches oder überschüssiges Moläquivalent von Methansulfonsäure langsam zugegeben wird, die Mischung gerührt wird, nach Abschluss der salzbildenden Reaktion eine Fest-Flüssig-Trennung durchgeführt wird, wodurch die Kristallform IV des Mesylats erhalten wird; oder
e) Dimethylsulfoxid zur freien Base von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on gegeben wird, dann ein gleiches oder überschüssiges Moläquivalent von Methansulfonsäure langsam zugegeben wird, die Mischung gerührt wird, nach Abschluss der salzbildenden Reaktion eine geeignete Menge von Ethylacetat zugegeben wird und das Rühren fortgesetzt wird, dann eine Fest-Flüssig-Trennung durchgeführt wird, wodurch die Kristallform V des Mesylats erhalten wird.

12. Pharmazeutische Zusammensetzung, **dadurch gekennzeichnet, dass** sie eine therapeutisch wirksame Menge der kristallinen freien Base von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on nach Anspruch 1 oder das kristalline saure Salz oder ein Polymorph davon nach einem der Ansprüche 2-8 und einen pharmazeutisch annehmbaren Träger oder Arzneimittelhilfsstoff umfasst.

13. Kristalline freie Base von 9-((8-Fluor-6-(1-methyl-1H-pyrazol-4-yl)-[1,2,4]triazolo[4,3-a]pyridin-3-yl)thio)-4-methyl-2H-[1,4]oxaazido[3,2-c]chinolin-3(4H)-on oder ein Polymorph davon nach Anspruch 1, kristallines saures Salz oder das Polymorph davon nach einem der Ansprüche 2-8 oder pharmazeutische Zusammensetzung nach Anspruch 12 zur Verwendung bei der Behandlung einer Krebserkrankung und einer Metastase einschließlich einer Krebserkrankung, eines hämatopoetischen Tumors des Lymphsystems, eines hämatopoetischen Tumors des Knochenmarksystems, eines mesenchymalen Tumors, eines Tumors des Zentral- und peripheren Nervensystems oder eines anderen Tumors;
wobei die Krebserkrankung vorzugsweise ausgewählt ist aus der Gruppe bestehend aus Blasenkrebs, Brustkrebs, dem Kolonkarzinom, Nierenkrebs, Leberkrebs, Lungenkrebs, Magenkrebs und Hautkrebs; wobei der hämatopoetische Tumor des Lymphsystems ausgewählt ist aus der Gruppe bestehend aus Leukämie, akuter lymphatischer Leukämie und chronischer lymphozytischer Leukämie; wobei der hämatopoetische Tumor des Knochenmarksystems ausgewählt ist aus der Gruppe bestehend aus akuter oder chronischer myelogener Leukämie, dem myelodysplastischen Syndrom und promyelozytärer Leukämie; wobei der mesenchymale Tumor ausgewählt ist aus der Gruppe bestehend aus einem Fibrosarkom, Rhabdomyosarkom, Weichgewebssarkom und einem Osteosarkom; wobei der Tumor des Zentral- und peripheren Nervensystems ausgewählt ist aus der Gruppe bestehend aus einem Astrozytom, Neuroblastom, Gliom und einem Nervenendfasertumor; wobei der andere Tumor ausgewählt ist aus der Gruppe bestehend aus einem bösartigen Melanom, Seminom, Teratokarzinom, Schilddrüsenkrebs und dem Kaposi-Sarkom; vorzugsweise von Leberkrebs, Lungenkrebs, Brustkrebs, des Plattenepithelkarzinoms der Haut und Magenkrebs.

## Revendications

1. Base cristalline libre de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one, dans laquelle la base cristalline libre est choisie dans le groupe constitué de la forme cristalline I, de la forme cristalline II, de la forme cristalline III et de la forme cristalline IV, dans laquelle un spectre de diffraction des rayons X sur poudre de la forme cristalline I de la base cristalline libre comprend des pics à des angles de diffraction (2θ) de 13,0 ± 0,2°, 17,9 ± 0,2°, 21,2 ± 0,2° et 31,4 ± 0,2°, de préférence comprend également des pics à des angles de diffraction (2 θ) de 10,3 ± 0,2°, 11,1 ± 0,2°, 23,3 ± 0,2°, 23,8 ± 0,2° et 33,6 ± 0,2°, de manière plus préférée comprend également des pics à des angles de diffraction (2 θ) de 15,7 ± 0,2°, 17,7 ± 0,2°, 26,8 ± 0,2°, 28,0 ± 0,2°, 31,7 ± 0,2° et 32,8 ± 0,2°, de la manière la plus préférée le spectre de diffraction des rayons X sur poudre comprend sensiblement le mêmes pics à des angles de diffraction (2θ) qu'indiqué sur la figure 1 ; dans laquelle un spectre de diffraction des rayons X sur poudre de la forme cristalline II de la base cristalline libre comprend des pics à des angles de diffraction (2θ) de 8,6 ± 0,2°, 11,5 ± 0,2°, 14,1 ± 0,2° et 19,8 ± 0,2°, de préférence comprend également des pics à des angles de diffraction (2θ) de 11,9 ± 0,2°, 14,7 ± 0,2°, 15,2 ± 0,2°, 17,2 ± 0,2° et 18,9 ± 0,2°, de manière plus préférée comprend en outre des pics à des angles de diffraction (2θ) de 5,8 ± 0,2°, 7,4 ± 0,2°, 20,9 ± 0,2°, 30,9 ± 0,2°, 31,4 ± 0,2° et 37,9 ± 0,2°, de la manière la plus préférée le spectre de diffraction des rayons X sur poudre comprend sensiblement les mêmes pics à des angles de diffraction (2θ) qu'indiqué sur la figure 4 ; dans laquelle un spectre de diffraction des rayons X sur poudre de la forme cristalline III de la base cristalline libre comprend des pics à des angles de diffraction (2θ) de 12,8 ± 0,2°, 14,8 ± 0,2°, 18,0 ± 0,2° et 20,5 ± 0,2°, de préférence comprend également des pics à des angles de diffraction (2θ) de 8,9 ± 0,2°, 9,2 ± 0,2°, 10,6 ± 0,2°, 15,8 ± 0,2° et 20,7 ± 0,2°, de manière plus préférée comprend en outre des pics à des angles de diffraction (20) de 5,3 ± 0,2°, 5,9 ± 0,2°, 12,0 ± 0,2°, 14,0 ± 0,2°, 17,3 ± 0,2° et 19,9 ± 0,2°, de la manière la plus préférée le spectre de diffraction des rayons X sur poudre comprend sensiblement les mêmes pics à des angles de diffraction (2θ) qu'indiqué sur la figure 5 ; dans laquelle un spectre de diffraction des rayons X sur poudre de la forme cristalline IV de la base cristalline libre comprend des pics à angles de diffraction (2θ) de 8,9 ± 0,2°, 12,6 ± 0,2°, 17,0 ± 0,2° et 17,9 ± 0,2°, de préférence comprend également des pics à des angles de diffraction (2θ) de 13,2 ± 0,2°, 14,5 ± 0,2°, 20,5 ± 0,2°, 23,9 ± 0,2° et 26,3 ± 0,2°, de la manière la plus préférée le spectre de diffraction des rayons X sur poudre comprend sensiblement les mêmes pics à des angles de diffraction (2θ) qu'indiqué sur la figure 7.

2. Sel d'acide cristallin de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one, dans lequel le sel d'acide comprend un sel d'acide inorganique ou un sel d'acide organique ; dans lequel le sel d'acide inorganique est de préférence choisi dans le groupe comprenant chlorhydrate, sulfate, hydrobromate, hydrofluorure, hydroiodure et phosphate, de manière plus préférée choisi dans le groupe comprenant chlorhydrate, sulfate et phosphate ; dans lequel le sel d'acide organique est de préférence choisi dans le groupe comprenant 2,5-dihydroxybenzèneformate, 1-hydroxy-2-naphtalèneformate, acétate, dichloroacétate, trichloroacétate, acétohydroxamate, adipate, sulfonate de benzène, sulfonate de 4-chlorobenzène, benzèneformate, 4-acétamidobenzèneformate, 4-aminobenzèneformate, caprate, caproate, caprilate, cinnamoate, citrate, cyclohexylsulfamate, camphorsulfonate, aspartate, camphorate, gluconate, glucuronate, glutamate, érythorbate, lactate, aspartate, mandélate, pyroglutamate, tartrate, sulfate de lauryle, dibenzoyltartrate, éthyl-1,2-disulfonate, ésylate, formate, fumarate galactonate, gentisate, glutarate, 2-oxoglutarate, glycollate, hippurate, iséthionate, lactobionate, ascorbate, aspartate, laurate, camphorate, maléate, malonate, mésylate, 1,5-naphtalènedisulfonate, naphtalène-2-sulfonate, nicotinate, oléate, orotate, oxalate, palmitate, embonate, propionate, salicylate, 4-aminosalicylate, sébacate, stéarate, butanedioate, thiocyanate, undécylénate, trifluoroacétate, succinate et p-toluènesulfonate, de préférence choisi dans le groupe comprenant mésylate, p-toluènesulfonate et 1,5-naphtalènedisulfonate.

3. Sel d'acide cristallin de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one selon la revendication 2, **caractérisé en ce que** le sel d'acide est la forme cristalline I de chlorhydrate ayant un spectre de diffraction des rayons X sur poudre comprenant des pics à des angles de diffraction (2θ) de 8,1 ± 0,2°, 19,2 ± 0,2°, 24,1 ± 0,2° et 26,2 ± 0,2°, comprenant de préférence également des pics à des angles de diffraction (2θ) de 9,1 ± 0,2°, 11,3 ± 0,2°, 13,4 ± 0,2°, 29,7 ± 0,2° et 23,4 ± 0,2°, de la manière la plus préférée, le spectre de diffraction des rayons X sur poudre comprenant sensiblement les mêmes pics à des angles de diffraction(2θ) qu'indiqué sur la figure 8.

4. Sel d'acide cristallin de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one selon la revendication 2, dans lequel le sel d'acide est un sulfate, de préférence choisi dans le groupe constitué de la forme cristalline I et de la forme cristalline II du sulfate, dans lequel un spectre de diffraction des rayons X sur poudre de la forme cristalline I de sulfate comprend des pics à des angles de diffraction (2θ) de 18,4 ± 0,2°, 19,7 ± 0,2°, 23,8 ± 0,2° et 24,5 ± 0,2°, comprend de préférence également des pics à des angles de diffraction (2θ) de 12,8 ± 0,2°, 14,4 ± 0,2°, 17,0 ± 0,2°, 20,0 ± 0,2° et 21,0 ± 0,2°, de manière plus préférée comprend en outre également des pics à des angles de diffraction (2θ) de 8,7 ± 0,2°, 13,2 ± 0,2°, 19,1 ± 0,2°, 26,3 ± 0,2°, 26,6 ± 0,2° et 29,0 ± 0,2° C, de la manière la plus préférée, le spectre de diffraction des rayons X sur poudre comprend sensiblement les mêmes pics à de angles de diffraction (2θ) qu'indiqué sur la figure 9 ; dans lequel un spectre de diffraction des rayons X sur poudre de la forme cristalline II de sulfate comprenant des pics à des angles de diffraction (2θ) de 6,3 ± 0,2°, 8,7 ± 0,2°, 12,7 ± 0,2° et 18,4 ± 0,2°, de préférence comprenant également des pics à des angles de diffraction (2θ) de 8,3 ± 0,2°, 17,5 ± 0,2°, 18,7 ± 0,2°, 20,4 ± 0,2° et 25,6 ± 0,2°, de manière plus préféré, comprend en outre également des pics à des angles de diffraction (2θ) de 9,1 ± 0,2°, 15,9 ± 0,2°, 16,8 ± 0,2°, 24,0 ± 0,2°, 25,2 ± 0,2° et 28,4 ± 0,2°, de la manière la plus préférée, le spectre de diffraction des rayons X sur poudres comprend sensiblement les mêmes pics à des angles de diffraction (2θ) qu'indiqué sur la figure 10.

5. Sel d'acide cristallin de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one selon la revendication 2, dans lequel le sel d'acide est un phosphate, de préférence choisi dans le groupe constitué de la forme cristalline I, de la forme cristalline II, de la forme cristalline III et de la forme cristalline IV de phosphate, dans lequel un spectre de diffraction des rayons X sur poudre de la forme cristalline I de phosphate comprend des pics à des angles de diffraction (2θ) de 7,9 ± 0,2°, 12,8 ± 0,2°, 15,9 ± 0,2° et 18,3 ± 0,2°, de préférence comprend également des pics à des angles de diffraction (2θ) de 5,3 ± 0,2°, 10,6 ± 0,2°, 13,4 ± 0,2°, 20,9 ± 0,2° et 24,7 ± 0,2°, de manière plus préférée comprend également des pics à des angles de diffraction (2θ) de 16,5 ± 0,2°, 18,7 ± 0,2°, 20,6 ± 0,2°, 21,8 ± 0,2°, 26,2 ± 0,2° et 27,4 ± 0,2°, de la manière la plus préférée, le spectre de diffraction des rayons X sur poudres comprend sensiblement les mêmes pics à des angles de diffraction (2θ) qu'indiqué sur la figure 11 ; dans lequel un spectre de diffraction des rayons X sur poudre de la forme cristalline II de phosphate comprend des pics à des angles de diffraction (2θ) de 13,7 ± 0,2°, 16,1 ± 0,2°, 22,8 ± 0,2° et 26,1 ± 0,2°, de préférence comprend également des pics à des angles de diffraction (2 θ) de 11,0 ± 0,2°, 14,6 ± 0,2°, 20,3 ± 0,2°, 20,8 ± 0,2° et 25,7 ± 0,2°, de manière plus préférée comprend en outre également des pics à des angles de diffraction (2θ) de 6,8 ± 0,2°, 17,0 ± 0,2°, 22,2 ± 0,2°, 26,6 ± 0,2°, 27,9 ± 0,2° et 31,2 ± 0,2°, de la manière la plus préférée, le spectre de diffraction des rayons X sur poudre comprend sensiblement les mêmes pics à des angles de diffraction (2θ), qu'indiqué sur la figure 12 ; dans lequel un spectre de diffraction des rayons X sur poudre de la forme cristalline III de phosphate comprend des pics à des angles de diffraction (2θ) de 9,7 ± 0,2°, 15,6 ± 0,2°, 16,8 ± 0,2° et 24,6 ± 0,2°, de préférence comprend également des pics à des angles de diffraction (2θ) de 4,8 ± 0,2°, 21,2 ± 0,2°, 25,0 ± 0,2°, 27,8 ± 0,2° et 28,1 ± 0,2°, de manière plus préférée comprend en outre également des pics à des angles de diffraction (2θ) de 5,2 ± 0,2°, 12,8 ± 0,2°, 14,5 ± 0,2°, 18,0 ± 0,2°, 20,1 ± 0,2° et 23,5 ± 0,2°, de la manière la plus préférée, le spectre de diffraction des rayons X sur poudres comprend sensiblement les mêmes pics à des angles de diffraction (2θ) qu'indiqué sur la figure 13 ; dans lequel un spectre de diffraction des rayons X sur poudre de la forme cristalline IV de phosphate comprend des pics à des angles de diffraction (2θ) de 7,8 ± 0,2°, 17,9 ± 0,2°, 25,0 ± 0,2° et 27,7 ± 0,2°, de préférence comprend également des pics à des angles de diffraction (2θ) de 6,7 ± 0,2°, 10,8 ± 0,2°, 15,6 ± 0,2°, 23,4 ± 0,2° et 24,6 ± 0,2°, de manière plus préférée comprend en outre également des pics à des angles de diffraction (2θ) de 5,2 ± 0,2°, 12,8 ± 0,2°, 20,9 ± 0,2°, 21,7 ± 0,2°, 22,3 ± 0,2° et 26,8 ± 0,2°, de la manière la plus préférée, le spectre de diffraction des rayons X sur poudres comprend sensiblement les mêmes pics à des angles de diffraction (2θ) qu'indiqué sur la figure 14.

6. Sel d'acide cristallin de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one selon la revendication 2, dans laquelle le sel d'acide est le mésylate, de préférence choisi dans le groupe constitué de la forme cristalline I, de la forme cristalline II, de la forme cristalline III, de la forme cristalline IV et de la forme cristalline V de mésylate, dans lequel un spectre de diffraction des rayons X sur poudre de la forme cristalline I de mésylate comprend des pics à des angles de diffraction (2θ) de 15,6 ± 0,2°, 17,0 ± 0,2°, 25,6 ± 0,2° et 26,0 ± 0,2°, de préférence comprend également des pics à des angles de diffraction (2θ) de 9,8 ± 0,2°, 21,8 ± 0,2°, 23,5 ± 0,2°, 23,8 ± 0,2° et 27,5 ± 0,2°, de manière plus préférée comprend en outre également des pics à des angles de diffraction (2θ) de 6,6 ± 0,2°, 15,3 ± 0,2°, 17,2 ± 0,2°, 18,3 ± 0,2°, 19,7 ± 0,2° et 26,4 ± 0,2°, de la manière la plus préférée, le spectre de diffraction des rayons X sur poudre comprend sensiblement les mêmes pics à des angles de diffraction (2θ) qu'indiqué sur la figure 15 ; dans lequel un spectre de diffraction des rayons X sur poudre de la forme cristalline II de mésylate comprend des pics à des angles de diffraction (2θ) de 9,4 ± 0,2°, 17,0 ± 0,2°, 18,9 ± 0,2° et 27,3 ± 0,2°, de préférence comprend également des pics à des angles de diffraction (2θ) de 6,6 ± 0,2°, 14,9 ± 0,2°, 21,1 ± 0,2°, 26,1 ± 0,2° et 26,9 ± 0,2°, de manière plus préférée, le spectre de diffraction des rayons X sur poudre comprend sensiblement les mêmes pics à des angles de diffraction (2θ) qu'indiqué sur la figure 16 ; dans lequel un spectre de diffraction des rayons X sur poudre de la forme cristalline III de mésylate comprend des pics à des angles de diffraction (2θ) de 16,7 ± 0,2°, 19,3 ± 0,2°, 23,2 ± 0,2° et 26,5 ± 0,2°, de préférence comprend également des pics à des angles de diffraction (2θ) de 8,7 ± 0,2°, 19,5 ± 0,2°, 21,8 ± 0,2°, 23,6 ± 0,2° et 24,3 ± 0,2°, de manière plus préférée comprend en outre également des pics à des angles de diffraction (2θ) de 11,7 ± 0,2°, 13,6 ± 0,2°, 14,1 ± 0,2°, 17,2 ± 0,2°, 18,7 ± 0,2° et 27,2 ± 0,2°, de la manière la plus préférée, le spectre de diffraction des rayons X sur poudre comprend sensiblement les mêmes pics à des angles de diffraction (2θ) qu'indiqué sur la figure 17 ; dans lequel un spectre de diffraction des rayons X sur poudre de la forme cristalline IV de mésylate comprend des pics à des angles de diffraction (2θ) de 16,8 ± 0,2°, 19,1 ± 0,2°, 19,3 ± 0,2° et 22,1 ± 0,2°, de préférence comprend également des pics à des angles de diffraction (2θ) de 21,9 ± 0,2°, 23,2 ± 0,2°, 24,4 ± 0,2°, 26,0 ± 0,2° et 27,2 ± 0,2°, de manière plus préférée comprend en outre également des pics à des angles de diffraction (2θ) de 8,7 ± 0,2°, 13,4 ± 0,2°, 13,6 ± 0,2°, 19,6 ± 0,2°, 21,6 ± 0,2° et 26,6 ± 0,2°, de la manière la plus préférée, le spectre de diffraction des rayons X sur poudre comprend sensiblement les mêmes pics à des angles de diffraction (2θ) qu'indiqué sur la figure 19 ; dans lequel un spectre de diffraction des rayons X sur poudre de la forme cristalline V de mésylate comprend des pics à des angles de diffraction (2θ) de 25,2 ± 0,2°, 9,3 ± 0,2°, 16,6 ± 0,2° et 19,1 ± 0,2°, de préférence comprend également des pics à des angles de diffraction (2θ) de 22,7 ± 0,2°, 16,3 ± 0,2°, 21,2 ± 0,2°, 8,9 ± 0,2° et 12,3 ± 0,2°, de manière plus préférée comprend en outre également des pics à des angles de diffraction (2θ) de 23,7 ± 0,2°, 20,0 ± 0,2°, 15,9 ± 0,2°, 24,6 ± 0,2°, 28,6 ± 0,2° et 25,5 ± 0,2°, de la manière la plus préférée, le spectre de diffraction des rayons X sur poudre comprend sensiblement les mêmes pics à des angles de diffraction (2θ) qu'indiqué sur la figure 20.

7. Sel d'acide cristallin de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one selon la revendication 2, dans lequel le sel d'acide est le p-toluènesulfonate, de préférence choisi dans le groupe constitué de la forme cristalline I, de la forme cristalline II et de la forme cristalline III du p-toluènesulfonate, dans lequel un spectre de diffraction des rayons X sur poudre de la forme cristalline I du p-toluènesulfonate comprend des pics à des angles de diffraction (2θ) de 13,0 ± 0,2°, 15,4 ± 0,2°, 24,3 ± 0,2° et 25,7 ± 0,2°, de préférence, comprend également des pics à des angles de diffraction (2θ) de 5,3 ± 0,2°, 12,1 ± 0,2°, 18,4 ± 0,2°, 22,6 ± 0,2° et 23,2 ± 0,2°, de manière plus préférée comprend en outre également des pics à des angles de diffraction (2θ) de 14,6 ± 0,2°, 16,9 ± 0,2°, 18,8 ± 0,2°, 19,9 ± 0,2°, 25,3 ± 0,2° et 29,3 ± 0,2°, de la manière la plus préférée, le spectre de diffraction des rayons X sur poudre comprend sensiblement les mêmes pics à des angles de diffraction (2θ) qu'indiqué sur la figure 21 ; dans lequel un spectre de diffraction des rayons X sur poudre de la forme cristalline II du p-toluènesulfonate comprend des pics à des angles de diffraction (2θ) de 13,7 ± 0,2°, 16,1 ± 0,2°, 25,7 ± 0,2° et 26,1 ± 0,2°, de préférence comprend également des pics à des angles de diffraction (2θ) de 11,0 ± 0,2°, 14,6 ± 0,2°, 17,0 ± 0,2°, 22,8 ± 0,2° et 26,6 ± 0,2°, de manière plus préférée comprend en outre également des pics à des angles de diffraction (2θ) de 6,8 ± 0,2°, 20,3 ± 0,2°, 20,8 ± 0,2°, 22,2 ± 0,2°, 24,6 ± 0,2° et 27,9 ± 0,2°, de la manière la plus préférée, le spectre de diffraction des rayons X sur poudre comprend sensiblement les mêmes pics à des angles de diffraction (2θ) qu'indiqué sur la figure 22 ; dans lequel un spectre de diffraction des rayons X sur poudre de la forme cristalline III de p-toluènesulfonate comprend des pics à des angles de diffraction (2θ) de 8,2 ± 0,2°, 14,4 ± 0,2°, 25,9 ± 0,2° et 26,3 ± 0,2 de préférence comprend également des pics à des angles de diffraction (2θ) de 10,3 ± 0,2°, 12,8 ± 0,2°, 17,2 ± 0,2°, 18,0 ± 0,2° et 19,9 ± 0,2°, de manière plus préférée comprend en outre également des pics à des angles de diffraction (2θ) de 4,8 ± 0,2°, 13,2 ± 0,2°, 15,1 ± 0,2°, 19,3 ± 0,2°, 24,2 ± 0,2° et 24,5 ± 0,2°, de la manière la plus préférée, le spectre de diffraction des rayons X sur poudre comprend sensiblement les mêmes pics à des angles de diffraction (2θ) qu'indiqué sur la figure 23.

8. Sel d'acide cristallin de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one selon la revendication 2, dans lequel le sel d'acide est le 1,5-naphtalènedisulfonate, de préférence choisi dans le groupe constitué par la forme cristalline I, la forme cristalline II et la forme cristalline III du 1,5-naphtalènedisulfonate, dans lequel un spectre de diffraction des rayons X sur poudre de la forme cristalline I de 1,5-naphtalènedisulfonate comprend des pics à des angles de diffraction (2θ) de 0,2°, 16,8 ± 0,2°, 21,8 ± 0,2° et 25,8 ± 0,2°, de préférence, comprend également des pics à des angles de diffraction (2θ) de 10,2 ± 0,2°, 16,0 ± 0,2°, 19,1 ± 0,2°, 20,8 ± 0,2° et 26,7 ± 0,2°, de manière plus préférée comprend en outre également des pics à des angles de diffraction (2θ) de 8,1 ± 0,2°, 13,6 ± 0,2°, 18,2 ± 0,2°, 18,7 ± 0,2°, 26,4 ± 0,2° et 30,9 ± 0,2°, de la manière la plus préférée, le spectre de diffraction des rayons X sur poudre comprend sensiblement les mêmes pics à des angles de diffraction (2θ) qu'indiqué sur la figure 24 ; dans lequel un spectre de diffraction des rayons X sur poudre de la forme cristalline II de 1,5-naphtalènedisulfonate comprend des pics à des angles de diffraction (2θ) de 4,2 ± 0,2°, 16,4 ± 0,2°, 22,8 ± 0,2° et 27,3 ± 0,2°, de préférence comprend également des pics à des angles de diffraction (2θ) de 8,5 ± 0,2°, 17,8 ± 0,2°, 19,1 ± 0,2°, 22,3 ± 0,2° et 28,1 ± 0,2°, de manière plus préférée comprend en outre également des pics à des angles de diffraction (2θ) de 10,4 ± 0,2°, 13,5 ± 0,2°, 15,1 ± 0,2°, 21,2 ± 0,2°, 24,0 ± 0,2° et 26,5 ± 0,2°, de la manière la plus préférée, le spectre de diffraction des rayons X sur poudre comprend sensiblement les mêmes pics à des angles de diffraction (2θ) qu'indiqué sur la figure 25 ; dans lequel un spectre de diffraction des rayons X sur poudre de la forme cristalline III de 1,5-naphtalènedisulfonate comprend des pics à des angles de diffraction (2θ) de 13,0 ± 0,2°, 22,7 ± 0,2°, 24,1 ± 0,2° et 25,7 ± 0,2°, de préférence comprend également des pics à des angles de diffraction (2θ) de 15,4 ± 0,2°, 18,8 ± 0,2°, 23,2 ± 0,2°, 25,4 ± 0,2° et 26,5 ± 0,2°, de manière plus préférée comprend également des pics à des angles de diffraction (2θ) de 12,6 ± 0,2°, 14,5 ± 0,2°, 16,9 ± 0,2°, 18,5 ± 0,2°, 20,0 ± 0,2° et 21,4 ± 0,2°, de la manière la plus préférée, le spectre de diffraction des rayons X sur poudre comprend sensiblement les mêmes pics à des angles de diffraction (2θ) qu'indiqué sur la figure 26.

9. Procédé de préparation d'une base cristalline libre de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one, comprenant les étapes suivantes consistant à :
dissoudre de la 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one dans un solvant aqueux, un solvant organique ou un mélange de solvants sous chauffage, puis refroidir la solution ou mélanger la solution avec un anti-solvant pour obtenir la base cristalline libre ; ou
évaporer une solution ou suspension de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one rapidement ou lentement pour obtenir la base cristalline libre ; ou
ajouter un composé solide original ou un autre additif de particules solides en tant que graine de cristal hétéronucléaire à une solution de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one pour induire la base cristalline libre ; ou
disperser la 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one dans un solvant aqueux, un solvant organique, un mélange de solvants ou une atmosphère de ces milieux pour obtenir la base cristalline libre ; ou
chauffer, sublimer, broyer, congeler ou fusionner-refroidir la 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one pour obtenir la base cristalline libre ; ou
ou combiner les procédés ci-dessus pour obtenir la base cristalline libre ;
dans lequel le solvant organique est de préférence choisi dans le groupe constitué par les alcools, les chloralcanes, les cétones, les éthers, les éthers cycliques, les esters, les alcanes, les cycloalcanes, les benzènes, les amides, les sulfoxydes et le mélange de ceux-ci, de manière davantage préférée le méthanol, l'éthanol, le n-propanol, l'isopropanol, le n-butanol, l'acétonitrile, l'acétone, la méthyléthylcétone, le tétrahydrofurane, le dioxanne, le N,N-diméthylcarboxamide, le diméthylsulfoxyde, l'acétate d'éthyle, le dichlorométhane, le trichloroéthane, le tétrachlorure de carbone, le n-heptane, le n-hexane, l'iso-octane, le pentane, le cyclohexane, le cyclopentane, l'éther diéthylique, l'éther méthylterbutylique, l'éther isopropylique, le benzène, le toluène, le xylène et le mélange de ceux-ci.

10. Procédé de préparation d'un sel d'acide cristallin de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one, comprenant les étapes suivantes consistant à :
1) dissoudre ou disperser une base libre de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one dans un solvant aqueux ou un solvant organique approprié, puis ajouter un acide inorganique liquide ou solide ou un acide organique ou une solution d'acide inorganique ou d'acide organique au système ci-dessus pour obtenir un sel d'acide de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one ; ou
ajouter un solide de base libre de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one à une solution acide pour obtenir un sel d'acide de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one ;
2) collecter le produit solide précipité pendant le processus de formation de sel, ou obtenir le produit solide en créant une sursaturation du système de formation de sel pour préparer le sel d'acide cristallin de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one, dans lequel un procédé pour créer une sursaturation comprend l'évaporation de solvant, ou l'addition d'un anti-solvant, ou un procédé de refroidissement ;
et/ou
transformer une forme cristalline du sel d'acide en une autre forme cristalline du sel acide par un procédé de transformation cristalline, le procédé de transformation cristalline comprenant : un chauffage ou un procédé de transformation cristalline d'une suspension dans un solvant approprié ;
dans lequel le solvant organique approprié du procédé de formation de sel dans l'étape 1) est choisi dans le groupe comprenant les alcools, les chloralcanes, les cétones, les éthers, les éthers cycliques, les esters, les alcanes, les cycloalcanes, les benzènes, les amides, les sulfoxydes et le mélange de ceux-ci, de préférence le méthanol, l'éthanol, le n-propanol, l'isopropanol, l'acétonitrile, l'acétone, le 1,4-dioxane, le tétrahydrofurane, le N,N-diméthylcarboxamide, l'acétate d'éthyle, l'acétate d'isopropyle, l'éther 2-méthoxyéthylique et le mélange de ceux-ci ;
dans lequel le « sel d'acide » comprend un sel d'acide inorganique ou un sel d'acide organique ; dans lequel le sel d'acide inorganique est de préférence choisi dans le groupe constitué d'un chlorhydrate, d'un sulfate, d'un hydrobromure, d'un hydrofluorure, d'un iodhydrate et d'un phosphate, de manière davantage préférée d'un chlorhydrate, d'un sulfate et d'un phosphate ; dans lequel le sel d'acide organique est de préférence choisi dans le groupe constitué du 2,5-dihydroxybenzèneformate, du 1-hydroxy-2-naphtalèneformate, de l'acétate, du dichloroacétate, du trichloroacétate, de l'acétohydroxamate, de l'adipate, du benzènesulfonate, du 4-chlorobenzènesulfonate, du 4-acétamidenzènesulfonate, du 4-aminobenzeneformate, du caprate, du caproate, du caprilate, du cinnamoate, du citrate, du cyclohexylsulfamate, du camphorsulfonate, de l'aspartate, du malate, du mandélate, du pyroglutamate, du tartrate, du sulfate de lauryle, du dibenzoyltartrate, du 1,2-disulfonate, l'ésylate, le formate, le fumarate, le galactonate, le gentisate, le glutarate, le 2-oneglutarate, le glycollate, l'hippurate, l'iséthionate, le lactobionate, l'ascorbate, l'aspartate, le laurate, le camphorate, le maléate, le malonate, le mésylate, le 1,5-naphthalènedisulfonate, le naphtalène-2-sulfonate, la nicotinate, l'oléate, l'orotate, l'oxalate, le palmitate, l'embonate, le propionate, le salicylate, le 4-aminosalicylate, le sébacate, le stéarate, le butanedioate, le thiocyanate, l'undécylénate, le trifluoroacétate, le succinate et le p-toluènesulfonate, de manière plus préférée le mésylate, le p-toluènesulfonate et le 1,5-naphtalènedisulfonate.

11. Procédé de préparation selon la revendication 10, **caractérisé en ce que**
a) un solvant approprié est ajouté à la base libre de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one, puis un équivalent molaire égal ou en excès d'acide méthanesulfonique est ajouté, et puis le mélange est agité, une fois la réaction de formation de sel terminée, une séparation solide-liquide est effectuée pour obtenir la forme cristalline I de mésylate, dans lequel le solvant approprié est choisi dans le groupe constitué par l'acétone, le tétrahydrofurane, l'acétate d'isopropyle, l'acétate d'éthyle, l'éther 2-méthoxyéthylique, le 1,4-dioxane et le mélange de ceux-ci, ou
b) une solution méthanol-eau avec un rapport volumique : 5 %-95 % est ajoutée à la base libre de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one, le mélange est agité, un équivalent molaire en excès ou équivalent d'acide méthanesulfonique est ajouté jusqu'à ce que la solution soit limpide, après précipitation du mésylate, une séparation solide-liquide est réalisée pour obtenir la forme cristalline II de mésylate, ou
c) du méthanol est ajouté à la base libre de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one, puis un équivalent molaire égal ou en excès d'acide méthanesulfonique est ajouté lentement, après dissolution du composé, un germe cristallin de forme cristalline III de mésylate est ajouté immédiatement, le mélange est agité, une fois la réaction de formation de sel terminée, une séparation solide-liquide est effectuée pour obtenir la forme cristalline III de mésylate ; ou
en l'absence de germes cristallins, la forme cristalline IV de mésylate est tout d'abord obtenue, puis on sèche la forme cristalline IV de mésylate est séchée sous vide à 100-120°C, et la forme cristalline IV du mésylate en la forme cristalline III du mésylate ; ou
la forme cristalline V du mésylate est dispersée dans un anti-solvant unique ou mélangé, puis le mélange est agité à température ambiante ou sous chauffage, et la forme cristalline V de mésylate est transformée en la forme cristalline III de mésylate, ou
ou d) du méthanol est ajouté à la base libre de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one, un équivalent molaire égal ou en excès d'acide méthanesulfonique est ajouté lentement, le mélange est agité, une fois la réaction de formation de sel terminée, une séparation solide-liquide est effectuée pour obtenir la forme cristalline IV de mésylate, ou
e) du diméthylsulfoxyde est ajouté à la base libre de 1,5-naphtalènedisulfonate, puis un équivalent molaire égal ou en excès d'acide méthanesulfonique est ajouté lentement, le mélange est agité, une fois la réaction de formation de sel terminée, une quantité appropriée d'acétate d'éthyle est ajoutée et l'agitation est poursuivie, puis une séparation solide-liquide est réalisée pour obtenir la forme cristalline V de mésylate.

12. Composition pharmaceutique, **caractérisée en ce qu'**elle comprend une quantité thérapeutiquement efficace de la base cristalline libre de 9 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one selon la revendication 1 ou du sel d'acide cristallin ou d'un polymorphe de celui-ci selon l'une quelconque des revendications 2 à 8, et un véhicule ou excipient pharmaceutiquement acceptable.

13. Base cristalline libre de 9-((8-fluoro-6-(1-méthyl-1H-pyrazol-4-yl))-[1,2,4]triazolo[4,3-a]pyridine-3-yl)thio)-4-méthyl-2H-[1,4]oxaazido[3,2-c]quinoléine-3(4H)-one ou polymorphe de celle-ci selon la revendication 1, son sel d'acide cristallin ou un polymorphe de celui-ci selon l'une quelconque une des revendications 2 à 8, ou la composition pharmaceutique selon la revendication 12, à utiliser dans le traitement du cancer et des métastases, y compris le cancer, une tumeur hématopoïétique du système lymphatique, une tumeur hématopoïétique du système de la moelle osseuse, une tumeur mésenchymateuse, une tumeur des systèmes nerveux central et périphérique ou une autre tumeur ;
de préférence, dans lequel le cancer est choisi dans le groupe constitué du cancer de la vessie, du cancer du sein, du cancer du côlon, du cancer du rein, du cancer du foie, du cancer du poumon, du cancer de l'estomac et du cancer de la peau ; dans lequel la tumeur hématopoïétique du système lymphatique est choisie dans le groupe comprenant la leucémie, la leucémie lymphocytaire aiguë et la leucémie lymphocytaire chronique ; dans lequel la tumeur hématopoïétique du système de moelle osseuse est choisie dans le groupe constitué par la leucémie myélogène aiguë ou chronique, le syndrome myélodysplasique et la leucémie promyélocytique ; dans lequel la tumeur mésenchymateuse est choisie dans le groupe comprenant le fibrosarcome, le rhabdomyosarcome, le sarcome des tissus mous et l'ostéosarcome ; dans lequel la tumeur des systèmes nerveux central et périphérique est choisie dans le groupe comprenant l'astrocytome, le neuroblastome, le gliome et la tumeur de terminaison nerveuse ; dans lequel l'autre tumeur est choisie dans le groupe comprenant le mélanome malin, le séminome, le tératocarcinome, le cancer du follicule thyroïdien et le sarcome de Kaposi ; de préférence le cancer du foie, le cancer du poumon, le cancer du sein, le carcinome épidermoïde de la peau et le cancer de l'estomac.
